# EUROPEAN PATENT SPECIFICATION

(11) **EP 1 678 164 B1**
(45) Date of publication and mention of the grant of the patent: **11.04.2007**
(21) Application number: 04769554.9
(22) Date of filing: 04.10.2004
(51) Int. Cl.: C07D 403/04, C07D 403/14, C07D 407/14, C07D 409/14, C07D 413/14, A61K 31/497, A61P 29/00

(54) **SUBSTITUTED PYRAZINONE COMPOUNDS FOR THE TREATMENT OF INFLAMMATION**
SUBSTITUIERTE PYRAZINONVERBINDUNGEN ZUR BEHANDLUNG VON ENTZÜNDUNGEN
COMPOSES DE PYRAZINONE SUBSTITUES POUR LE TRAITEMENT DE L'INFLAMMATION

(30) Priority: 14.10.2003 US 510870 P
(43) Date of publication of application: 12.07.2006
(73) Proprietor: Pharmacia Corporation, Peapack, NJ 07977 (US)
(72) Inventor: BOYS, Mark, L., Ann Arbor, MI 48105 (US); CLARE, Michael, Chesterfield, MO 63017-1732 (US); MITTON-FRY, Mark, J., Groton, CT 06340 (US)
(74) Representative: Nevant, Marc
(86) International application number: PCT/IB2004/003238
(87) International publication number: WO 2005/035527

(56) References cited:
- WO-A-01/58890
- WO-A-02/44153
- WO-A-03/040131

## Description

### CROSS-REFERENCE TO RELATED APPLICATION

This application claims the benefit of U.S. Provisional Application Serial No. 60/510,870, filed October 14, 2003.

### FIELD OF THE INVENTION

This invention generally relates to anti-inflammatory pharmaceutical agents and specifically relates to pyrazinone compounds as inhibitors of IKK-2, an IκB kinase. The invention is further related to compositions comprising such compounds, and methods for treating cancer, inflammation, and inflammation-associated disorders such as arthritis.

### BACKGROUND OF THE INVENTION

Rheumatoid arthritis is a common inflammatory disease affecting approximately 1 % of the population. The disease is characterized by multiple painful swollen joints that severely limit the patient's daily function, and can progress to the destruction of the affected joints. A common treatment for rheumatoid arthritis is anti-inflammatory steroids. Steroids are clinically very effective, but are limited in their use because of multiple severe side-effects. Thus, a need exists for an anti-rheumatoid arthritis treatment that offers the potency of steroids without the associated toxicity. One of the mechanisms by which steroids exert their broad spectrum anti-inflammatory action is by inhibiting the activation of the transcription factor NF-κB. NF-κB plays a prominent role in immune and inflammatory responses by regulating the transcription of many early, inducible genes in a variety of cells including inflammatory enzymes such as COX-2 (i.e., cyclooxygenase-2) and iNOS (i.e., inducible nitric oxide synthase). NF-κB is sequestered in an inactive form in the cytoplasm by a member of the IκB family of inhibitory proteins, and this prevents gene transcription of these responsive genes in the nucleus. Stimulation of cells leads to the phosphorylation, ubiquination and degradation of IκB thereby releasing NF-κB to the nucleus for activation of gene transcription. Chronic activation of NF-κB has been demonstrated in vascular endothelium and synovial lining cells from patients with RA. Recently the IκB kinases (IKK-1 and IKK-2), which phosphorylate IκB and thereby initiate its degradation, have been cloned and initially characterized; these kinases appear to represent the critical, common denominator in the activation of NF-κB since antisense or dominant-negative IKK constructs block NF-κB nuclear translocation and inhibit NF-κB linked reported genes. Therefore, IKK-1 and/or IKK-2 represent novel and powerful targets for drug development.

It has been reported that selective IKK-2 inhibitors could be useful for the treatment of inflammatory diseases. See, e.g., Karin et al., Nat. Revs. **3**, 17-26, 2004.

Substituted pyrazines, pyrimidines and pyridazines useful for the treatment of senile dementia are described in U.S. Patent No. 5,260,293.

PCT Publication No. WO 01/05772 discloses substituted pyrazinones as capsase-3 inhibitors.

Diaryl piperazines and related compounds are disclosed as selective modulators of capsaicin receptors in PCT Publication No. WO 02/08221.

Pyrazinones, triazinones, and derivatives thereof are disclosed for the treatment of psychiatric disorders and neurological diseases in PCT Publication No. WO 98/11075.

WO 0158890, WO 0 244 153 and WO 03040131 disclose compounds which inhibit ink enzymes.

### SUMMARY OF THE INVENTION

This invention provides for, in part, IKK-2-inhibiting compounds of Formula I:

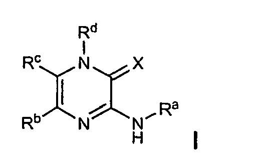

wherein X is selected from the group consisting of O, S, and NR^{5a};

wherein R^{a} and R^{c} are independently selected from the group consisting of hydrido, hydroxyl, alkoxy, alkyl, haloalkyl, aryl, and heteroaryl;

wherein R^{b} is a 3- to 12-membered cyclic moiety selected from the group consisting of cycloalkyl, cycloalkenyl, aryl, heterocycloalkyl, heterocycloalkenyl, and heteroaryl, wherein R^{b} is optionally substituted by one or more substituents selected from the group consisting of R², cycloalkyl, and cycloalkylalkyl;

wherein R^{d} is selected from the group consisting of -(CH₂)_{q}NH₂, -(CH₂)_{q}NHR², and a 5- to 7-membered heterocycloalkyl having ring members selected from the group consisting of carbon and nitrogen, wherein said heterocycloalkyl may be optionally substituted by one or more substituents selected from the group consisting of R²;

wherein R² is selected from the group consisting of halo, alkylsulfinyl, alkylsulfonyl, cyano, alkoxycarbonyl, alkyl, haloalkyl, hydroxyalkyl, haloalkoxy, nitro, acylamino, R⁷, -OR³, -(CH₂)ₘOR³, -(CH₂)ₚCO₂R³, -SR³, -SO₂N(R^{4a})R^{4b}, -NR^{5a}R^{5b}, -NR^{5a}COR^{5b}, -NR^{5a}CO(OR^{5b}), -NR^{5a}SO₂R⁶, -NR^{5a}SO₂N(R^{6a})R^{6b}, -NR^{5a}CON(R^{6a})R^{6b}, -COR^{5a}, and -CON(R^{4a})R^{4b};

wherein R³, R^{4a}, and R^{4b} are independently selected from the group consisting of hydrido, aryl, heteroaryl, heteroaralkyl, alkyl, haloalkyl, alkenyl, alkynyl, hydroxyalkyl, aminoalkyl, alkylaminoalkyl, N,N-dialkylaminoalkyl, alkoxy, alkoxyalkyl, heterocycloalkyl, heterocycloalkenyl, cycloalkyl, cycloalkylalkyl, aralkyl, and aralkylamino wherein said aryl is optionally substituted with one or more radicals selected from the group consisting of alkyl, aminoalkyl, alkoxy and halo, wherein R^{4a} and R^{4b} may be taken together to form a 3- to 7-membered heterocyclic ring having from 1 to 3 heteroatoms selected from S, SO, SO₂, O, N, and NR^{5a};

wherein R^{5a} and R^{5b} are independently selected from the group consisting of hydrido, alkyl, aryl, heteroaryl, aralkyl, heterocycloalkenyl, cycloalkyl, heterocycloalkyl, haloalkyl, aralkylamino, amino, aminoalkyl, aminoacyl, nitro, azido, and heteroaralkyl, wherein said alkyl, aryl, heteroaryl, aminoalkyl, and aralkyl moieties are optionally substituted with one or more substituents selected from the group consisting of alkylsulfonamido, sulfamyl, alkyl, alkylthio, alkylsulfinyl, alkylsulfonyl, N-alkylamino, aminoalkyl, alkylaminoalkyl, alkoxy, halo, acyloxy, oxy, formyl, haloalkyl, cyano, haloalkoxy, acyl, carboxyl, hydroxy, hydroxyalkoxy, phenoxy, nitro, azido, benzyloxy, N,N-dialkylaminoacyl, thioalkyl, aminoacyloxy, thiocyanato, isothiocyanato, alkyldioxy, hydroxyalkyl, N-alkylamino, alkoxycarbonyl, alkoxyalkyl, alkenylamino, alkynylamino, alkenyl, alkynyl, N,N-dialkylaminoalkoxy, heterocycloalkyl, heterocycloalkenyl, and heteroaryl;

wherein R^{6a} and R^{6b} are independently selected from the group consisting of hydrido, alkyl, heteroaryl, heterocycloalkenyl, haloalkyl, aralkylamino, heteroaralkyl, aryl, and aralkyl, wherein said aryl, heteroaryl, heterocycloalkenyl, and aralkyl moieties are optionally substituted with one or more substituents selected from alkyl, alkoxy, halo, haloalkyl, cyano, haloalkoxy, acyl, carboxyl, hydroxy, hydroxyalkoxy, phenoxy, benzyloxy, N,N-dialkylaminoalkoxy, heterocycloalkyl, heterocycloalkenyl, and heteroaryl, wherein R^{6a} and R^{6b} may be taken together to form a 3- to 7-membered heterocyclic ring having 1 to 3 heteroatoms selected from S, SO, SO₂, O, N, and NR^{5a};

wherein R⁷ is selected from the group consisting of aryl, heterocycloalkenyl, heteroaryl, and alkenyl, wherein R⁷ is optionally substituted with one or more substituents selected from the group consisting of R^{5a};

wherein n is 1, 2, or 3

wherein m is 1, 2, or 3;

wherein p is 0, 1, or 2;

wherein q is an integer between 0 and 9;

or a pharmaceutically acceptable salt thereof.

The instant invention is also directed to pharmaceutical compositions comprising a compound of Formula I or a pharmaceutically-acceptable salt thereof, as defined above, and a pharmaceutically acceptable carrier, diluent, or adjuvant.

The compounds of the invention can be used in a method of treating or preventing inflammation or an inflammation-associated disorder, the method comprising administering a compound of Formula I or a pharmaceutically acceptable salt thereof to a subject in need of such treatment or susceptible to such inflammation or inflammation-associated disorder.

Other objects of the invention will be in part apparent and in part pointed out hereinafter.

### DETAILED DESCRIPTION OF THE PREFERRED EMBODIMENTS

In accordance with the present invention, Applicants have discovered a class of IKK-2-inhibiting compounds of Formula I:

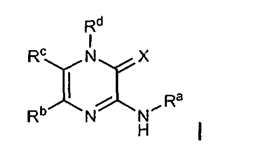

wherein X is selected from the group consisting of O, S, and NR^{5a};

wherein R^{a} and R^{c} are independently selected from the group consisting of hydrido, hydroxyl, alkoxy, alkyl, haloalkyl, aryl, and heteroaryl;

wherein R^{b} is a 3- to 12-membered cyclic moiety selected from the group consisting of cycloalkyl, cycloalkenyl, aryl, heterocycloalkyl, heterocycloalkenyl, and heteroaryl, wherein R^{b} is optionally substituted by one or more substituents selected from the group consisting of R², cycloalkyl, and cycloalkylalkyl;

wherein R^{d} is selected from the group consisting of -(CH₂)_{q}NH₂, -(CH₂)_{q}NHR², and a 5- to 7-membered heterocycloalkyl having ring members selected from the group consisting of carbon and nitrogen, wherein said heterocycloalkyl may be optionally substituted by one or more substituents selected from the group consisting of R²;

wherein R² is selected from the group consisting of halo, alkylsulfinyl, alkylsulfonyl, cyano, alkoxycarbonyl, alkyl, haloalkyl, hydroxyalkyl, haloalkoxy, nitro, acylamino, R⁷, -OR³, -(CH₂)ₘOR³, -(CH₂)ₚCO₂R³, -SR³, -SO₂N(R^{4a})R^{4b}, -NR^{5a}R^{5b}, -NR^{5a}COR^{5b}, -NR^{5a}CO(OR^{5b}), -NR^{5a}SO₂R⁶, -NR^{5a}SO₂N(R^{6a})R^{6b}, -NR^{5a}CON(R^{6a})R^{6b}, -COR^{5a}, and -CON(R^{4a})R^{4b};

wherein R³, R^{4a}, and R^{4b} are independently selected from the group consisting of hydrido, aryl, heteroaryl, heteroaralkyl, alkyl, haloalkyl, alkenyl, alkynyl, hydroxyalkyl, aminoalkyl, alkylaminoalkyl, N,N-dialkylaminoalkyl, alkoxy, alkoxyalkyl, heterocycloalkyl, heterocycloalkenyl, cycloalkyl, cycloalkylalkyl, aralkyl, and aralkylamino wherein said aryl is optionally substituted with one or more radicals selected from the group consisting of alkyl, aminoalkyl, alkoxy and halo, wherein R^{4a} and R^{4b} may be taken together to form a 3- to 7-membered heterocyclic ring having from 1 to 3 heteroatoms selected from S, SO, SO₂, O, N, and NR^{5a};

wherein R^{5a} and R^{5b} are independently selected from the group consisting of hydrido, alkyl, aryl, heteroaryl, aralkyl, heterocycloalkenyl, cycloalkyl, heterocycloalkyl, haloalkyl, aralkylamino, amino, aminoalkyl, aminoacyl, nitro, azido, and heteroaralkyl, wherein said alkyl, aryl, heteroaryl, aminoalkyl, and aralkyl moieties are optionally substituted with one or more substituents selected from the group consisting of alkylsulfonamido, sulfamyl, alkyl, alkylthio, alkylsulfinyl, alkylsulfonyl, N-alkylamino, aminoalkyl, alkylaminoalkyl, alkoxy, halo, acyloxy, oxy, formyl, haloalkyl, cyano, haloalkoxy, acyl, carboxyl, hydroxy, hydroxyalkoxy, phenoxy, nitro, azido, benzyloxy, N,N-dialkylaminoacyl, thioalkyl, aminoacyloxy, thiocyanato, isothiocyanato, alkyldioxy, hydroxyalkyl, N-alkylamino, alkoxycarbonyl, alkoxyalkyl, alkenylamino, alkynylamino, alkenyl, alkynyl, N,N-dialkylaminoalkoxy, heterocycloalkyl, heterocycloalkenyl, and heteroaryl;

wherein R^{6a} and R^{6b} are independently selected from the group consisting of hydrido, alkyl, heteroaryl, heterocycloalkenyl, haloalkyl, aralkylamino, heteroaralkyl, aryl, and aralkyl, wherein said aryl, heteroaryl, heterocycloalkenyl, and aralkyl moieties are optionally substituted with one or more substituents selected from alkyl, alkoxy, halo, haloalkyl, cyano, haloalkoxy, acyl, carboxyl, hydroxy, hydroxyalkoxy, phenoxy, benzyloxy, N,N-dialkylaminoalkoxy, heterocycloalkyl, heterocycloalkenyl, and heteroaryl, wherein R^{6a} and R^{6b} may be taken together to form a 3- to 7-membered heterocyclic ring having 1 to 3 heteroatoms selected from S, SO, SO₂, O, N, and NR^{5a};

wherein R⁷ is selected from the group consisting of aryl, heterocycloalkenyl, heteroaryl, and alkenyl, wherein R⁷ is optionally substituted with one or more substituents selected from the group consisting of R^{5a};

wherein n is 1, 2, or 3

wherein m is 1, 2, or 3;

wherein p is 0, 1, or 2;

wherein q is an integer between 0 and 9;

or a pharmaceutically acceptable salt thereof.

Compounds of Formula I may be useful for treating, among other things, inflammation in a subject, such as, as an analgesic in the treatment of pain and headaches, or as an antipyretic for the treatment of fever. For example, compounds of the present invention may be useful to treat arthritis, including but not limited to rheumatoid arthritis, spondyloarthropathies, gouty arthritis, osteoarthritis, systemic lupus erythematosus, juvenile arthritis, acute rheumatic arthritis, enteropathic arthritis, neuropathic arthritis, psoriatic arthritis, and pyogenic arthritis.

Compounds of the invention may be further useful in the treatment of frailty, asthma, chronic obstructive pulmonary disease (COPD), bronchitis, menstrual cramps (e.g., dysmenorrhea), premature labor, tendinitis, bursitis, dermatological conditions such as psoriasis, eczema, burns, sunburn, dermatitis, pancreatitis, hepatitis, and from post-operative inflammation including from ophthalmic surgery such as cataract surgery and refractive surgery. Compounds of the invention also would be useful to treat gastrointestinal conditions such as inflammatory bowel disease, Crohn's disease, gastritis, irritable bowel syndrome and ulcerative colitis. Compounds of the invention would be useful for the prevention or treatment of cancer, such as colorectal cancer, and cancer of the breast, lung, prostate, bladder, cervix and skin, as well as treatment of cancer stem cells. Compounds of the invention would be useful in treating inflammation and tissue damage in such diseases as vascular diseases, migraine headaches, periarteritis nodosa, thyroiditis, aplastic anemia, Hodgkin's disease, sclerodoma, rheumatic fever, type I diabetes, neuromuscular junction disease including myasthenia gravis, white matter disease including multiple sclerosis, sarcoidosis, nephrotic syndrome, Behcet's syndrome, polymyositis, gingivitis, nephritis, hypersensitivity, swelling occurring after injury, myocardial ischemia, and the like.

The compounds would also be useful in the treatment of pulmonary inflammation, such as that associated with viral infections and cystic fibrosis. The compounds would also be useful for the treatment of certain central nervous system disorders, such as cortical dementias including Alzheimer's disease, and central nervous system damage resulting from stroke, ischemia and trauma. The compounds of the invention are useful as anti-inflammatory agents, such as for the treatment of arthritis, with the additional benefit of having significantly less harmful side effects. These compounds would also be useful in the treatment of allergic rhinitis, respiratory distress syndrome, and atherosclerosis. The compounds would also be useful in the treatment of pain, but not limited to postoperative pain, dental pain, muscular pain, and pain resulting from cancer. The compounds would be useful for the prevention of dementias, such as Alzheimer's disease.

Besides being useful for human treatment, these compounds are also useful for veterinary treatment of companion animals, exotic animals and farm animals, including mammals, rodents, and the like. More preferred animals include horses, dogs, and cats.

The present compounds may also be used in co-therapies, partially or completely, in place of other conventional antiinflammatory therapies, such as together with steroids, NSAIDs, COX-2 selective inhibitors, 5-lipoxygenase inhibitors, LTB₄ antagonists and LTA₄ hydrolase inhibitors.

Other conditions in which the compounds of the present invention may provide an advantage include cardiovascular ischemia, diabetes (type I or type II), congestive heart failure, myocarditis, atherosclerosis, migraine, glaucoma, aortic aneurysm, reflux esophagitis, diarrhea, irritable bowel syndrome, cystic fibrosis, emphysema, asthma, bronchiectasis, hyperalgesia (allodynia), and cerebral ischemia (both focal ischemia, thrombotic stroke and global ischemia (for example, secondary to cardiac arrest).

The compounds of the present invention may also be useful in the treatment of pain including somatogenic (either nociceptive or neuropathic), both acute and chronic. A compound of the present invention could be used in any situation including neuropathic pain that a common NSAID or opioid analgesic would traditionally be administered.

Conjunctive treatment of a compound of the present invention with an antineoplastic agent may produce a beneficial effect or alternatively reduce the toxic side effects associated with chemotherapy by reducing the therapeutic dose of the side effect-causing agent needed for therapeutic efficacy or by directly reducing symptoms of toxic side effects caused by the side effect-causing agent. A compound of the present invention may further be useful as an adjunct to radiation therapy to reduce side effects or enhance efficacy. In the present invention, another agent which can be combined therapeutically with a compound of the present invention includes any therapeutic agent which is capable of inhibiting COX-2. Preferably such COX-2 inhibiting agents inhibit COX-2 selectively relative to the enzyme cyclooxygenase-1 ("COX-1 "). Such a COX-2 inhibitor is known as a "COX-2 selective inhibitor". More preferably, a compound of the present invention can be therapeutically combined with a COX-2 selective inhibitor wherein the COX-2 selective inhibitor selectively inhibits COX-2 at a ratio of at least 10:1 relative to inhibition of COX-1, more preferably at least 30:1, and still more preferably at least 50:1 in an *in vitro* test. COX-2 selective inhibitors useful in therapeutic combination with the compounds of the present invention include celecoxib, valdecoxib, deracoxib, etoricoxib, rofecoxib, ABT-963 (2-(3,4-difluorophenyl)-4-(3-hydroxy-3-methyl-1-butoxy)-5-[4-(methylsulfonyl)phenyl-3(2H)-pyridazinone; described in PCT Publication No. WO 00/24719), or meloxicam. A compound of the present invention can also be advantageously used in therapeutic combination with a prodrug of a COX-2 selective inhibitor, for example parecoxib.

Another chemotherapeutic agent which may be useful in combination with a compound of the present invention can be selected, for example, from the following non-comprehensive and non-limiting list: Alpha-difluoromethylornithine (DFMO), 5-FU-fibrinogen, acanthifolic acid, aminothiadiazole, brequinar sodium, carmofur, Ciba-Geigy CGP-30694, cyclopentyl cytosine, cytarabine phosphate stearate, cytarabine conjugates, Lilly DATHF, Merrill Dow DDFC, dezaguanine, dideoxycytidine, dideoxyguanosine, didox, Yoshitomi DMDC, doxifluridine, Wellcome EHNA, Merck & Co. EX-015, fazarabine, floxuridine, fludarabine phosphate, 5-fluorouracil, N-(2'-furanidyl)-5-fluorouracil, Daiichi Seiyaku FO-152, isopropyl pyrrolizine, Lilly LY-188011, Lilly LY-264618, methobenzaprim, methotrexate, Wellcome MZPES, norspermidine, NCI NSC-127716, NCI NSC-264880, NCI NSC-39661, NCI NSC-612567, Warner-Lambert PALA, pentostatin, piritrexim, plicamycin, Asahi Chemical PL-AC, Takeda TAC-788, thioguanine, tiazofurin, Erbamont TIF, trimetrexate, tyrosine kinase inhibitors, tyrosine protein kinase inhibitors, Taiho UFT, uricytin, Shionogi 254-S, aldo-phosphamide analogues, altretamine, anaxirone, Boehringer Mannheim BBR-2207, bestrabucil, budotitane, Wakunaga CA-102, carboplatin, carmustine, Chinoin-139, Chinoin-153, chlorambucil, cisplatin, cyclophosphamide, American Cyanamid CL-286558, Sanofi CY-233, cyplatate, Degussa D-19-384, Sumimoto DACHP(Myr)2, diphenylspiromustine, diplatinum cytostatic, Erba distamycin derivatives, Chugai DWA-2114R, ITI E09, elmustine, Erbamont FCE-24517, estramustine phosphate sodium, fotemustine, Unimed G-6-M, Chinoin GYKI-17230, hepsul-fam, ifosfamide, iproplatin, lomustine, mafosfamide, mitolactol, Nippon Kayaku NK-121, NCI NSC-264395, NCl NSC-342215, oxaliplatin, Upjohn PCNU, prednimustine, Proter PTT-119, ranimustine, semustine, SmithKline SK&F-101772, Yakult Honsha SN-22, spiromus-tine, Tanabe Seiyaku TA-077, tauromustine, temozolomide, teroxirone, tetraplatin, trimelamol, Taiho 4181-A, aclarubicin, actinomycin D, actinoplanone, Erbamont ADR-456, aeroplysinin derivative, Ajinomoto AN-201-II, Ajinomoto AN-3, Nippon Soda anisomycins, anthracycline, azino-mycin-A, bisucaberin, Bristol-Myers BL-6859, Bristol-Myers BMY-25067, Bristol-Myers BMY-25551, Bristol-Myers BMY-26605, Bristol-Myers BMY-27557, Bristol-Myers BMY-28438, bleomycin sulfate, bryostatin-1, Taiho C-1027, calichemycin, chromoximycin, dactinomycin, daunorubicin, Kyowa Hakko DC-102, Kyowa Hakko DC-79, Kyowa Hakko DC-88A, Kyowa Hakko DC89-A1, Kyowa Hakko DC92-B, ditrisarubicin B, Shionogi DOB-41, doxorubicin, doxorubicin-fibrinogen, elsamicin-A, epirubicin, erbstatin, esorubicin, esperamicin-A1, esperamicin-Alb, Erbamont FCE-21954, Fujisawa FK-973, fostriecin, Fujisawa FR-900482, glidobactin, gregatin-A, grincamycin, herbimycin, idarubicin, illudins, kazusamycin, kesarirhodins, Kyowa Hakko KM-5539, Kirin Brewery KRN-8602, Kyowa Hakko KT-5432, Kyowa Hakko KT-5594, Kyowa Hakko KT-6149, American Cyanamid LL-D49194, Meiji Seika ME 2303, menogaril, mitomycin, mitoxantrone, SmithKline M-TAG, neoenactin, Nippon Kayaku NK-313, Nippon Kayaku NKT-01, SRI International NSC-357704, oxalysine, oxaunomycin, peplomycin, pilatin, pirarubicin, porothramycin, pyrindamycin A, Tobishi RA-I, rapamycin, rhizoxin, rodorubicin, sibanomicin, siwenmycin, Sumitomo SM-5887, Snow Brand SN-706, Snow Brand SN-07, sorangicin-A, sparsomycin, SS Pharmaceutical SS-21020, SS Pharmaceutical SS-7313B, SS Pharmaceutical SS-9816B, steffimycin B, Taiho 4181-2, talisomycin, Takeda TAN-868A, terpentecin, thrazine, tricrozarin A, Upjohn U-73975, Kyowa Hakko UCN-10028A, Fujisawa WF-3405, Yoshitomi Y-25024 zorubicin, alpha-carotene, alpha-difluoromethyl-arginine, acitretin, Biotec AD-5, Kyorin AHC-52, alstonine, amonafide, amphethinile, amsacrine, Angiostat, ankinomycin, anti-neoplaston A10, antineoplaston A2, antineoplaston A3, antineoplaston A5, antineoplaston AS2-1, Henkel APD, aphidicolin glycinate, asparaginase, Avarol, baccharin, batracylin, benfluron, benzotript, Ipsen-Beaufour BIM-23015, bisantrene, Bristo-Myers BMY-40481, Vestar boron-10, bromofosfamide, Wellcome BW-502, Wellcome BW-773, caracemide, carmethizole hydrochloride, Ajinomoto CDAF, chlorsulfaquinoxalone, Chemex CHX-2053, Chemex CHX-100, Warner-Lambert CI-921, Warner-Lambert CI-937, Warner-Lambert CI-941, Warner-Lambert CI-958, clanfenur, claviridenone, ICN compound 1259, ICN compound 4711, Contracan, Yakult Honsha CPT-11, crisnatol, curaderm, cytochalasin B, cytarabine, cytocytin, Merz D-609, DABIS maleate, dacarbazine, datelliptinium, didemnin-B, dihaematoporphyrin ether, dihydrolenperone, dinaline, distamycin, Toyo Pharmar DM-341, Toyo Pharmar DM-75, Daiichi Seiyaku DN-9693, elliprabin, elliptinium acetate, Tsumura EPMTC, ergotamine, etoposide, etretinate, fenretinide, Fujisawa FR-57704, gallium nitrate, genkwadaphnin, Chugai GLA-43, Glaxo GR-63178, grifolan NMF-5N, hexadecylphosphocholine, Green Cross HO-221, homoharringtonine, hydroxyurea, BTG ICRF-187, ilmofosine, isoglutamine, isotretinoin, Otsuka JI-36, Ramot K-477, Otsuak K-76COONa, Kureha Chemical K-AM, MECT Corp KI-8110, American Cyanamid L-623, leukoregulin, lonidamine, Lundbeck LU-23-112, Lilly LY-186641, NCI (US) MAP, marycin, Merrel Dow MDL-27048, Medco MEDR-340, merbarone, merocyanine derivatives, methylanilinoacridine, Molecular Genetics MGI-136, minactivin, mitonafide, mitoquidone, mopidamol, motretinide, Zenyaku Kogyo MST-16, N-(retinoyl)amino acids, Nisshin Flour Milling N-021, N-acylated-dehydroalanines, nafazatrom, Taisho NCU-190, nocodazole derivative, Normosang, NCI NSC-145813, NCI NSC-361456, NCI NSC-604782, NCI NSC-95580, octreotide, Ono ONO-112, oquizanocine, Akzo Org-10172, pancratistatin, pazelliptine, Warner-Lambert PD-111707, Warner-Lambert PD-115934, Warner-Lambert PD-131141, Pierre Fabre PE-1001, ICRT peptide D, piroxantrone, polyhaematoporphyrin, polypreic acid, Efamol porphyrin, probimane, procarbazine, proglumide, Invitron protease nexin I, Tobishi RA-700, razoxane, Sapporo Breweries RBS, restrictin-P, retelliptine, retinoic acid, Rhone-Poulenc RP-49532, Rhone-Poulenc RP-56976, SmithKline SK&F-104864, Sumitomo SM-108, Kuraray SMANCS, SeaPharm SP-10094, spatol, spirocyclopropane derivatives, spirogermanium, Unimed, SS Pharmaceutical SS-554, strypoldinone, Stypoldione, Suntory SUN 0237, Suntory SUN 2071, superoxide dismutase, Toyama T-506, Toyama T-680, taxol, Teijin TEI-0303, teniposide, thaliblastine, Eastman Kodak TJB-29, tocotrienol, Topostin, Teijin TT-82, Kyowa Hakko UCN-01, Kyowa Hakko UCN-1028, ukrain, Eastman Kodak USB-006, vinblastine sulfate, vincristine, vindesine, vinestramide, vinorelbine, vintriptol, vinzolidine, withanolides, Yamanouchi YM-534, uroguanylin, combretastatin, dolastatin, idarubicin, epirubicin, estramustine, cyclophosphamide, 9-amino-2-(S)-camptothecin, topotecan, irinotecan (Camptosar), exemestane, decapeptyl (tryptorelin), or an omega-3 fatty acid.

Examples of radioprotective agents which may be used in a combination therapy with the compounds of this invention include AD-5, adchnon, amifostine analogues, detox, dimesna, I-102, MM-159, N-acylated-dehydroalanines, TGF-Genentech, tiprotimod, amifostine, WR-151327, FUT-187, ketoprofen transdermal, nabumetone, superoxide dismutase (Chiron) and superoxide dismutase Enzon.

The compounds of the present invention may also be useful in treatment or prevention of angiogenesis-related disorders or conditions, for example, tumor growth, metastasis, macular degeneration, and atherosclerosis.

In a further embodiment, the present invention also provides therapeutic combinations for the treatment or prevention of ophthalmic disorders or conditions such as glaucoma. For example the present inventive compounds advantageously may be used in therapeutic combination with a drug which reduces the intraocular pressure of patients afflicted with glaucoma. Such intraocular pressure-reducing drugs include without limitation latanoprost, travoprost, bimatoprost, or unoprostol. The therapeutic combination of a compound of the present invention plus an intraocular pressure-reducing drug may be useful because each is believed to achieve its effects by affecting a different mechanism.

In another combination of the present invention, the present inventive compounds can be used in therapeutic combination with an antihyperlipidemic or cholesterol-lowering drug such as a benzothiepine or a benzothiazepine antihyperlipidemic drug. Examples of benzothiepine antihyperlipidemic drugs useful in the present inventive therapeutic combination can be found in U.S. Patent No. 5,994,391, herein incorporated by reference. Some benzothiazepine antihyperlipidemic drugs are described in PCT Publication No. WO 93/16055. Alternatively, the antihyperlipidemic or cholesterol-lowering drug useful in combination with a compound of the present invention can be an HMG Co-A reductase inhibitor. Examples of HMG Co-A reductase inhibitors useful in the present therapeutic combination include, individually, benfluorex, fluvastatin, lovastatin, pravastatin, simvastatin, atorvastatin, cerivastatin, bervastatin, ZD-9720 (described in PCT Publication No. WO 97/06802), ZD-4522 (CAS No. 147098-20-2 for the calcium salt; CAS No. 147098-18-8 for the sodium salt; described in European Patent No. EP 521471), BMS 180431 (CAS No. 129829-03-4), or NK-104 (CAS No. 141750-63-2). The therapeutic combination of a compound of the present invention plus an antihyperlipidemic or cholesterol-lowering drug may be useful, for example, in reducing the risk of formation of atherosclerotic lesions in blood vessels. For example, atherosclerotic lesions often initiate at inflamed sites in blood vessels. It is established that antihyperlipidemic or cholesterol-lowering drug reduce risk of formation of atherosclerotic lesions by lowering lipid levels in blood. Without limiting the invention to a single mechanism of action, it is believed that one way the compounds of the present combination may work in concert to provide improved control of atherosclerotic lesions by, for example, reducing inflammation of the blood vessels in concert with lowering blood lipid levels.

In another embodiment of the invention, the present compounds can be used in combination with other compounds or therapies for the treatment of central nervous conditions or disorders such as migraine. For example, the present compounds can be used in therapeutic combination with caffeine, a 5-HT-1B/1D agonist (for example, a triptan such as sumatriptan, naratriptan, zolmitriptan, rizatriptan, almotriptan, or frovatriptan), a dopamine D4 antagonist (e.g., sonepiprazole), aspirin, acetaminophen, ibuprofen, indomethacin, naproxen sodium, isometheptene, dichloralphenazone, butalbital, an ergot alkaloid (e.g., ergotamine, dihydroergotamine, bromocriptine, ergonovine, or methyl ergonovine), a tricyclic antidepressant (e.g., amitriptyline or nortriptyline), a serotonergic antagonist (e.g., methysergide or cyproheptadine), a beta-andrenergic antagonist (e.g., propranolol, timolol, atenolol, nadolol, or metprolol), or a monoamine oxidase inhibitor (e.g., phenylzine or isocarboxazid).

The present invention includes compounds that selectively inhibit IKK-2 over other kinases. Such other kinases include, but are not limited to, Abl(h), Abl(T315I), Abl(T315I), AMPK, Aurora-A, BTK, CAMKII, CaMKIV, CDK1/cyclinB, CDK2, CDK2/cyclin A, CDK2/cyclinE, CHK1, CHK2, CK1, CK1(y), CK1δ, CK2, c-RAF(h), CSK, cSRC(h), DYRK1a, ERK2, Fyn, GSK3β, IGF-1R, IKK1, IKKi, IKK2(h), JNK/SAPK1c, JNK1, JNK1α1(h), JNK2, JNK2α2(h), JNK3, Lck, MAPK1(h), MAPK2(h), MAPK2/ERK2, MAPKAP-K1a, MAPKAP-K2, MEK1, MK-2, MK-3, MKK1, MKK4, MKK6, MKK7, MKK7β(h), MNK, MRSK2/APKAPk1b, MSK, MSK1, NEK2a, NEK6, p38 alpha, p38 beta, p38 delta, p38 gamma, p70 S6K, PAK2, PDGFRβ, PDK1, PHK, PKA, PKBΔph, PKCζ, PKCα, PKCγ, PKCδ, PKCε, PRAK, ROCK-II, Rsk1, Rsk2, RSKB. SAPK2a/p38, SAPK2b, SAPK2b/p38β2, SAPK3, SAPK3/p38g, SAPK4, SAPK4/p38d, SGK, TBK-1, and ZAP-70. The compounds may have an IKK-2 IC₅₀ of less than about 10 µM, preferably less than about 1 µM, and have a selectivity ratio of IKK-2 inhibition over IKK-1 inhibition of at least 50, or at least 100. The compounds may have an IKK-1 IC₅₀ of greater than 10 µM, or greater than 100 µM.

In one preferred embodiment, the compound of Formula I is a compound wherein R^{a} and R^{c} are independently selected from the group consisting of hydrido, hydroxyl, C₁₋₆ alkoxy, C₁₋₆ alkyl, C₁₋₆ haloalkyl, C₃₋₁₂ aryl, and 3- to 12-membered heteroaryl; wherein R^{b} is a 3- to 12-membered cyclic moiety selected from the group consisting of C₃₋₁₂ cycloalkyl, C₃₋₁₂ cycloalkenyl, C₃₋₁₂ aryl, 3- to 12-membered heterocycloalkyl, 3- to 12-membered 3- to 12-membered heterocycloalkenyl, and 3- to 12-membered heteroaryl, wherein R^{b} is optionally substituted by one or more substituents selected from the group consisting of R², C₃₋₁₂ cycloalkyl, and C₄₋₁₈ cycloalkylalkyl; wherein R^{d} is selected from the group consisting of -(CH₂)_{q}NH₂, -(CH₂)_{q}NHR², and a 5- to 7-membered heterocycloalkyl having ring members selected from the group consisting of carbon and nitrogen, wherein said heterocycloalkyl may be optionally substituted by one or more substituents selected from the group consisting of R²; wherein R² is selected from the group consisting of halo, C₁₋₆ alkylsulfinyl, C₁₋₆ alkylsulfonyl, cyano, C₂₋₇ alkoxycarbonyl, C₁₋₆ alkyl, C₁₋₆ haloalkyl, C₁₋₆ hydroxyalkyl, C₁₋₆ haloalkoxy, nitro, C₂₋₁₀ acylamino, R⁷, -OR³, -(CH₂)ₘOR³, -(CH₂)ₚCO₂R³, -SR³, -SO₂N(R^{4a})R^{4b}, -NR^{5a}R^{5b}, -NR^{5a}COR^{5b}, -NR^{5a}CO(OR^{5b}), -NR^{5a}SO₂R⁶, -NR^{5a}SO₂N(R^{6a})R^{6b}, -NR^{5a}CON(R^{6a})R^{6b}, -COR^{5a}, and -CON(R^{4a})R^{4b}; wherein R³, R^{4a}, and R^{4b} are independently selected from the group consisting of hydrido, C₃₋₁₂ aryl, 3- to 12-membered heteroaryl, 4- to 18-membered heteroaralkyl, C₁₋₆ alkyl, C₁₋₆ haloalkyl, C₂₋₆ alkenyl, C₂₋₆ alkynyl, C₁₋₆ hydroxyalkyl, C₁₋₆ aminoalkyl, C₂₋₁₂ alkylaminoalkyl, N-N-di(C₁₋₆alkyl)amino(C₁₋₆alkyl), C₁₋₆ alkoxy, C₂₋₁₂ alkoxyalkyl, 3- to 12-membered heterocycloalkyl, 3- to 12-membered heterocycloalkenyl, C₃₋₁₂ cycloalkyl, C₄₋₁₈ cycloalkylalkyl, C₄₋₁₈ aralkyl, and C₄₋₁₈ aralkylamino, wherein said aryl is optionally substituted with one or more radicals selected from the group consisting of C₁₋₆ alkyl, C₁₋₆ aminoalkyl, C₁₋₆ alkoxy and halo, wherein R^{4a} and R^{4b} may be taken together to form a 3- to 7-membered heterocyclic ring having from 1 to 3 heteroatoms selected from S, SO, SO₂, O, N, and NR^{5a}; wherein R^{5a} and R^{5b} are independently selected from the group consisting of hydrido, C₁₋₆ alkyl, C₃₋₁₂ aryl, 3- to 12-membered heteroaryl, C₄₋₁₈ aralkyl, 3- to 12-membered heterocycloalkenyl, C₃₋₁₂ cycloalkyl, 3- to 12-membered heterocycloalkyl, C₁₋₆ haloalkyl, C₄₋₁₈ aralkylamino, amino, C₁₋₆ aminoalkyl, C₂₋₁₀ aminoacyl, nitro, azido, and 4- to 18-membered heteroaralkyl, wherein said alkyl, aryl, heteroaryl, aminoalkyl, and aralkyl moieties are optionally substituted with one or more substituents selected from the group consisting of C₁₋₆ alkylsulfonamido, sulfamyl, C₁₋₆ alkyl, C₁₋₆ alkylthio, C₁₋₆ alkylsulfinyl, C₁₋₆ alkylsulfonyl, N-(C₁₋₆ alkyl)amino, C₁₋₆ aminoalkyl, C₂₋₁₂ alkylaminoalkyl, C₁₋₆ alkoxy, halo, C₂₋₁₀ acyloxy, oxy, formyl, C₁₋₆ haloalkyl, cyano, C₁₋₆ haloalkoxy, C₂₋₁₀ acyl, carboxyl, hydroxy, C₁₋₆ hydroxyalkoxy, phenoxy, nitro, azido, benzyloxy, N,N-di(C₁₋₆ alkyl)amino(C₂₋₁₀ acyl), C₁₋₆ thioalkyl, C₂₋₁₀ aminoacyloxy, thiocyanato, isothiocyanato, C₁₋₆ alkyldioxy, C₁₋₆ hydroxyalkyl, N-(C₁₋₆ alkyl)amino, C₂₋₇ alkoxycarbonyl, C₂₋₁₂ alkoxyalkyl, C₂₋₆ alkenylamino, C₂₋₆ alkynylamino, C₂₋₆ alkenyl, C₂₋₆ alkynyl, N,N-di(C₁₋₆ alkyl)amino(C₁₋₆ alkoxy), 3- to 12-membered heterocycloalkenyl, 3- to 12-membered heterocycloalkyl, and 3- to 12-membered heteroaryl; wherein R^{6a} and R^{6b} are independently selected from the group consisting of hydrido, C₁₋₆ alkyl, 3-to 12-membered heteroaryl, 3- to 12-membered heterocycloalkenyl, C₁₋₆ haloalkyl, C₄₋₁₈ aralkylamino, 4- to 18-membered heteroaralkyl, C₃₋₁₂ aryl, and C₄₋₁₈ aralkyl, wherein said aryl, heteroaryl, heterocycloalkenyl, and aralkyl moieties are optionally substituted with one or more substituents selected from C₁₋₆ alkyl, C₁₋₆ alkoxy, halo, C₁₋₆ haloalkyl, cyano, C₁₋₆ haloalkoxy, C₂₋₁₀ acyl, carboxyl, hydroxy, C₁₋₆ hydroxyalkoxy, phenoxy, benzyloxy, N,N-di(C₁₋₆ alkyl)amino(C₁₋₆ alkoxy), 3- to 12-membered heterocycloalkenyl, 3- to 12-membered heterocycloalkyl, and 3- to 12-membered heteroaryl; wherein R^{6a} and R^{6b} may be taken together to form a 3- to 7-membered heterocyclic ring having 1 to 3 heteroatoms selected from S, SO, SO₂, O, N, and NR^{5a}; and wherein R⁷ is selected from the group consisting of C₃₋₁₂ aryl, 3- to 12-membered heterocycloalkenyl, 3- to 12-membered heteroaryl, and C₂₋₆ alkenyl, wherein R⁷ is optionally substituted with one or more substituents selected from the group consisting of R^{5a}; or a pharmaceutically acceptable salt thereof.

In one particularly preferred embodiment, the compound of Formula I is a compound wherein R^{a} and R^{c} are independently selected from the group consisting of hydrido, hydroxyl, methoxy, ethoxy, propoxy, butoxy, methyl, ethyl, propyl, butyl, pentyl, hexyl, chloromethyl, dichloromethyl, trichloromethyl, fluoromethyl, difluoromethyl, trifluoromethyl, phenyl, biphenyl, naphthyl, indenyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl; wherein R^{b} is a 3-to 12-membered cyclic moiety selected from the group consisting of cyclopropyl, cyclobutyl, cyclopentyl, cyclohexyl, cyclopropenyl, cyclobutenyl, cyclopentenyl, cyclohexenyl, phenyl, biphenyl, naphthyl, indenyl, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl, wherein R^{b} is optionally substituted by one or more substituents selected from the group consisting of R², cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl, and cyclohexylethyl; wherein R^{d} is selected from the group consisting of -(CH₂)_{q}NH₂, -(CH₂)_{q}NHR², piperidinyl, pyrrolidinyl, pyrazolidinyl, and imidazolidinyl, wherein said piperidinyl, pyrrolidinyl, pyrazolidinyl, or imidazolidinyl may be optionally substituted by one or more substituents selected from the group consisting of R²; wherein R² is selected from the group consisting of chloro, fluoro, bromo, iodo, methylsulfinyl, ethylsulfinyl, propylsulfinyl, butylsulfinyl, methylsulfonyl, ethylsulfonyl, propylsulfonyl, butylsulfonyl, cyano, methoxycarbonyl, ethoxycarbonyl, propoxycarbonyl, butoxycarbonyl, methyl, ethyl, propyl, butyl, pentyl, hexyl, chloromethyl, dichloromethyl, trichloromethyl, fluoromethyl, difluoromethyl, trifluoromethyl, hydroxymethyl, hydroxyethyl, hydroxypropyl, hydroxybutyl, hydroxypentyl, hydroxyhexyl, chloromethoxy, dichloromethoxy, trichloromethoxy, fluoromethoxy, difluoromethoxy, trifluoromethoxy, nitro, methylcarbonylamino, ethylcarbonylamino, propylcarbonylamino, butylcarbonylamino, pentylcarbonylamino, hexylcarbonylamino, phenylcarbonylamino, benzylcarbonylamino, R⁷, -OR³, -(CH₂)ₘOR³, -(CH₂)ₚCO₂R³, -SR³, -SO₂N(R^{4a})R^{4b}, -NR^{5a}R^{5b}, -NR^{5a}COR^{5b}, -NR^{5a}CO(OR^{5b}), -NR^{5a}SO₂R⁶, -NR^{5a}SO₂N(R^{6a})R^{6b}, -NR^{5a}CON(R^{5a})R^{6b}, -COR^{5a}, and -CON(R^{4a})R^{4b}; wherein R³, R^{4a}, and R^{4b} are independently selected from the group consisting of hydrido, phenyl, biphenyl, naphthyl, indenyl, pyridinyl, benzothiophenyl, indolyl, isoquinolinyl, quinolinyl, thienyl, pyrrolyl, furyl, pyrazolyl, imidazolyl, isoxazolyl, oxazolyl, isoindoledionyl, pyridinylmethyl, pyridinylethyl, benzothiophenylmethyl, benzothiophenylethyl, indolylmethyl, indolylethyl, isoquinolinylmethyl, isoquinolinylethyl, quinolinylmethyl, quinolinylethyl, thienylmethyl, thienylethyl, pyrrolylmethyl, pyrrolylethyl, furylmethyl, furylethyl, pyrazolylmethyl, pyrazolylethyl, imidazolylmethyl, imidazolylethyl, isoxazolylmethyl, isoxazolylethyl, oxazolylmethyl, oxazolylethyl, isoindoledionylmethyl, isoindoledionylethyl, methyl, ethyl, propyl, butyl, pentyl, hexyl, chloromethyl, dichloromethyl, trichloromethyl, fluoromethyl, difluoromethyl, trifluoromethyl, ethenyl, propenyl, butenyl, pentenyl, ethynyl, propynyl, butynyl, pentynyl, hydroxymethyl, hydroxyethyl, hydroxypropyl, hydroxybutyl, hydroxypentyl, hydroxyhexyl, aminomethyl, aminoethyl, aminopropyl, aminobutyl, aminopentyl, aminohexyl, methylaminomethyl, ethylaminomethyl, propylaminomethyl, methylaminoethyl, ethylaminoethyl, propylaminoethyl, methylaminopropyl, ethylaminopropyl, propylaminopropyl, methylaminobutyl, ethylaminobutyl, propylaminobutyl, methylaminopentyl, ethylaminopentyl, propylaminopentyl, methylaminohexyl, ethylaminohexyl, propylaminohexyl, N,N-dimethylaminomethyl, N,N-dimethylaminoethyl, N-methyl-N-ethylaminomethyl, N-methyl-N-ethylaminoethyl, N-methyl-N-propylaminomethyl, N-methyl-N-propylaminoethyl, N,N-diethylaminomethyl, N,N-diethylaminoethyl, N-ethyl-N-propylaminomethyl, N-ethyl-N-propylaminoethyl, N,N-dipropylaminomethyl, N,N-dipropylaminoethyl, methoxy, ethoxy, propoxy, butoxy, methoxymethyl, methoxyethyl, methoxypropyl, ethoxymethyl, ethoxyethyl, ethoxypropyl, propoxymethyl, propoxyethyl, propoxypropyl, butoxymethyl, butoxyethyl, butoxypropyl, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl, cyclohexylethyl, benzyl, phenylethyl, benzylamino, and phenylethylamino, wherein said phenyl, biphenyl, naphthyl, or indenyl moiety is optionally substituted with one or more radicals selected from the group consisting of methyl, ethyl, propyl, butyl, pentyl, hexyl, aminomethyl, aminoethyl, aminopropyl, aminobutyl, aminopentyl, aminohexyl, methoxy, ethoxy, propoxy, butoxy, chloro, fluoro, bromo, and iodo, wherein R^{4a} and R^{4b} may be taken together to form a moiety selected from the group consisting of piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl; wherein R^{5a} and R^{5b} are independently selected from the group consisting of hydrido, methyl, ethyl, propyl, butyl, pentyl, hexyl, phenyl, biphenyl, naphthyl, indenyl, pyridinyl, benzothiophenyl, indolyl, isoquinolinyl, quinolinyl, thienyl, pyrrolyl, furyl, pyrazolyl, imidazolyl, isoxazolyl, oxazolyl, isoindoledionyl, benzyl, phenylethyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, cyclopropyl, cyclobutyl, cyclopentyl, cyclohexyl, piperidinyl, pyrrolidinyl, pyrazolidinyl, imidazolidinyl, isoxazolidinyl, oxazolidinyl, chloromethyl, dichloromethyl, trichloromethyl, fluoromethyl, difluoromethyl, trifluoromethyl, benzylamino, phenylethylamino, amino, aminomethyl, aminoethyl, aminopropyl, aminobutyl, aminopentyl, aminohexyl, aminomethylcarbonyl, aminoethylcarbonyl, aminopropylcarbonyl, aminobutylcarbonyl, aminopentylcarbonyl, aminohexylcarbonyl, aminophenylcarbonyl, aminobenzylcarbonyl, nitro, azido, pyridinylmethyl, pyridinylethyl, benzothiophenylmethyl, benzothiophenylethyl, indolylmethyl, indolylethyl, isoquinolinylmethyl, isoquinolinylethyl, quinolinylmethyl, quinolinylethyl, thienylmethyl, thienylethyl, pyrrolylmethyl, pyrrolylethyl, furylmethyl, furylethyl, pyrazolylmethyl, pyrazolylethyl, imidazolylmethyl, imidazolylethyl, isoxazolylmethyl, isoxazolylethyl, oxazolylmethyl, oxazolylethyl, isoindoledionylmethyl, and isoindoledionylethyl, wherein said methyl, ethyl, propyl, butyl, pentyl, hexyl, phenyl, biphenyl, naphthyl, indenyl, pyridinyl, benzothiophenyl, indolyl, isoquinolinyl, quinolinyl, thienyl, pyrrolyl, furyl, pyrazolyl, imidazolyl, isoxazolyl, oxazolyl, isoindoledionyl, aminomethyl, aminoethyl, aminopropyl, aminobutyl, aminopentyl, aminohexyl, benzyl, and phenylethyl moieties are optionally substituted with one or more substituents selected from the group consisting of methylsulfonamido, ethylsulfonamido, propylsulfonamido, butylsulfonamido, sulfamyl, methyl, ethyl, propyl, butyl, pentyl, hexyl, methylthio, ethylthio, propylthio, butylthio, methylsulfinyl, ethylsulfinyl, propylsulfinyl, butylsulfinyl, methylsulfonyl, ethylsulfonyl, propylsulfonyl, butylsulfonyl, N-methylamino, N-ethylamino, N-propyfamino, aminomethyl, aminoethyl, aminopropyl, aminobutyl, aminopentyl, aminohexyl, methylaminomethyl, ethylaminomethyl, propylaminomethyl, methylaminoethyl, ethylaminoethyl, propylaminoethyl, methylaminopropyl, ethylaminopropyl, propylaminopropyl, methylaminobutyl, ethylaminobutyl, propylaminobutyl, methylaminopentyl, ethylaminopentyl, propylaminopentyl, methylaminohexyl, ethylaminohexyl, propylaminohexyl, methoxy, ethoxy, propoxy, butoxy, chloro, fluoro, bromo, iodo, acyloxy, oxy, formyl, chloromethyl, dichloromethyl, trichloromethyl, fluoromethyl, difluoromethyl, trifluoromethyl, cyano, chloromethoxy, dichloromethoxy, trichloromethoxy, fluoromethoxy, difluoromethoxy, trifluoromethoxy, methylcarbonyl, ethylcarbonyl, propylcarbonyl, butylcarbonyl, pentylcarbonyl, hexylcarbonyl, phenylcarbonyl, benzylcarbonyl, carboxyl, hydroxy, hydroxymethoxy, hydroxyethoxy, hydroxypropoxy, hydroxybutoxy, phenoxy, nitro, azido, benzyloxy, N,N-dimethylaminomethylcarbonyl, N,N-dimethylaminoethylcarbonyl, N,N-dimethylarninophenylcarbcnyl, N-methyl-N-ethylaminomethylcarbonyl, N-methyl-N-ethylaminoethylcarbonyl, N-methyl-N-ethylaminophenylcarbonyl, N-methyl-N-propylaminomethylcarbonyl, N-methyl-N-propylaminoethylcarbonyl, N-methyl-N-propylaminophenylcarbonyl, N,N-diethylaminomethylcarbonyl, N,N-diethylaminoethylcarbonyl, N,N-diethylaminophenylcarbonyl, N-ethyl-N-propylaminomethylcarbonyl, N-ethyl-N-propylaminoethylcarbonyl, N-ethyl-N-propylaminophenylcarbonyl, N,N-dipropylaminomethylcarbonyl, N,N-dipropylaminoethylcarbonyl, N,N-dipropylaminophenylcarbonyl, thiomethyl, thioethyl, thiopropyl, thiobutyl, thiopentyl, thiohexyl, aminomethylcarbonyloxy, aminoethylcarbonyloxy, aminopropylcarbonyloxy, aminobutylcarbonyloxy, aminopentylcarbonyloxy, aminohexylcarbonyloxy, aminophenylcarbonyloxy, aminobenzylcarbonyloxy, thiocyanato, isothiocyanato, methyldioxy, ethyldioxy, propyldioxy, butyldioxy, pentyldioxy, hexyldioxy, hydroxymethyl, hydroxyethyl, hydroxypropyl, hydroxybutyl, hydroxypentyl, hydroxyhexyl, N-methylamino, N-ethylamino, N-propylamino, methoxycarbonyl, ethoxycarbonyl, propoxycarbonyl, butoxycarbonyl, methoxymethyl, methoxyethyl, methoxypropyl, ethoxymethyl, ethoxyethyl, ethoxypropyl, propoxymethyl, propoxyethyl, propoxypropyl, butoxymethyl, butoxyethyl, butoxypropyl, ethenylamino, propenylamino, butenylamino, pentenylamino, ethynylamino, propynylamino, butynylamino, pentynylamino, ethenyl, propenyl, butenyl, pentenyl, ethynyl, propynyl, butynyl, pentynyl, N,N-dimethylaminomethoxy, N,N-dimethylaminoethoxy, N-methyl-N-ethylaminomethoxy, N-methyl-N-ethylaminoethoxy, N-methyl-N-propylaminomethoxy, N-methyl-N-propylaminoethoxy, N,N-diethylaminomethoxy, N,N-diethylaminoethoxy, N-ethyl-N-propylaminomethoxy, N-ethyl-N-propylaminoethoxy, N,N-dipropylaminomethoxy, N,N-dipropylaminoethoxy, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazoiyi, imidazolyl, isoxazolyl, oxazolyl, and isoindoledionyl; wherein R^{6a} and R^{6b} are independently selected from the group consisting of hydrido, methyl, ethyl, propyl, butyl, pentyl, hexyl, pyridinyl, benzothiophenyl, indolyl, isoquinolinyl, quinolinyl, thienyl, pyrrolyl, furyl, pyrazolyl, imidazolyl, isoxazolyl, oxazolyl, isoindoledionyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, chloromethyl, dichloromethyl, trichloromethyl, fluoromethyl, difluoromethyl, trifluoromethyl, benzylamino, phenylethylamino, pyridinylmethyl, pyridinylethyl, benzothiophenylmethyl, benzothiophenylethyl, indolylmethyl, indolylethyl, isoquinolinylmethyl, isoquinolinylethyl, quinolinylmethyl, quinolinylethyl, thienylmethyl, thienylethyl, pyrrolylmethyl, pyrrolylethyl, furylmethyl, furylethyl, pyrazolylmethyl, pyrazolylethyl, imidazolylmethyl, imidazolylethyl, isoxazolylmethyl, isoxazolylethyl, oxazolylmethyl, oxazolylethyl, isoindoledionylmethyl, isoindoledionylethyl, phenyl, biphenyl, naphthyl, indenyl, benzyl, and phenylethyl, wherein said phenyl, biphenyl, naphthyl, indenyl, pyridinyl, benzothiophenyl, indolyl, isoquinolinyl, quinolinyl, thienyl, pyrrolyl, furyl, pyrazolyl, imidazolyl, isoxazolyl, oxazolyl, isoindoledionyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, benzyl, and phenylethyl moieties are optionally substituted with one or more substituents selected from methyl, ethyl, propyl, butyl, pentyl, hexyl, methoxy, ethoxy, propoxy, butoxy, chloro, fluoro, bromo, iodo, chloromethyl, dichloromethyl, trichloromethyl, fluoromethyl, difluoromethyl, trifluoromethyl, cyano, chloromethoxy, dichloromethoxy, trichloromethoxy, fluoromethoxy, difluoromethoxy, trifluoromethoxy, methylcarbonyl, ethylcarbonyl, propylcarbonyl, butylcarbonyl, pentylcarbonyl, hexylcarbonyl, phenylcarbonyl, benzylcarbonyl, carboxyl, hydroxy, hydroxymethoxy, hydroxyethoxy, hydroxypropoxy, hydroxybutoxy, phenoxy, benzyloxy, N,N-dimethylaminomethoxy, N,N-dimethylaminoethoxy, N-methyl-N-ethylaminomethoxy, N-methyl-N-ethylaminoethoxy, N-methyl-N-propylaminomethoxy, N-methyl-N-propylaminoethoxy, N,N-diethylaminomethoxy, N,N-diethylaminoethoxy, N-ethyl-N-propylaminomethoxy, N-ethyl-N-propylaminoethoxy, N,N-dipropylaminomethoxy, N,N-dipropylaminoethoxy, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl; wherein R^{6a} and R^{6b} may be taken together to form a moiety selected from the group consisting of piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl; and wherein R⁷ is selected from the group consisting of phenyl, biphenyl, naphthyl, indenyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, isoindoledionyl, ethenyl, propenyl, butenyl, and pentenyl, wherein R⁷ is optionally substituted with one or more substituents selected from the group consisting of R^{5a}; or a pharmaceutically acceptable salt thereof.

In a particularly preferred embodiment, the compound of Formula I is a compound of Formula II:

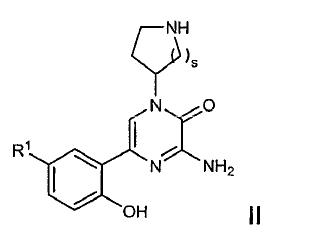

wherein s is 1, 2, or 3;

wherein R¹ is selected from the group consisting of hydrido, -OR³, C₁₋₆ alkyl, C₅₋₇ cycloalkyl, benzyl, -CH₂(C₃₋₇ cycloalkyl), aryl, halo, heterocycloalkyl, heterocycloalkenyl, and heteroaryl;

wherein R³ is selected from the group consisting of hydrido, C₁₋₆ alkyl, C₅₋₇ cycloalkyl, benzyl, -CH₂(C₃₋₇ cycloalkyl), and aryl;

or a pharmaceutically acceptable salt thereof.

In one preferred embodiment, the compound of Formula II is a compound wherein R¹ is selected from the group consisting of hydrido, -OR³, C₁₋₆ alkyl, C₅₋₇ cycloalkyl, benzyl, -CH₂(C₃₋₇ cycloalkyl), C₃₋₁₂ aryl, halo, 3- to 12-membered heterocycloalkyl, 3- to 12-membered heterocycloalkenyl, and 3- to 12-membered heteroaryl; and wherein R³ is selected from the group consisting of hydrido, C₁₋₆ alkyl, C₅₋₇ cycloalkyl, benzyl, -CH₂(C₃₋₇ cycloalkyl), and C₃₋₁₂ aryl; or a pharmaceutically acceptable salt thereof.

In one particularly preferred embodiment, the compound of Formula 11 is a compound wherein R¹ is selected from the group consisting of hydrido, -OR³, methyl, ethyl, propyl, butyl, pentyl, hexyl, cyclopentyl, cyclohexyl, cycloheptyl, benzyl, methylcyclopropyl, methylcyclobutyl, methylcyclopentyl, methylcyclohexyl, phenyl, biphenyl, naphthyl, indenyl, chloro, fluoro, bromo, iodo, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl; and wherein R³ is selected from the group consisting of hydrido, methyl, ethyl, propyl, butyl, pentyl, hexyl, cyclopentyl, cyclohexyl, cycloheptyl, benzyl, methylcyclopropyl, methylcyclobutyl, methylcyclopentyl, methylcyclohexyl, phenyl, biphenyl, naphthyl, and indenyl; or a pharmaceutically acceptable salt thereof.

In a particularly preferred embodiment, the compound of Formula I is a compound of Formula III:

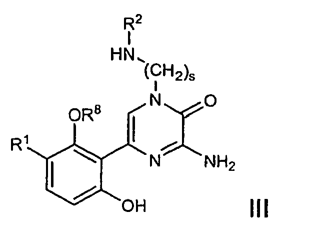

wherein s is 1, 2, or 3;

wherein R¹ is selected from the group consisting of hydrido, -OR³, C₁₋₆ alkyl, C₅₋₇ cycloalkyl, benzyl, -CH₂(C₃₋₇ cycloalkyl), aryl, halo, heterocycloalkyl, heterocycloalkenyl, and heteroaryl;

wherein R² is hydrido or C₁₋₆ alkyl;

wherein R³ is selected from the group consisting of C₁₋₆ alkyl, C₅₋₇ cycloalkyl, benzyl, -CH₂(C₃₋₇ cycloalkyl), and aryl; and

wherein R⁸ is selected from the group consisting of C₁₋₆ alkyl, C₅₋₇ cycloalkyl, benzyl, and -CH₂(C₃₋₇ cycloalkyl);

or a pharmaceutically acceptable salt thereof.

In one preferred embodiment, the compound of Formula III is a compound wherein R¹ is selected from the group consisting of hydrido, -OR³, C₁₋₆ alkyl, C₅₋₇ cycloalkyl, benzyl, -CH₂(C₃₋₇ cycloalkyl), C₃₋₁₂ aryl, halo, 3- to 12-membered heterocycloalkyl, 3- to 12-membered heterocycloalkenyl, and 3- to 12-membered heteroaryl; wherein R² is hydrido or C₁₋₆ alkyl; wherein R³ is selected from the group consisting of C₁₋₆ alkyl, C₅₋₇ cycloalkyl, benzyl, -CH₂(C₃₋₇ cycloalkyl), and C₃₋₁₂ aryl; and wherein R⁸ is selected from the group consisting of C₁₋₆ alkyl, C₅₋₇ cycloalkyl, benzyl, and -CH₂(C₃₋₇ cycloalkyl); or a pharmaceutically acceptable salt thereof.

In one particularly preferred embodiment, the compound of Formula III is a compound wherein R¹ is selected from the group consisting of hydrido, -OR³, methyl, ethyl, propyl, butyl, pentyl, hexyl, cyclopentyl, cyclohexyl, cycloheptyl, benzyl, methylcyclopropyl, methylcyclobutyl, methylcyclopentyl, methylcyclohexyl, phenyl, biphenyl, naphthyl, indenyl, chloro, fluoro, bromo, iodo, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl; wherein R² is selected from the group consisting of hydrido, methyl, ethyl, propyl, butyl, pentyl, and hexyl; wherein R³ is selected from the group consisting of methyl, ethyl, propyl, butyl, pentyl, hexyl, cyclopentyl, cyclohexyl, cycloheptyl, benzyl, methylcyclopropyl, methylcyclobutyl, methylcyclopentyl, methylcyclohexyl, phenyl, biphenyl, naphthyl, and indenyl; and wherein R⁸ is selected from the group consisting of methyl, ethyl, propyl, butyl, pentyl, hexyl, cyclopentyl, cyclohexyl, cycloheptyl, benzyl, methylcyclopropyl, methylcyclobutyl, methylcyclopentyl, and methylcyclohexyl; or a pharmaceutically acceptable salt thereof.

In another particularly preferred embodiment, the compound of Formula I is a compound of Formula IV:

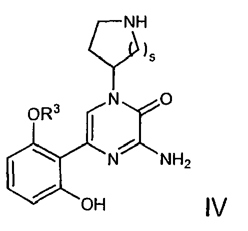

wherein s is 1, 2, or 3; and

wherein R³ is selected from the group consisting of hydrido, C₁₋₆ alkyl, C₅₋₇ cycloalkyl, benzyl, and -CH₂(C₃₋₇ cycloalkyl);

or a pharmaceutically acceptable salt thereof.

In one particularly preferred embodiment, the compound of Formula IV is a compound wherein R³ is selected from the group consisting of hydrido, methyl, ethyl, propyl, butyl, pentyl, hexyl, cyclopentyl, cyclohexyl, cycloheptyl, benzyl, methylcyclopropyl, methylcyclobutyl, methylcyclopentyl, and methylcyclohexyl; or a pharmaceutically acceptable salt thereof.

In another particularly preferred embodiment, the compound of Formula I is a compound of Formula V:

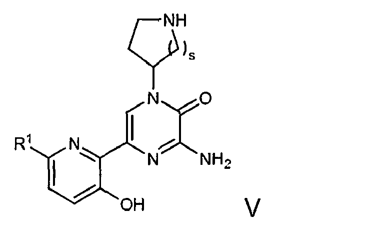

wherein s is 1, 2, or 3;

wherein R¹ is selected from the group consisting of hydrido, -OR³, C₁₋₆alkyl, C₅₋₇ cycloalkyl, benzyl, -CH₂(C₃₋₇ cycloalkyl), aryl, halo, heterocycloalkyl, heterocycloalkenyl, and heteroaryl; and

wherein R³ is selected from the group consisting of C₁₋₆ alkyl, C₅₋₇ cycloalkyl, benzyl, -CH₂(C₃₋₇ cycloalkyl), and aryl;

or a pharmaceutically acceptable salt thereof.

In one preferred embodiment, the compound of Formula V is a compound wherein R¹ is selected from the group consisting of hydrido, -OR³, C₁₋₆ alkyl, C₅₋₇ cycloalkyl, benzyl, -CH₂(C₃₋₇ cycloalkyl), C₃₋₁₂ aryl, halo, 3- to 12-membered heterocycloalkyl, 3- to 12-membered heterocycloalkenyl, and 3- to 12-membered heteroaryl; and wherein R³ is selected from the group consisting of C₁₋₆ alkyl, C₅₋₇ cycloalkyl, benzyl, -CH₂(C₃₋₇ cycloalkyl), and C₃₋₁₂ aryl; or a pharmaceutically acceptable salt thereof.

In one particularly preferred embodiment, the compound of Formula V is a compound wherein R¹ is selected from the group consisting of hydrido, -OR³, methyl, ethyl, propyl, butyl, pentyl, hexyl, cyclopentyl, cyclohexyl, cycloheptyl, benzyl, methylcyclopropyl, methylcyclobutyl, methylcyclopentyl, methylcyclohexyl, phenyl, biphenyl, naphthyl, indenyl, chloro, fluoro, bromo, iodo, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl; and wherein R³ is selected from the group consisting of methyl, ethyl, propyl, butyl, pentyl, hexyl, cyclopentyl, cyclohexyl, cycloheptyl, benzyl, methylcyclopropyl, methylcyclobutyl, methylcyclopentyl, methylcyclohexyl, phenyl, biphenyl, naphthyl, and indenyl; or a pharmaceutically acceptable salt thereof.

In another particularly preferred embodiment, the compound of Formula I is a compound of Formula VI:

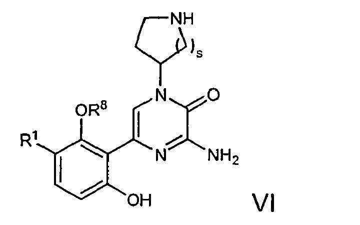

wherein s is 1, 2, or 3;

wherein R¹ is selected from the group consisting of hydrido, -OR³, C₁₋₆ alkyl, C₅₋₇ cycloalkyl, benzyl, -CH₂(C₃₋₇ cycloalkyl), aryl, halo, heterocycloalkyl, heterocycloalkenyl, and heteroaryl;

wherein R³ is selected from the group consisting of C₁₋₆ alkyl, C₅₋₇ cycloalkyl, benzyl, -CH₂(C₃₋₇ cycloalkyl), and aryl; and

wherein R⁸ is selected from the group consisting of C₁₋₆ alkyl, C₅₋₇ cycloalkyl, benzyl, and -CH₂(C₃₋₇ cycloalkyl);

or a pharmaceutically acceptable salt thereof.

In one preferred embodiment, the compound of Formula VI is a compound wherein R¹ is selected from the group consisting of hydrido, -OR³, C₁₋₆ alkyl, C₅₋₇ cycloalkyl, benzyl, -CH₂(C₃₋₇ cycloalkyl), C₃₋₁₂ aryl, halo, 3- to 12-membered heterocycloalkyl, 3- to 12-membered heterocycloalkenyl, and 3- to 12-membered heteroaryl; wherein R³ is selected from the group consisting of C₁₋₆ alkyl, C₅₋₇ cycloalkyl, benzyl, -CH₂(C₃₋₇ cycloalkyl), and C₃₋₁₂ aryl; and wherein R⁸ is selected from the group consisting of C₁₋₆ alkyl, C₅₋₇ cycloalkyl, benzyl, and -CH₂(C₃₋₇ cycloalkyl); or a pharmaceutically acceptable salt thereof.

In one particularly preferred embodiment, the compound of Formula VI is a compound wherein R¹ is selected from the group consisting of hydrido, -OR³, methyl, ethyl, propyl, butyl, pentyl, hexyl, cyclopentyl, cyclohexyl, cycloheptyl, benzyl, methylcyclopropyl, methylcyclobutyl, methylcyclopentyl, methylcyclohexyl, phenyl, biphenyl, naphthyl, indenyl, chloro, fluoro, bromo, iodo, piperidinyl, pyrrolidinyl, pyrazolidinyl, imidazolidinyl, isoxazolidinyl oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl; wherein R³ is selected from the group consisting of methyl, ethyl, propyl, butyl, pentyl, hexyl, cyclopentyl, cyclohexyl, cycloheptyl, benzyl, methylcyclopropyl, methylcyclobutyl, methylcyclopentyl, methylcyclohexyl, phenyl, biphenyl, naphthyl, and indenyl; and wherein R⁸ is selected from the group consisting of methyl, ethyl, propyl, butyl, pentyl, hexyl, cyclopentyl, cyclohexyl, cycloheptyl, benzyl, methylcyclopropyl, methylcyclobutyl, methylcyclopentyl, and methylcyclohexyl; or a pharmaceutically acceptable salt thereof.

In another particularly preferred embodiment, the compound of Formula I is a compound of Formula VII:

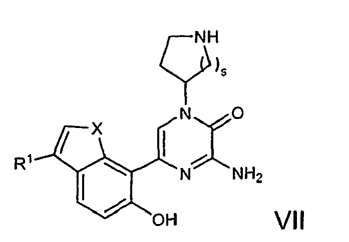

wherein s is 1, 2, or 3;

wherein X is O or S;

wherein R¹ is -(CH₂)ₘOR³ or -(CH₂)ₚCO₂R³;

wherein m is 1, 2, or 3;

wherein p is 0, 1, or 2; and

wherein R³ is hydrido or C₁₋₆ alkyl;

or a pharmaceutically acceptable salt thereof.

In one particularly preferred embodiment, the compound of Formula VII is a compound wherein R³ is selected from the group consisting of hydrido, methyl, ethyl, propyl, butyl, pentyl, and hexyl; or a pharmaceutically acceptable salt thereof.

In a particularly preferred embodiment, the compound of Formula I is selected from the group of compounds shown in Table I below:

**Table I**

| **Example** | **Structure** | **Name** |
|---|---|---|
| 1 | 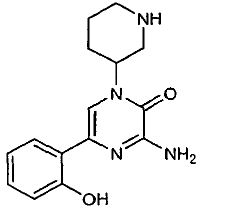 | 3-amino-5-(2-hydroxyphenyl)-1-piperidin-3-ylpyrazin-2(1H)-one |
| 2 | 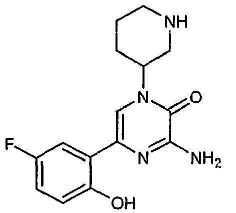 | 3-amino-5-(5-fluoro-2-hydroxyphenyl)-1-piperidin-3-ylpyrazin-2(1H)-one |
| 3 | 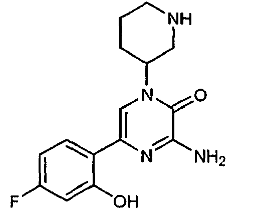 | 3-amino-5-(4-fluoro-2-hydroxyphenyl)-1-piperidin-3-ylpyrazin-2(1H)-one |
| 4 | 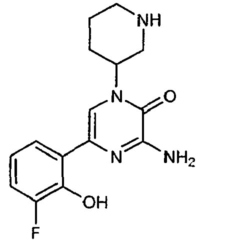 | 3-amino-5-(3-fluoro-2-hydroxyphenyl)-1-piperidin-3-ylpyrazin-2(1H)-one |
| 5 | 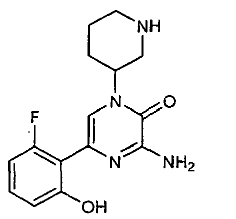 | 3-amino-5-(2-fluorc-6-hydroxyphenyl)-1-piperidin-3-ylpyrazin-2(1H)-one |
| 6 | 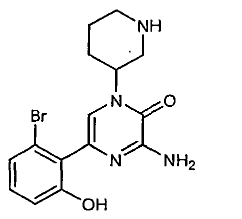 | 3-amino-5-(2-bromo-6-hydroxyphenyl)-1-piperidin-3-ylpyrazin-2(1H)-one |
| 7 | 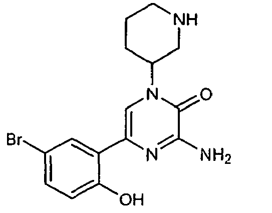 | 3-amino-5-(5-bromo-2-hydroxyphenyl)-1-piperidin-3-ylpyrazin-2(1H)-one |
| 8 | 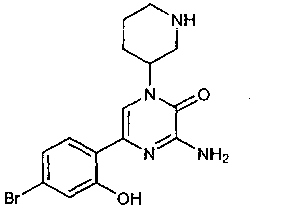 | 3-amino-5-(4-bromo-2-hydroxyphenyl)-1-piperidin-3-ylpyrazin-2(1H)-one |
| 9 | 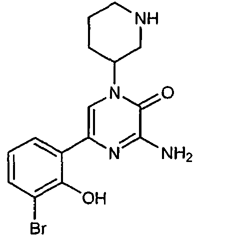 | 3-amino-5-(3-bromo-2-hydroxyphenyl)-1-piperidin-3-ylpyrazin-2(1H)-one |
| 10 | 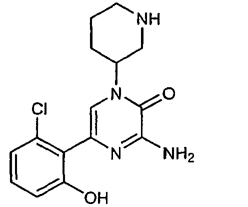 | 3-amino-5-(2-chloro-6-hydroxyphenyl)-1-piperidin-3-ylpyrazin-2(1H)-one |
| 11 | 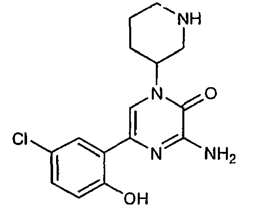 | 3-amino-5-(5-chloro-2-hydroxyphenyl)-1-piperidin-3-ylpyrazin-2(1H)-one |
| 12 | 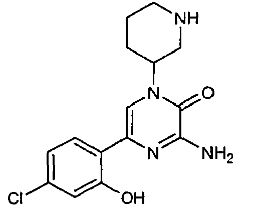 | 3-amino-5-(4-chloro-2-hydroxyphenyl)-1-piperidin-3-ylpyrazin-2(1H)-one |
| 13 | 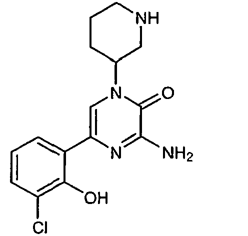 | 3-amino-5-(3-chloro-2-hydroxyphenyl)-1-piperidin-3-ylpyrazin-2(1H)-one |
| 14 | 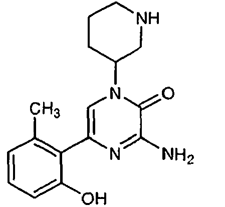 | 3-amino-5-(2-hydroxy-6-methylphenyl)-1-piperidin-3-ylpyrazin-2(1H)-one |
| 15 | 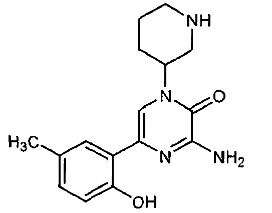 | 3-amino-5-(2-hydroxy-5-methylphenyl)-1-piperidin-3-ylpyrazin-2(1H)-one |
| 16 | 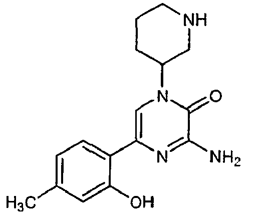 | 3-amino-5-(2-hydroxy-4-methylphenyl)-1-piperidin-3-ylpyrazin-2(1H)-one |
| 17 | 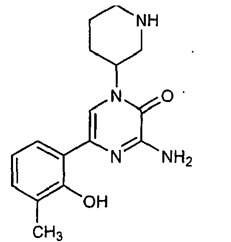 | 3-amino-5-(2-hydroxy-3-methylphenyl)-1-piperidin-3-ylpyrazin-2(1H)-one |
| 18 | 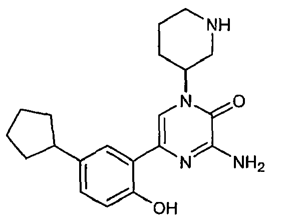 | 3-amino-5-(5-cyclopentyl-2-hydroxyphenyl)-1-piperidin-3-ylpyrazin-2(1H)-one |
| 19 | 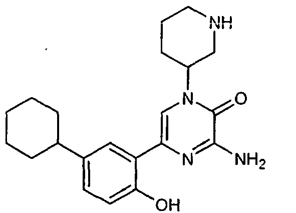 | 3-amino-5-(5-cyclohexyl-2-hydroxyphenyl)-1-piperidin-3-ylpyrazin-2(1H)-one |
| 20 | 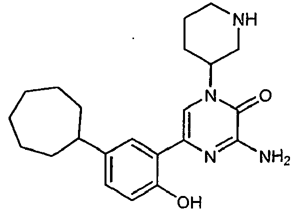 | 3-amino-5-(5-cycloheptyl-2-hydroxyphenyl)-1-piperidin-3-ylpyrazin-2(1H)-one |
| 21 | 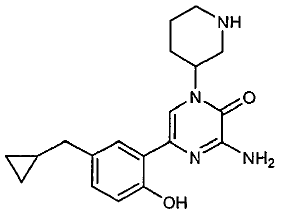 | 3-amino-5-(5-cyclopropylmethyl-2-hydroxyphenyl)-1-piperidin-3-ylpyrazin-2(1H)-one |
| 22 | 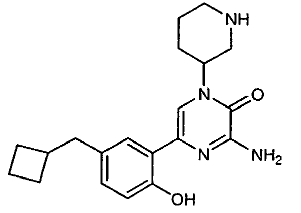 | 3-amino-5-(5-cyclobutylmethyl-2-hydroxyphenyl)-1-piperidin-3-ylpyrazin-2(1 H)-one |
| 23 | 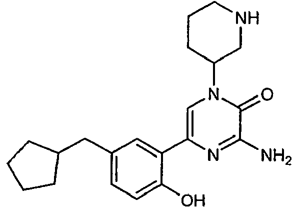 | 3-amino-5-(5-cyclopentylmethyl-2-hydroxyphenyl)-1-piperidin-3-ylpyrazin-2(1H)-one |
| 24 | 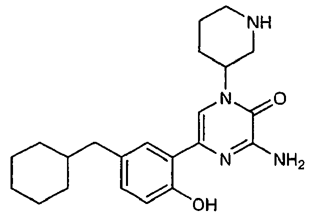 | 3-amino-5-(5-cyclohexylmethyl-2-hydroxyphenyl)-1-piperidin-3-ylpyrazin-2(1H)-one |
| 25 | 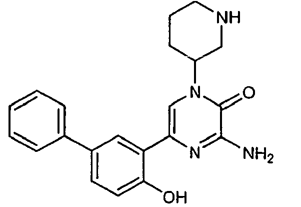 | 3-amino-5-(5-phenyl-2-hydroxyphenyl)-1-piperidin-3-ylpyrazin-2(1H)-one |
| 26 | 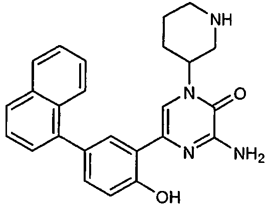 | 3-amino-5-[2-hydroxy-5-(1-naphthyl)phenyl]-1-piperidin-3-ylpyrazin-2(1H)-one |
| 27 | 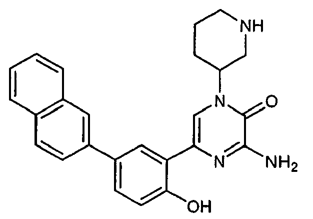 | 3-amino-5-[2-hydroxy-5-(2-naphthyl)phenyl]-1-piperidin-3-ylpyrazin-2(1H)-one |
| 28 | 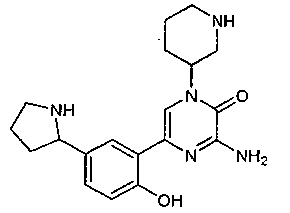 | 3-amino-5-(2-hydroxy-5-pyrrolidin-2-ylphenyl)-1-piperidin-3-ylpyrazin-2(1H)-one |
| 29 | 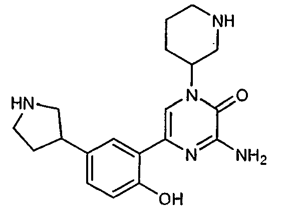 | 3-amino-5-(2-hydroxy-5-pyrrolidin-3-ylphenyl)-1-piperidin-3-ylpyrazin-2(1H)-one |
| 30 | 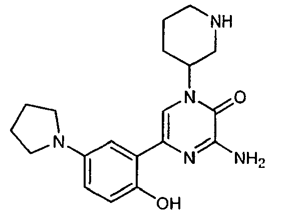 | 3-amino-5-(2-hydroxy-5-pyrrolidin-1-ylphenyl)-1-piperidin-3-ylpyrazin-2(1H)-one |
| 31 | 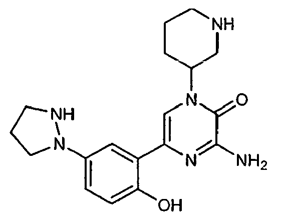 | 3-amino-5-(2-hydroxy-5-pyrazolidin-1-ylphenyl)-1-piperidin-3-ylpyrazin-2(1H)-one |
| 32 | 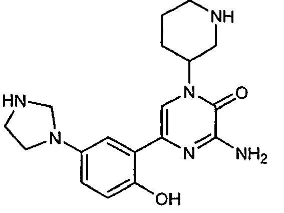 | 3-amino-5-(2-hydroxy-5-imidazolidin-1-ylphenyl)-1-piperidin-3-ylpyrazin-2(1H)-one |
| 33 | 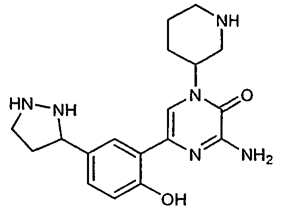 | 3-amino-5-(2-hydroxy-5-pyrazolidin-3-ylphenyl)-1-piperidin-3-ylpyrazin-2(1H)-one |
| 34 | 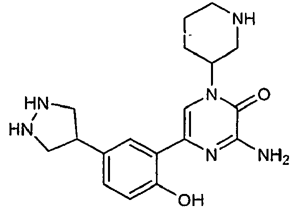 | 3-amino-5-(2-hydroxy-5-pyrazolidin-4-ylphenyl)-1-piperidin-3-ylpyrazin-2(1H)-one |
| 35 | 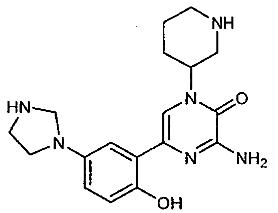 | 3-amino-5-(2-hydroxy-5-imidazolidin-1-ylphenyl)-1-piperidin-3-ylpyrazin-2(1H)-one |
| 36 | 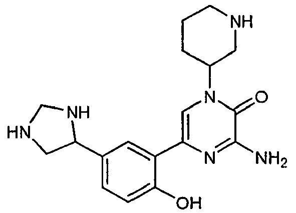 | 3-amino-5-(2-hydroxy-5-imidazolidin-4-ylphenyl)-1-piperidin-3-ylpyrazin-2(1H)-one |
| 37 | 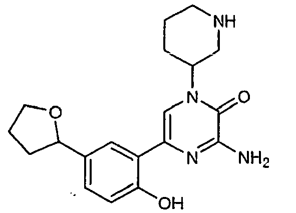 | 3-amino-5-(2-hydroxy-5-tetrahydrofuran-2-ylphenyl)-1-piperidin-3-ylpyrazin-2(1H)-one |
| 38 | 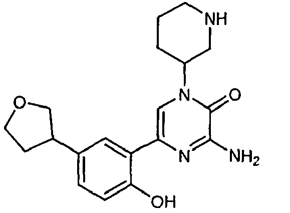 | 3-amino-5-(2-hydroxy-5-tetrahydrofuran-3-ylphenyl)-1-piperidin-3-ylpyrazin-2(1H)-one |
| 39 | 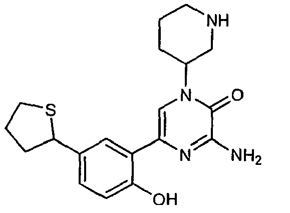 | 3-amino-5-(2-hydroxy-5-tetrahydrothien-2-ylphenyl)-1-piperidin-3-ylpyrazin-2(1H)-one |
| 40 | 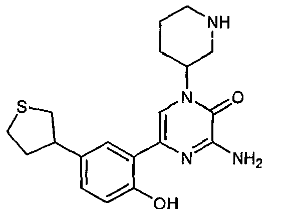 | 3-amino-5-(2-hydroxy-5-tetrahydrothien-3-ylphenyl)-1-piperidin-3-ylpyrazin-2(1H)-one |
| 41 | 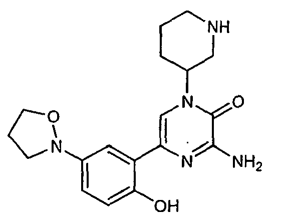 | 3-amino-5-(2-hydroxy-5-isoxazolidin-2-ylphenyl)-1-piperidin-3-ylpyrazin-2(1H)-one |
| 42 | 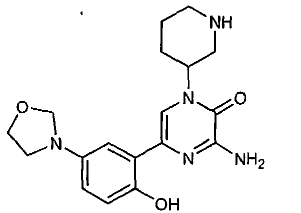 | 3-amino-5-[2-hydroxy-5-(1,3-oxazolidin-3-yl)phenyl]-1-piperidin-3-ylpyrazin-2(1H)-one |
| 43 | 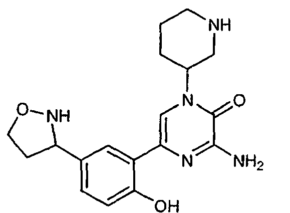 | 3-amino-5-(2-hydroxy-5-isoxazolidin-3-ylphenyl)-1-piperidin-3-ylpyrazin-2(1H)-one |
| 44 | 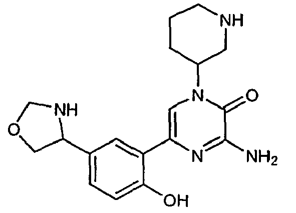 | 3-amino-5-[2-hydroxy-5-(1,3-oxazolidin-4-yl)phenyl]-1-piperidin-3-ylpyrazin-2(1H)-one |
| 45 | 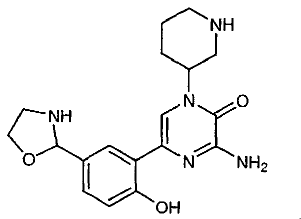 | 3-amino-5-[2-hydroxy-5-(1,3-oxazolidin-2-yl)phenyl]-1-piperidin-3-ylpyrazin-2(1H)-one |
| 46 | 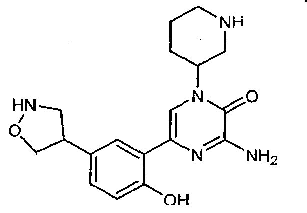 | 3-amino-5-(2-hydroxy-5-isoxazolidin-4-ylphenyl)-1-piperidin-3-yipyrazin-2(1H)-one |
| 47 | 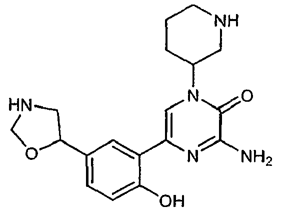 | 3-amino-5-[2-hydroxy-5-(1,3-oxazolidin-5-yl)phenyl]-1-piperidin-3-ylpyrazin-2(1H)-one |
| 48 | 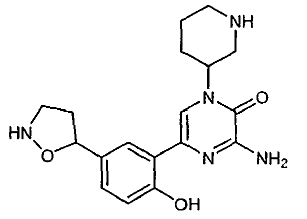 | 3-amino-5-(2-hydroxy-5-isoxazolidin-5-ylphenyl)-1-piperidin-3-ylpyrazin-2(1H)-one |
| 49 | 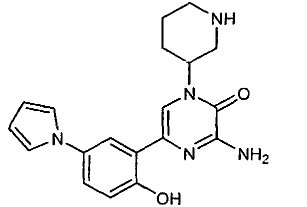 | 3-amino-5-[2-hydroxy-5-(1H-pyrrol-1-yl)phenyl]-1-piperidin-3-ylpyrazin-2(1H)-one |
| 50 | 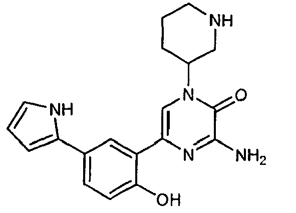 | 3-amino-5-[2-hydroxy-5-(1H-pyrrol-2-yl)phenyl]-1-piperidin-3-ylpyrazin-2(1H)-one |
| 51 | 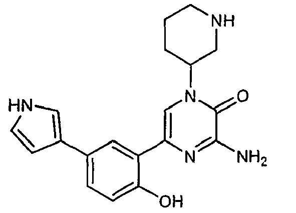 | 3-amino-5-[2-hydroxy-5-(1H-pyrrol-3-yl)phenyl]-1-piperidin-3-ylpyrazin-2(1H)-one |
| 52 | 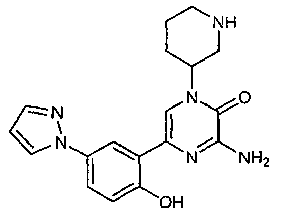 | 3-amino-5-[2-hydroxy-5-(1H-pyrazol-1-yl)phenyl]-piperidin-3-ylpyrazin-2(1H)-one |
| 53 | 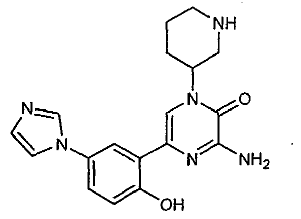 | 3-amino-5-[2-hydroxy-5-(1H-imidazol-1-yl)phenyl]-1-piperidin-3-ylpyrazin-2(1H)-one |
| 54 | 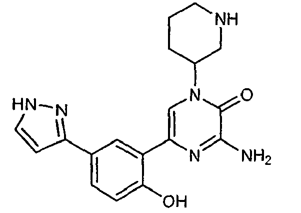 | 3-amino-5-[2-hydroxy-5-(1H-pyrazol-3-yl)phenyl]-1-piperidin-3-ylpyrazin-2(1H)-one |
| 55 | 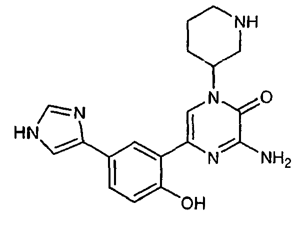 | 3-amino-5-[2-hydroxy-5-(1H-imidazol-4-yl)phenyl]-1-piperidin-3-ylpyrazin-2(1H)-one |
| 56 | 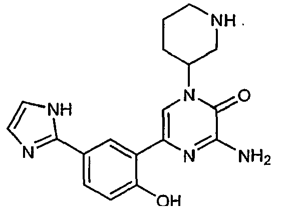 | 3-amino-5-[2-hydroxy-5-(1H-imidazol-2-yl)phenyl]-1-piperidin-3-ylpyrazin-2(1H)-one |
| 57 | 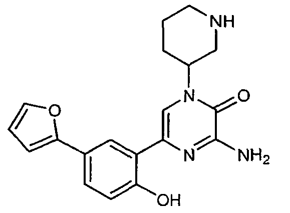 | 3-amino-5-[5-(2-furyl)-2-hydroxyphenyl]-1-piperidin-3-ylpyrazin-2(1H)-one |
| 58 | 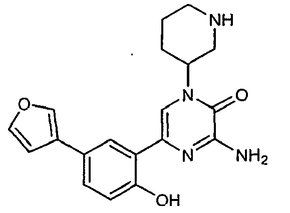 | 3-amino-5-[5-(3-furyl)-2-hydroxyphenyl]-1-piperidin-3-ylpyrazin-2(1H)-one |
| 59 | 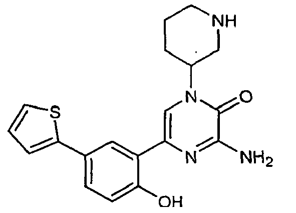 | 3-amino-5-(2-hydroxy-5-thien-2-ylphenyl)-1-piperidin-3-ylpyrazin-2(1H)-one |
| 60 | 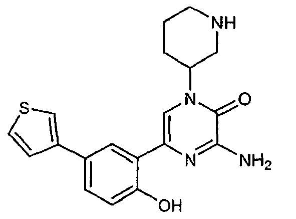 | 3-amino-5-(2-hydroxy-5-thien-3-ylphenyl)-1-piperidin-3-ylpyrazin-2(1H)-one |
| 61 | 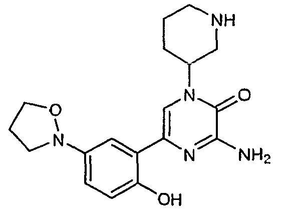 | 3-amino-5-(2-hydroxy-5-isoxazolidin-2-ylphenyl)-1-piperidin-3-ylpyrazin-2(1H)-one |
| 62 | 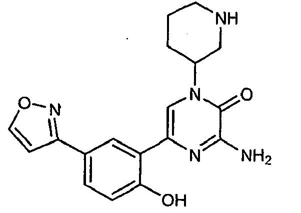 | 3-amino-5-(2-hydroxy-5-isoxazol-3-ylphenyl)-1-piperidin-3-ylpyrazin-2(1H)-one |
| 63 | 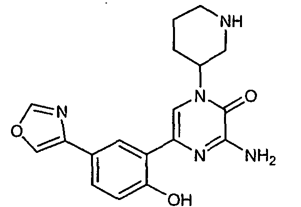 | 3-amino-5-[2-hydroxy-5-(1,3-oxazol-4-yl)phenyl]-1-piperidin-3-ylpyrazin-2(1H)-one |
| 64 | 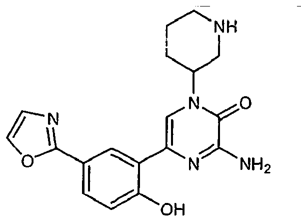 | 3-amino-5-[2-hydroxy-5-(1,3-oxazol-2-yl)phenyl]-1-piperidin-3-ylpyrazin-2(1H)-one |
| 65 | 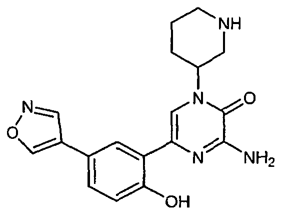 | 3-amino-5-(2-hydroxy-5-isoxazol-4-ylphenyl)-1-piperidin-3-ylpyrazin-2(1H)-one |
| 66 | 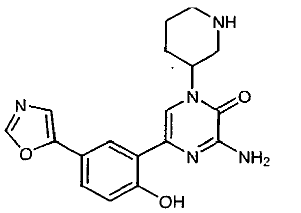 | 3-amino-5-[2-hydroxy-5-(1,3-oxazol-5-yl)phenyl]-1-piperidin-3-ylpyrazin-2(1H)-one |
| 67 | 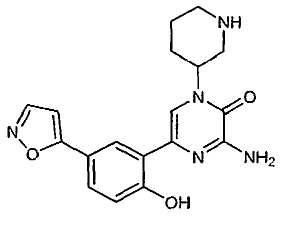 | 3-amino-5-(2-hydroxy-5-isoxazol-5-ylphenyl)-1-piperidin-3-yipyrazin-2(1H)-one |
| 68 | 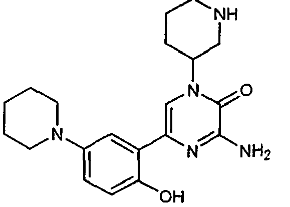 | 3-amino-5-(2-hydroxy-5-piperidin-1-ylphenyl)-1-piperidin-3-ylpyrazin-2(1H)-one |
| 69 | 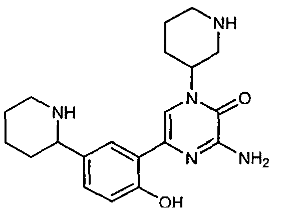 | 3-amino-5-(2-hydroxy-5-piperidin-2-ylphenyl)-1-piperidin-3-ylpyrazin-2(1H)-one |
| 70 | 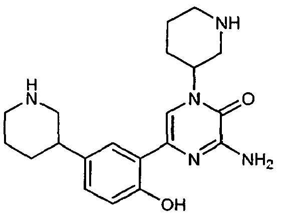 | 3-amino-5-(2-hydroxy-5-piperidin-3-ylphenyl)-1-piperidin-3-ylpyrazin-2(1H)-one |
| 71 | 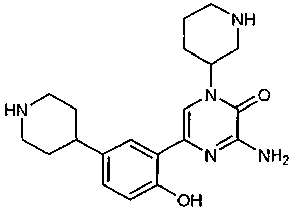 | 3-amino-5-(2-hydroxy-5-piperidin-4-ylphenyl)-1-piperidin-3-ylpyrazin-2(1H)-one |
| 72 | 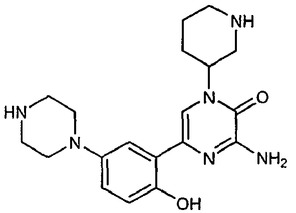 | 3-amino-5-(2-hydroxy-5-piperazin-1-ylphenyl)-1-piperidin-3-ylpyrazin-2(1H)-one |
| 73 | 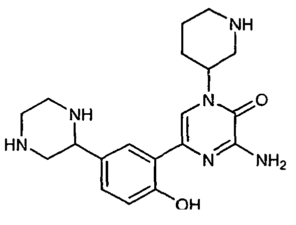 | 3-amino-5-(2-hydroxy-5-piperazin-2-ylphenyl)-1-piperidin-3-ylpyrazin-2(1H)-one |
| 74 | 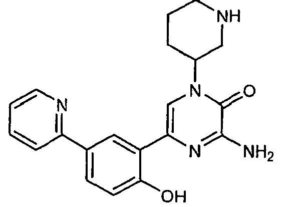 | 3-amino-5-(2-hydroxy-5-pyridin-2-ylphenyl)-1-piperidin-3-ylpyrazin-2(1H)-one |
| 75 | 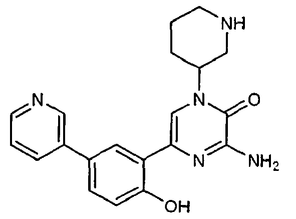 | 3-amino-5-(2-hydroxy-5-pyridin-3-ylphenyl)-1-piperidin-3-ylpyrazin-2(1H)-one |
| 76 | 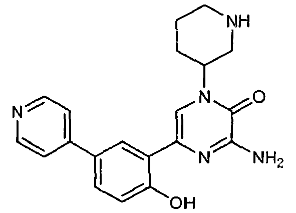 | 3-amino-5-(2-hydroxy-5-pyridin-4-ylphenyl)-1-piperidin-3-ylpyrazin-2(1H)-one |
| 77 | 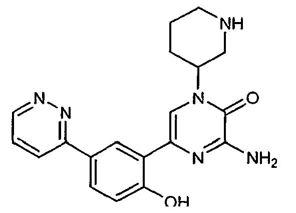 | 3-amino-5-(2-hydroxy-5-pyridazin-3-ylphenyl)-1-piperidin-3-ylpyrazin-2(1H)-one |
| 78 | 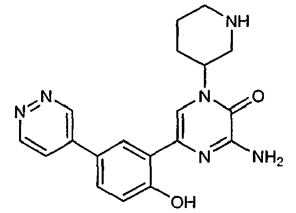 | 3-amino-5-(2-hydroxy-5-pyridazin-4-ylphenyl)-1-piperidin-3-ylpyrazin-2(1H)-one |
| 79 | 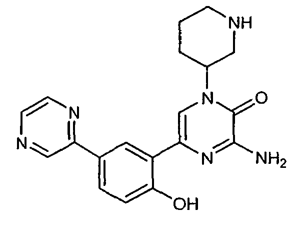 | 3-amino-5-(2-hydroxy-5-pyrazin-2-ylphenyl)-1-piperidin-3-ylpyrazin-2(1H)-one |
| 80 | 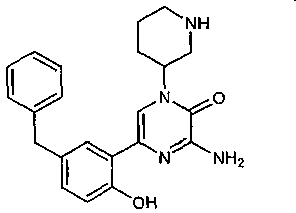 | 3-amino-5-(5-benzyl-2-hydroxyphenyl)-1-piperidin-3-ylpyrazin-2(1H)-one |
| 81 | 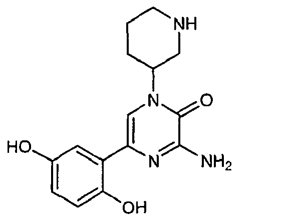 | 3-amino-5-(2,5-dihydroxyphenyl)-1-piperidin-3-ylpyrazin-2(1H)-one |
| 82 | 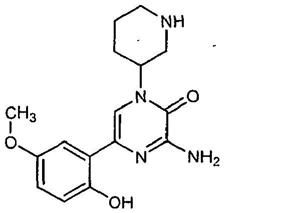 | 3-amino-5-(2-hydroxy-5-methoxyphenyl)-1-piperidin-3-ylpyrazin-2(1H)-one |
| 83 | 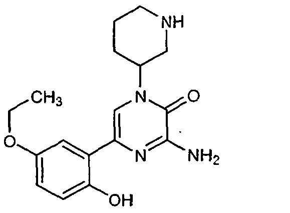 | 3-amino-5-(5-ethoxy-2-hydroxyphenyl)-1-piperidin-3-ylpyrazin-2(1H)-one |
| 84 | 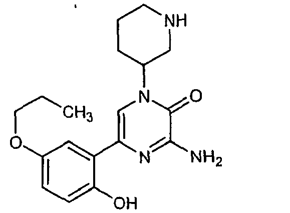 | 3-amino-5-(2-hydroxy-5-propoxyphenyl)-1-piperidin-3-ylpyrazin-2(1H)-one |
| 85 | 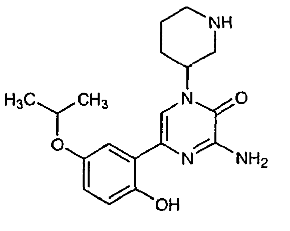 | 3-amino-5-(2-hydroxy-5-isopropoxyphenyl)-1-piperidin-3-ylpyrazin-2(1H)-one |
| 86 | 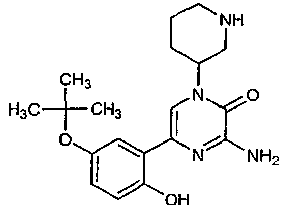 | 3-amino-5-(2-hydroxy-5-t-butoxyphenyl)-1-piperidin-3-ylpyrazin-2(1H)-one |
| 87 | 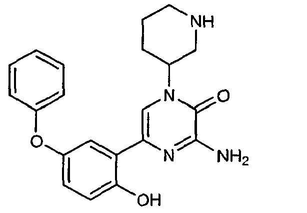 | 3-amino-5-(2-hydroxy-5-phenoxyphenyl)-1-piperidin-3-ylpyrazin-2(1H)-one |
| 88 | 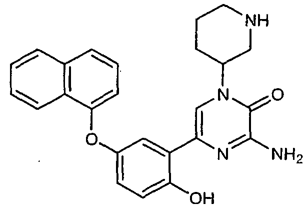 | 3-amino-5-[2-hydroxy-5-(1-naphthyloxy)phenyl]-1-piperidin-3-ylpyrazin-2(1H)-one |
| 89 | 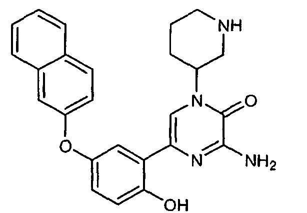 | 3-amino-5-[2-hydroxy-5-(2-naphthyloxy)phenyl]-1-piperidin-3-ylpyrazin-2(1H)-one |
| 90 | 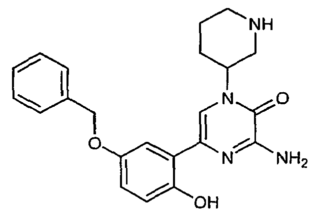 | 3-amino-5-[5-(benzyloxy)-2-hydroxyphenyl]-1-piperidin-3-ylpyrazin-2(1H)-one |
| 91 | 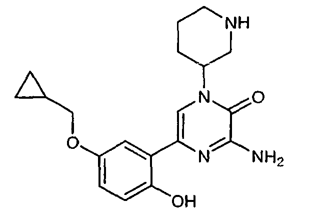 | 3-amino-5-[5-(cyclopropylmethoxy)-2-hydroxyphenyl]-1-piperidin-3-ylpyrazin-2(1H)-one |
| 92 | 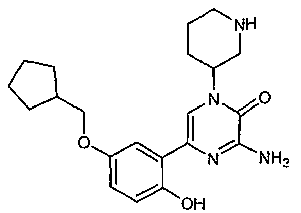 | 3-amino-5-[5-(cyclopentylmethoxy)-2-hydroxyphenyl]-1-piperidin-3-ylpyrazin-2(1H)-one |
| 93 | 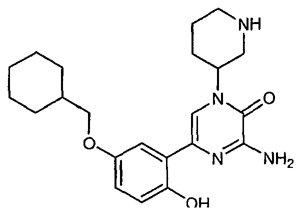 | 3-amino-5-[5-(cyclohexylmethoxy)-2-hydroxyphenyl]-1-piperidin-3-ylpyrazin-2(1H)-one |
| 94 | 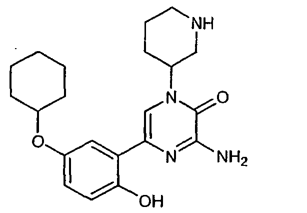 | 3-amino-5-[5-(cyclohexyloxy)-2-hydroxyphenyl]-1-piperidin-3-ylpyrazin-2(1H)-one |
| 95 | 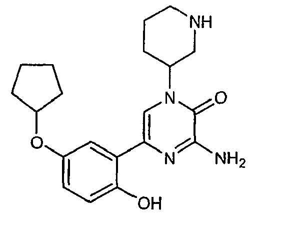 | 3-amino-5-[5-(cyclopentyloxy)-2-hydroxyphenyl]-1-piperidin-3-ylpyrazin-2(1H)-one |

### DEFINITIONS

The term "hydrido" denotes a single hydrogen atom (H). This hydrido radical may be attached, for example, to an oxygen atom to form a hydroxyl radical or two hydrido radicals may be attached to a carbon atom to form a methylene (-CH₂-) radical.

The term "halo" denotes halogen atoms such as fluorine, chlorine, bromine, or iodine.

The term "carbonyl", whether used alone or with other terms such as "alkylcarbonyl", denotes -(C=O)-.

The terms "carboxy" or "carboxyl", whether used alone or with other terms, such as "carboxyalkyl", denotes -CO₂H.

The term "sulfonyl," whether used alone or linked to other terms such as alkylsulfonyl, denotes the divalent radical -SO₂-.

The term "amido" when used by itself or with other terms such as "amidoalkyl", "N-monoalkylamido", "N-monoarylamido", "N,N-dialkylamido", "N-alkyl-N-arylamido", "N-alkyl-N-hydroxyamido" and "N-alkyl-N-hydroxyamidoalkyl", embraces a carbonyl radical substituted with an amino radical.

The terms "N-alkylamido" and "N,N-dialkylamido" denote amido groups which have been substituted with one alkyl radical and with two alkyl radicals, respectively.

The terms "N-monoarylamido" and "N-alkyl-N-arylamido" denote amido radicals substituted, respectively, with one aryl radical, and one alkyl and one aryl radical.

The term "N-alkyl-N-hydroxyamido" embraces amido radicals substituted with a hydroxyl radical and with an alkyl radical.

The terms "sulfamyl" or "sulfonamidyl" denotes a sulfonyl radical substituted with an amino radical, forming a sulfonamide (-SO₂NH₂). The amino radical may be substituted with alkyl and/or aryl moieties to form, e.g., "N-alkylsulfamyl", "N-arylsulfamyl", "N,N-dialkylsulfamyl," and "N-alkyl-N-arylsulfamyl" radicals.

The term "amidino" denotes a -C(=NH)NH₂ radical.

The term "cyanoamidino" denotes a -C(=N-CN)NH₂ radical.

The term "alkyl," used alone or within other terms such as "haloalkyl" and "alkylsulfonyl," embraces linear or branched radicals having one to about twenty carbon atoms. More preferred are "lower alkyl" radicals having one to about eight carbon atoms. Examples of alkyl radicals include methyl, ethyl, propyl (including n-propyl and isopropyl), butyl (including n-butyl, isobutyl, sec-butyl, and t-butyl), pentyl (including n-pentyl and isoamyl), hexyl, octyl and the like.

The term "cycloalkyl" embraces radicals having three to ten carbon atoms, and includes monocyclic, bicyclic, and tricyclic radicals. Examples of cycloalkyl radicals include cyclopropyl, cyclobutyl, cyclopentyl, cyclohexyl, cycloheptyl, decahydronaphthyl, octahydroindyl, octahydropentalene, bicyclo[1.1.0]butyl, bicyclo[2.1.0]pentyl, bicyclo[1.1.1]pentyl, bicyclo[2.1.1]hexyl, bicyclo[2.2.1]heptyl, bicyclo[3.1.1]heptyl, bicyclo[3.2.1]octyl, bicyclo[2.2.2]octyl, and bicyclo[4.2.2]decyl

The term "alkylcarbonyl" embraces radicals having a carbonyl radical substituted with an alkyl radical. An example of an alkylcarbonyl radical is acetyl.

The term "alkylthio" embraces radicals containing a linear or branched alkyl radical, of one to ten carbon atoms, attached to a divalent sulfur atom. An example of an alkylthio radical is methylthio (CH₃S-).

The term "alkylsulfinyl" embraces radicals containing a linear or branched alkyl radical, of one to ten carbon atoms, attached to a divalent -S(=O)- radical. An example of an alkylsulfinyl radical is methylsulfinyl (CH₃S(=O)-).

The term "alkylsulfonyl" embraces alkyl radicals as defined above attached to a divalent sulfonyl radical, -SO₂-.

The term "amidoalkyl" embraces alkyl radicals substituted with amido radicals.

The term "N-alkyl-N-hydroxyamidoalkyl" embraces alkyl radicals substituted with an N-alkyl-N-hydroxyamido radical.

The term "aminoalkyl" embraces alkyl radicals substituted with amino radicals.

The term "carboxyalkyl" embraces radicals having a carboxyl moiety attached to an alkyl radical.

The term "haloalkyl" embraces radicals wherein any one or more of the alkyl carbon atoms is substituted with halo as defined above. Specifically embraced are monohaloalkyl, dihaloalkyl, and polyhaloalkyl radicals. A monohaloalkyl radical, for one example, may have a bromo, chloro, or a fluoro atom within the radical. Dihaloalkyl radicals may have two of the same halo atoms or a combination of different halo radicals; polyhaloalkyl radicals may have more than two of the same halo atoms or a combination of different halo radicals.

The term "hydroxyalkyl" embraces linear or branched alkyl radicals having one to about ten carbon atoms, any of which may be substituted with one or more hydroxyl radicals.

The terms "N-alkylamino" and "N, N-dialkylamino" denote amino groups which have been substituted with one alkyl radical and with two alkyl radicals, respectively.

The term "alkoxy" embraces linear or branched oxy-containing alkyl radicals having one to about ten carbon atoms. Examples of "alkoxy" radicals include methoxy and butoxy.

The term "alkoxyalkyl" embraces linear or branched alkyl radicals having one to about ten carbon atoms substituted by one or more alkoxy radicals each having one to about ten carbon atoms.

"Alkoxy" or "alkoxyalkyl" radicals may be further substituted with one or more halo atoms, such as fluoro, chloro, or bromo, to provide "haloalkoxy" or "haloalkoxyalkyl" radicals.

The term "alkoxycarbonyl" means a radical containing an alkoxy radical, as defined above, attached via an oxygen atom to a carbonyl radical. Examples of such alkoxycarbonyl radicals include methoxycarbonyl and t-butoxycarbonyl.

The term "alkoxycarbonylalkyl" embraces radicals having alkoxycarbonyl moiety, as defined above substituted to an alkyl radical. Examples of such alkoxycarbonylalkyl radicals include methoxycarbonylethyl (-(CHz)₂(O=)COCH₃) and t-butoxycarbonylethyl (-(CH₂)₂(O=)COC(CH₃)₃)

The term "alkylaminoalkyl" embraces aminoalkyl radicals wherein the nitrogen atom is substituted with an alkyl radical.

The term "alkylcarbonylalkyl" denotes an alkyl radical substituted with an "alkylcarbonyl" radical.

The term "alkenyl," used alone or within other terms such as "haloalkenyl," embraces unsaturated linear or branched radicals having two to about twenty carbon atoms and containing at least one carbon-carbon double bond. Examples of alkenyl radicals include ethenyl, propenyl butenyl, pentenyl, and the like.

The term "cycloalkenyl" embraces unsaturated radicals having three to ten carbon atoms and containing at least one carbon-carbon double bond, and includes monocyclic, bicyclic, and tricyclic radicals. Examples of cycloalkenyl radicals include cyclopropenyl, cyclobutenyl, cyclopentenyl, cyclohexenyl, cycloheptenyl, decahydronaphthenyl, hexahydroindenyl, hexahydropentalenyl, bicyclo[2.1.0]pentenyl, bicyclo[1.1.1]pentenyl, bicyclo[2.1.1]hexenyl, bicyclo[2.2.1]heptenyl, bicyclo[3.1.1]heptenyl, bicyclo[3.2.1]octenyl, bicyclo[2.2.2]octenyl, and bicyclo[4.2.2]decenyl.

The term "alkynyl," used alone or within other terms such as "haloalkynyl," embraces unsaturated linear or branched radicals having two to about twenty carbon atoms and containing at least one carbon-carbon triple bond. Examples of alkynyl radicals include ethynyl, propynyl butynyl, pentynyl, and the like.

The term "aryl", alone or in combination, means a carbocyclic aromatic system containing one, two, or three rings wherein at least one of the rings is aromatic, and wherein such rings may be attached together in a pendant manner or may be fused. Examples of aryl radicals include phenyl, naphthyl, tetrahydronapthyl, indyl, and biphenyl. Aryl moieties, alone or in combination, may be optionally substituted by one or more substituents selected from the group consisting of amino, halo, cyano, hydroxyl, alkyl, alkoxy, and carboxyl.

The term "aralkyl" embraces aryl-substituted alkyl radicals such as benzyl, diphenylmethyl, triphenylmethyl, phenethyl, and diphenethyl.

The term "arylsulfonyl" embraces aryl radicals as defined above attached to a sulfonyl radical.

The term "acyl," whether used alone or within a term such as "acylamino," denotes a radical provided by the residue after removal of hydroxyl from an organic acid.

The term "acylamino" embraces an amino radical substituted with an acyl group. An examples of an "acylamino" radical is acetylamino (CH₃C(=O)NH-).

The term "heterocyclic" or "heterocycle" means a saturated or unsaturated mono- or multi-ring carbocyclic system wherein one or more carbon atoms in the system are replaced by nitrogen, sulfur, phosphorous, and/or oxygen. The term "heterocyclic" embraces "heteroaryl" groups, which means a carbocyclic aromatic system containing one, two, or three rings wherein at least one of the rings is aromatic, wherein such rings may be attached together in a pendant manner or may be fused, and wherein one or more carbon atoms in the system are replaced' by nitrogen, sulfur, phosphorous, and/or oxygen. "Heterocyclic" includes, for example, the following structures:

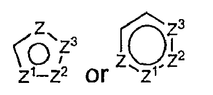

wherein Z, Z¹, Z², and Z³ are independently carbon, sulfur, phosphorous, oxygen, or nitrogen, with the proviso that one of Z, Z¹, Z², or Z³ is other than carbon, but is not oxygen or sulfur when attached to another Z atom by a double bond or when attached to another oxygen or sulfur atom. Furthermore, the optional substituents are understood to be attached to Z, Z¹, Z², or Z³ only when each is carbon. For example, the term "heterocyclyl" embraces each of the following groups, although this listing is not meant to limit the definition to these groups only: furanyl; thienyl; pyrrolyl; 2-isopyrrolyl; 3-isopyrrolyl; pyrazolyl; 2-isoimidazolyl; 1,2,3-triazolyl; 1,2,4-triazolyl; 1,2-dithiolyl; 1,3-dithiolyl; 1,2,3-oxathiolyl; isoxazolyl; oxazolyl; thiazolyl; isothiazolyl; 1,2,3-oxadiazolyl; 1,2,4-oxadiazolyl; 1,2,5-oxadiazolyl; 1,3,4-oxadiazolyl; 1,2,3,4-oxatriazolyl; 1,2,3,5-oxatriazolyl; 1,2,3-dioxazolyl; 1,2,4-dioxazolyl; 1,3,2-dioxazolyl; 1,3,4-dioxazolyl; 1,2,5-oxathiazolyl; 1,3-oxathiolyl; 1,2-pyranyl; 1,4-pyranyl; 1,2-pyranonyl; 1,4-pyranonyl; 1,2-dioxinyl; 1,3-dioxinyl; pyridyl; pyridazyl; pyrimidyl; pyrazinyl; piperazyl; 1,3,5-triazinyl; 1,2,4-triazinyl; 1,2,3-triazinyl; 1,2,4-oxazinyl; 1,3,2-oxazinyl; 1,3,6-oxazinyl; 1,2,6-oxazinyl; 1,4-oxazinyl; o-isoxazinyl; p-isoxazinyl; 1,2,5-oxathiazinyl; 1,4-oxazir:yl; o-isoxazinyl; p-isoxazinyl; 1,2,5-oxathiainzyl; 1,2,6-oxathiainzyl; 1,4,2-oxadiainzyl; 1,3,5,2-oxadiainzyl; morpholino; azepinyl; oxepinyl; thiepinyl; 1,2,4-diazepinyl; benzofuranyl; isobenzofuranyl; benzothiofuranyl; isobenzothiofuranyl; indolyl; indoleninyl; 2-isobenzazolyl; 1,5-pyrindinyl; pyrano[3,4-b]pyrrolyl; isoindazolyl; indoxazinyl; benzoxazolyl; anthranilyl; 1,2-benzopyranyl; quinolyl; isoquinolyl; cinnolyl; quinazolyl; naphthyridyl; pyrido[3,4-b]pyridyl; pyrido[3,2-b]pyridyl; pyrido[4,3-b]pyridyl; 1,3,2-benzoxazyl; 1,4,2-benzoxazyl; 2,1,3-benzoxazyl; 3,1,4-benzoxazyl; 1,2-benzoisoxazyl; 1,4-benzoisoxazyl; carbazolyl; xanthenyl; acridinyl; purinyl; thiazolidyl; piperidyl; pyrrolidyl; 1,2-dihydroazinyl; 1,4-dihydroazinyl; 1,2,3,6-tetrahydro-1,3-diazinyl; perhydro-1,4-diazinyl; 1,2-thiapyranyl; and 1,4-thiapyranyl. Heterocyclic moieties, alone or in combination, may be optionally substituted by one or more substituents selected from the group consisting of amino, halo, cyano, hydroxyl, alkyl, alkoxy, and carboxyl.

The term "heteroaryl" also embraces radicals where heterocyclic radicals are fused with aryl radicals as defined herein. Examples of such fused bicyclic radicals include benzofuran, benzothiophene, and the like.

The term "heterocycloalkyl" embraces heterocyclic-substituted alkyl radicals such as pyridylmethyl and thienylmethyl.

The terms benzyl and phenylmethyl are interchangeable.

The phrases "combination therapy", "co-administration", "administration with", or "co-therapy", in defining the use of a selective IKK-2 inhibitory agent in combination with another therapeutic agent such as another analgesic agent, is intended to embrace administration of each agent in a sequential manner in a regimen that may provide beneficial effects of the drug combination, and is intended as well to embrace co-administration of these agents in a substantially simultaneous manner, such as in a single capsule or dosage device having a fixed ratio of these active agents or in multiple, separate capsules or dosage devices for each agent, where the separate capsules or dosage devices can be taken together contemporaneously, or taken within a period of time sufficient to receive a beneficial effect from both of the constituent agents of the combination.

The term "subject" for purposes of treatment includes any human or animal subject who is in need of the prevention of, or who has pain, inflammation and/or any one of the known inflammation-associated disorders. The subject is typically a human subject.

The phrase "therapeutic combination" as used herein refers to the combination of two or more therapeutic compounds and, optionally, one or more pharmaceutically acceptable carrier used to provide dosage forms that produce a beneficial effect of each therapeutic compound in the subject at the desired time, whether the therapeutic compounds are administered substantially simultaneously, or sequentially.

The phrase "therapeutically effective" as used herein refers to an amount of a therapeutic compound, or amounts of combined therapeutic compounds in combination therapy. The amount or combined amounts achieve one or more of the goals of preventing, inhibiting, reducing or eliminating the inflammation or inflammation-related disease or condition. A "therapeutically-effective" amount of each agent in a combination therapy is expected to be less than an amount used in treatment using agent by itself, thus while avoiding adverse side effects typically associated with alternative therapies, namely higher dose monotherapy of each agent by itself.

The terms "treating" or "to treat" means to alleviate symptoms, eliminate the causation either on a temporary or permanent basis, or to prevent or slow the appearance of symptoms in a subject. The term "treatment" includes alleviation, elimination of causation of or prevention of pain and/or inflammation associated with, but not limited to, any of the diseases or disorders described above.

Pharmaceutically acceptable salts of the compounds of Formula I include the acid addition and base salts thereof.

Suitable acid addition salts are formed from acids that form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

Pharmaceutically acceptable salts of compounds of Formula I may be prepared by one or more of three methods: (i) by reacting the compound of Formula I with the desired acid or base; (ii) by removing an acid- or base-labile protecting group from a suitable precursor of the compound of Formula I or by ring-opening a suitable cyclic precursor, for example, a lactone or lactam, using the desired acid or base; or (iii) by converting one salt of the compound of Formula I to another by reaction with an appropriate acid or base or by means of a suitable ion exchange column. All three reactions are typically carried out in solution. The resulting salt may precipitate out and be collected by filtration or may be recovered by evaporation of the solvent. The degree of ionization in the resulting salt may vary from completely ionized to almost non-ionized.

The compounds of the invention may exist in both unsolvated and solvated forms. The term "solvate" is used herein to describe a molecular complex comprising the compound of the invention and a stoichiometric amount of one or more pharmaceutically acceptable solvent molecules, for example, ethanol. The term "hydrate" is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionized, partially ionized, or non-ionized. For a review of such complexes, see Haleblian, J. Pharm. Sci., **64(8)**, 1269-1288 (1975).

Hereinafter all references to compounds of Formula I include references to salts, solvates and complexes thereof and to solvates and complexes of salts thereof.

The compounds of the invention include compounds of Formula I as hereinbefore defined, including all polymorphs and crystal habits thereof, prodrugs and isomers thereof (including optical, geometric and tautomeric isomers) as hereinafter defined and isotopically-labeled compounds of Formula I.

As indicated, so-called prodrugs of the compounds of Formula I are also within the scope of the invention. The term "prodrug" refers to a compound that is a drug precursor which, following administration to a subject and subsequent absorption, is converted to an active species *in vivo* via some process, such as a metabolic process. Other products from the conversion process are easily disposed of by the body. The more preferred prodrugs are those involving a conversion process that produces products that are generally accepted as safe.

Prodrugs in accordance with the invention can, for example, be produced by replacing appropriate functionalities present in the compounds of Formula I with certain moieties known to those skilled in the art as "pro-moieties."

Some examples of prodrugs in accordance with the invention include: (i) where the compound of Formula I contains a carboxylic acid functionality (-CO₂H), an ester thereof, for example, a compound wherein the hydrogen of the carboxylic acid functionality of the compound of Formula I is replaced by C₁-C₈ alkyl; (ii) where the compound of Formula I contains an alcohol functionality (-OH), an ether thereof, for example, a compound wherein the hydrogen of the alcohol functionality of the compound of Formula I is replaced by C₁-C₆ alkanoyloxymethyl; and (iii) where the compound of Formula I contains a primary or secondary amino functionality (-NH₂ or -NHR where R ≠ H), an amide thereof, for example, a compound wherein, as the case may be, one or both hydrogens of the amino functionality of the compound of Formula I is/are replaced by C₁-C₁₀ alkanoyl.

Further examples of replacement groups in accordance with the foregoing examples and examples of other prodrug types may be found in the aforementioned references.

Moreover, certain compounds of Formula I may themselves act as prodrugs of other compounds of Formula I.

Also included within the scope of the invention are metabolites of compounds of Formula I, that is, compounds formed *in vivo* upon administration of the drug. Some examples of metabolites in accordance with the invention include: (i) where the compound of Formula I contains a methyl group, an hydroxymethyl derivative thereof (-CH₃ → -CH₂OH); (ii) where the compound of Formula I contains an alkoxy group, an hydroxy derivative thereof (-OR → -OH); (iii) where the compound of Formula I contains a tertiary amino group, a secondary amino derivative thereof (-NR^{a}R^{b} → -NHR^{a} or -NHR^{b}); (iv) where the compound of Formula I contains a secondary amino group, a primary derivative thereof (-NHR → -NH₂); (v) where the compound of Formula I contains a phenyl moiety, a phenol derivative thereof (-Ph → -PhOH); and (vi) where the compound of Formula I contains an amide group, a carboxylic acid derivative thereof (-CONH₂ -> -COOH).

Compounds of Formula I containing one or more asymmetric carbon atoms can exist as two or more stereoisomers. Where a compound of Formula I contains an alkenyl or alkenylene group, geometric cis/trans (or Z/E) isomers are possible. Where structural isomers are interconvertible via a low energy barrier, tautomeric isomerism ("tautomerism") can occur. This can take the form of proton tautomerism in compounds of Formula I containing, for example, an imino, keto, or oxime group, or so-called valence tautomerism in compounds which contain an aromatic moiety. It follows that a single compound may exhibit more than one type of isomerism.

Included within the scope of the present invention are all stereoisomers, geometric isomers and tautomeric forms of the compounds of Formula I, including compounds exhibiting more than one type of isomerism, and mixtures of one or more thereof. Also included are acid addition or base salts wherein the counterion is optically active, for example, d-lactate or I-lysine, or racemic, for example, dl-tartrate or dl-arginine.

Cis/trans isomers may be separated by conventional techniques well known to those skilled in the art, for example, chromatography and fractional crystallization.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (chiral HPLC).

Alternatively, the racemate (or a racemic precursor) may be reacted with a suitable optically active compound, for example, an alcohol, or, in the case where the compound of Formula I contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2 to 20%, and from 0 to 5% by volume of an alkylamine, typically 0.1% diethylamine. Concentration of the eluate affords the enriched mixture.

Stereoisomeric conglomerates may be separated by conventional techniques known to those skilled in the art.

The present invention includes all pharmaceutically acceptable isotopically-labeled compounds of Formula I wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labeled compounds of Formula I, for example, those incorporating a radioactive isotope, are useful in drug and/or substrate tissue distribution studies. The radioactive isotopes tritium (³H) and ¹⁴C are particularly useful for this purpose in view of their ease of incorporation and ready means of detection.

Substitution with heavier isotopes such as deuterium (²H) may afford certain therapeutic advantages resulting from greater metabolic stability, for example, increased *in vivo* half-life or reduced dosage requirements, and hence may be preferred in some circumstances.

Substitution with positron-emitting isotopes, such as ¹¹C, ¹⁸F, ¹⁵O and ¹³N, can be useful in Positron Emission Topography (PET) studies for examining substrate receptor occupancy.

Isotopically-labeled compounds of Formula I can generally be prepared by conventional techniques known to those skilled in the art or by processes analogous to those described in the accompanying Examples using an appropriate isotopically-labeled reagent in place of the non-labeled reagent previously employed.

Pharmaceutically acceptable solvates in accordance with the invention include those wherein the solvent of crystallization may be isotopically substituted, e.g. D₂O, d₆-acetone, or d₆-DMSO.

Compounds of the invention intended for pharmaceutical use may be administered as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

Generally, the compounds of the invention may be administered as a formulation in association with one or more pharmaceutically acceptable excipients. The term "excipient" is used herein to describe any ingredient other than the compound(s) of the invention. The choice of excipient will to a large extent depend on factors such as the particular mode of administration, the effect of the excipient on solubility and stability, and the nature of the dosage form.

The compounds of the invention may be administered alone or in combination with one or more other compounds of the invention or in combination with one or more other drugs (or as any combination thereof). For example, compounds of Formula I may be used in co-therapies, partially or completely, in place of other conventional antiinflammatory therapies, such as together with other IKK-2 inhibitors, steroids, NSAIDs, COX-2 selective inhibitors, matrix metalloproteinase inhibitors, 5-lipoxygenase inhibitors, LTB₄ antagonists and LTA₄ hydrolase inhibitors.

Pharmaceutical compositions suitable for the delivery of compounds of the present invention and methods for their preparation will be readily apparent to those skilled in the art.

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, solid solution, liposome, films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Liang and Chen, Expert Opinion in Therapeutic Patents, **11(6),** 981-986 (2001).

For tablet dosage forms, depending on dose, the drug may make up from 1 to 80 wt.% of the dosage form, more typically from 5 to 60 wt.% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 to 25 wt.%, preferably from 5 to 20 wt.% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 to 5 wt.% of the tablet, and glidants may comprise from 0.2 to 1 wt.% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 to 10 wt.%, preferably from 0.5 to 3 wt.% of the tablet.

Other possible ingredients include anti-oxidants, colorants, flavoring agents, preservatives and taste-masking agents.

Exemplary tablets contain up to about 80% drug, from about 10 to about 90 wt.% binder, from about 0 to about 85 wt.% diluent, from about 2 to about 10 wt.% disintegrant, and from about 0.25 to about 10 wt.% lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

Consumable oral films for human or veterinary use are typically pliable water-soluble or water-swellable thin film dosage forms which may be rapidly dissolving or mucoadhesive and typically comprise a compound of Formula I, a film-forming polymer, a binder, a solvent, a humectant, a plasticiser, a stabilizer or emulsifier, a viscosity-modifying agent and a solvent. Some components of the formulation may perform more than one function.

The compound of Formula I may be water-soluble or insoluble. A water-soluble compound typically comprises from 1 to 80 wt.%, more typically from 20 to 50 wt.%, of the solutes. Less soluble compounds may comprise a greater proportion of the composition, typically up to 88 wt.% of the solutes. Alternatively, the compound of Formula I may be in the form of multiparticulate beads.

The film-forming polymer may be selected from natural polysaccharides, proteins, or synthetic hydrocolloids and is typically present in the range 0.01 to 99 wt.%, more typically in the range 30 to 80 wt.%.

Other possible ingredients include anti-oxidants, colorants, flavorings and flavor enhancers, preservatives, salivary stimulating agents, cooling agents, co-solvents (including oils), emollients, bulking agents, antifoaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted- and programmed-release.

Suitable modified release formulations for the purposes of the invention are described in U.S. Patent No. 6,106,864. Details of other suitable release technologies such as high energy dispersions and osmotic and coated particles are to be found in Verma et al., Pharmaceutical Technology On-line, **25(2)**, 1-14 (2001). The use of chewing gum to achieve controlled release is described in PCT Publication No. WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art.

The solubility of compounds of Formula I used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted- and programmed-release. Thus compounds of the invention may be formulated as a solid, semi-solid, or thixotropic liquid for administration as an implanted depot providing modified release of the active compound. Examples of such formulations include drug-coated stents and poly(dl-lactic-coglycolic)acid (PGLA) microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated; see, e.g., Finnin and Morgan, J Pharm Sci, **88(10),** 955-958 (1999).

Other means of topical administration include delivery by electroporation, iontophoresis, phonophoresis, sonophoresis and microneedle or needle-free (e.g. Powderject^{™} Bioject^{™} , etc.) injection.

Formulations for topical administration may be formulated to be immediate and/or modified release. Modified release formulations include delayed-, sustained-, pulsed-, controlled-, targeted- and programmed-release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurized container, pump, spray, atomizer, or nebulizer contains a solution or suspension of the compound(s) of the invention comprising, for example, ethanol, aqueous ethanol, or a suitable alternative agent for dispersing, solubilizing, or extending release of the active, a propellant(s) as solvent and an optional surfactant, such as sorbitan trioleate, oleic acid, or an oligolactic acid.

Prior to use in a dry powder or suspension formulation, the drug product is micronized to a size suitable for delivery by inhalation (typically less than 5 µm). This may be achieved by any appropriate comminuting method, such as spiral jet milling, fluid bed jet milling, supercritical fluid processing to form nanoparticles, high pressure homogenization, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as I-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomizer using electrohydrodynamics to produce a fine mist may contain from 1 µg to 20 mg of the compound of the invention per actuation and the actuation volume may vary from 1 to 100 µL. A typical formulation may comprise a compound of Formula I, propylene glycol, sterile water, ethanol and sodium chloride. Alternative solvents which may be used instead of propylene glycol include glycerol and polyethylene glycol.

Suitable flavors, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted- and programmed-release.

In the case of dry powder inhalers and aerosols, the dosage unit is determined by means of a valve which delivers a metered amount. Units in accordance with the invention are typically arranged to administer a metered dose or "puff" containing from 20 to 1000 µg of the compound of Formula I. The overall daily dose will typically be in the range 100 µg to 10 mg which may be administered in a single dose or, more usually, as divided doses throughout the day, for example 2, 3, 4 or 8 times, giving for example, 1, 2 or 3 doses each time.

The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted- and programmed-release.

The compounds of the invention may also be administered directly to the eye or ear, typically in the form of drops of a micronized suspension or solution in isotonic, pH-adjusted, sterile saline. Other formulations suitable for ocular and aural administration include ointments, biodegradable (e.g., absorbable gel sponges, collagen) and non-biodegradable (e.g., silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted- or programmed-release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e., as a carrier, diluent, or solubilizer. Most commonly used for these purposes are alpha-, beta- and gamma-cyclodextrins, such as those described in PCT Publication No. WO 98/55148.

Inasmuch as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions.

Such kits comprises two or more separate pharmaceutical compositions, at least one of which contains a compound of Formula I in accordance with the invention, and means for separately retaining said compositions, such as a container, divided bottle, or divided foil packet. An example of such a kit is the familiar blister pack used for the packaging of tablets, capsules and the like.

Such kits are particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

The amount of therapeutically active compounds that are administered and the dosage regimen for treating a disease condition with the compounds and/or compositions of this invention depends on a variety of factors, including the age, weight, sex and medical condition of the subject, the severity of the inflammation or inflammation related disorder, the route and frequency of administration, and the particular compound employed, and thus may vary widely. The pharmaceutical compositions may contain active ingredients in the range of about 0.1 to 1000 mg, preferably in the range of about 7.0 to 350 mg. A daily dose of about 0.01 to 100 mg/kg body weight, preferably between about 0.1 and about 50 mg/kg body weight and most preferably between about 0.5 to 30 mg/kg body weight, may be appropriate. The daily dose can be administered in one to four doses per day. In the case of skin conditions, it may be preferable to apply a topical preparation of compounds of this invention to the affected area two to four times a day.

It will be understood, however, that the specific dose level for any particular patient will depend upon a variety of factors including the activity of the specific compound employed, the age, body weight, general health, sex, diet, time of administration, route of administration, and rate of excretion, drug combination and the severity of the particular disease undergoing therapy.

These dosages are based on an average human subject having a weight of about 60 to 70 kg. The physician will readily be able to determine doses for subjects whose weight falls outside this range, such as infants and the elderly.

For the avoidance of doubt, references herein to "treatment" include references to curative, palliative and prophylactic treatment.

"DMF" is N,N-dimethylformamide.

"DMSO" is dimethylsulfoxide.

"ESI" is electrospray ionization Mass spectrometry.

"HATU" is O-(7-azabenzotriazol-1-yl)-N,N,N',N'-tetramethyluronium hexafluorophosphate.

"HBTU" is O-benzotriazolo-1-yl)-N,N,N', N'-tetramethyluronium.

"HRMS" is high resolution mass spectrometry.

"NMR" is nuclear magnetic resonance.

"Ph" is phenyl.

"i.d." is inner diameter.

"EtOAc" is ethyl acetate.

"TFA" is trifluoroacetic acid.

### EXAMPLES

Example 1: 3-amino-5-(2-hydroxyphenyl)-1-piperidin-3-ylpyrazin-2(1H)-one

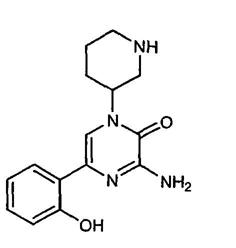

Step 1: Preparation of (2-(benzyloxy)phenyl)(1,3-dithian-2-yl)methanamine

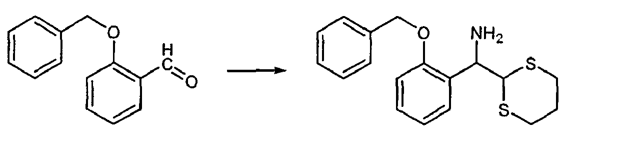

To a cooled (0°C) solution of 1,3-dithiane (1.31 g) in THF (10 mL) was added *n*-BuLi (1.9M in hexanes, 6.0 mL) slowly by syringe. The resulting mixture was stirred for 2 h at 0°C. In a separate flask, LHMDS (1.0M in THF, 10 mL) was slowly added to a cooled (0°C) solution of 2-benzyloxybenzaldehyde (2.10 g) in THF (20 mL). The resulting solution was stirred 1 h at 0°C. The lithiated dithiane generated above was then added slowly via cannula. The reaction was allowed to warm to room temperature and stirred overnight, then quenched by addition of saturated aqueous NH₄Cl. Phases were separated and the aqueous phase extracted with ethyl acetate. The combined organic phases were dried (MgSO₄) and concentrated under reduced pressure. The crude product was purified via flash chromatography (1.5:1 ethyl acetate:hexanes) to give the title compound as an extremely viscous oil.

Step 2: Preparation of methyl ((2-(benzyloxy)phenyl)(1,3-dithian-2-yl)methylcarbamoyl)formate

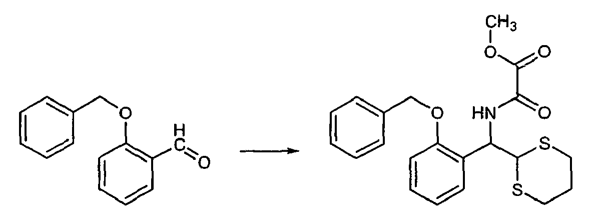

A mixture of (2-(benzyloxy)phenyl)(1,3-dithian-2-yl)methanamine from Step 1 (2.4 g) and dimethyl oxalate (2.6 g) was heated at 55°C in methanol (50 mL) under N₂ for 60.5 h. The reaction mixture was then diluted with water (50 mL) and the white solid product isolated by vacuum filtration, washing repeatedly with 1:1 methanol:water. Drying in a vacuum oven at 70°C provided the title compound as a white powdery solid.

Step 3: Preparation of benzyl 4-{[{[[2-(benzyloxy)phenyl](1,3-dithian-2-yl)methyl]amino}(oxo)acety)]amino}piperidine-1-carboxylate

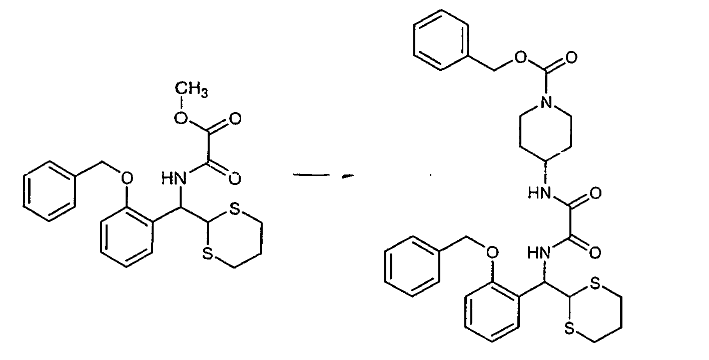

A mixture of methyl ((2-(benzyloxy)phenyl)(1,3-dithian-2-yl)methylcarbamoyl)formate from Step 2 (2.09 g) and 1-benzyloxycarbonyl-3-aminopiperidine (1.405 g) in ethyl acetate (25 mL) was heated at 60°C overnight, then at 75°C for 5.5 h. After cooling to room temperature, the reaction mixture was diluted with hexanes (25 mL) and a white solid isolated by vacuum filtration, washing repeatedly with 1:1 hexanes:ethyl acetate. Owing to the presence of the desired product in the filtrate, the filtrate was concentrated and combined with the filtered solid. The title compound was purified by flash chromatography (20:1 to 10:1 dichloromethane:acetonitrile) to give a white solid.

Step 4: Preparation of benzyl 4-{[({1-[2-(benzyloxy)phenyl]-2-oxoethyl}amino)(oxo)acetyl]amino}piperidine-1-carboxylate

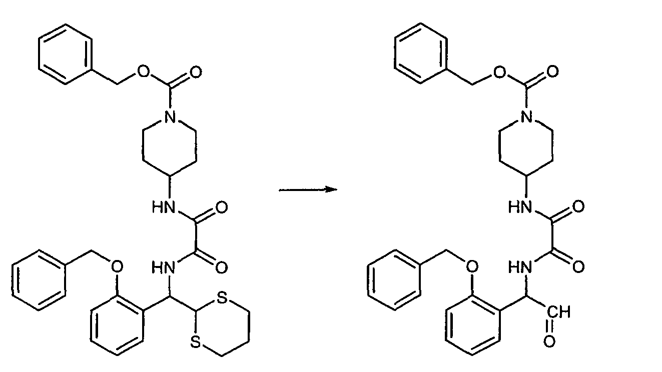

To a cooled (-30°C) solution of benzyl 4-{[{[[2-(benzyloxy)phenyl](1,3-dithian-2-yl)methyl]amino}(oxo)acetyl]amino}piperidine-1-carboxylate from Step 3 (1.65 g) in dichloromethane (75 mL) was added a solution of methylbis(methylthio) sulfonium hexachloroantimonate (4.56 g) in dichloromethane (50 mL) by cannula over 10 min, maintaining a cooling bath temperature less than -32°C (see Prato et al. (1982) Synthesis p. 679-80). The reaction was stirred for 5 min., then quenched by pouring onto 1 M NaOH (250 mL). The biphasic mixture was diluted with dichloromethane (100 mL) and phases separated. The aqueous phase was extracted three times with dichloromethane. The combined organic phases were dried (MgSO₄) and concentrated under reduced pressure. The crude product was purified by flash chromatography (10:1 to 5:1 dichloromethane:acetonitrile) to give the title compound as an off-white solid.

Step 5: Preparation of benzyl 3-[5-[2-(benzyloxy)phenyl]-2,3-dioxo-3,4-dihydropyrazin-1(2H)-yl]piperidine-1-carboxylate

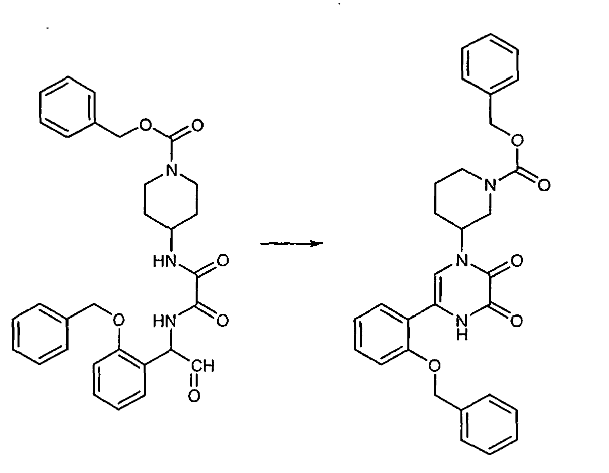

To a suspension of the aldehyde prepared in Step 4 (1.05 g) in acetic acid (16 mL) was added a solution of trifluoroacetic acid:trifluoroacetic anhydride:acetic acid (3.3 mL, 0.6:1.1:4.4). The resulting suspension was heated for 70 minutes in a microwave at 110°C. An additional 1 mL of the trifluoroacetic acid:trifluoroacetic anhydride:acetic acid solution was added and the mixture heated an additional 25 min at 110°C in the microwave (see Fleitz et al. (2000) Synth. Commun. 30, p. 3171-80). The reaction mixture was then concentrated under reduced pressure. The crude oil was diluted with a small amount of dichloromethane and treated with pyridine (1 mL). The title compound was isolated by flash chromatography (1.5:1 acetonitrile:dichloromethane) to give a lightly peach-colored foamy solid.

Step 6: Preparation of benzyl 3-[5-[2-(benzyloxy)phenyl]-3-bromo-2-oxopyrazin-1 (2H)-yl]piperidine-1-carboxylate

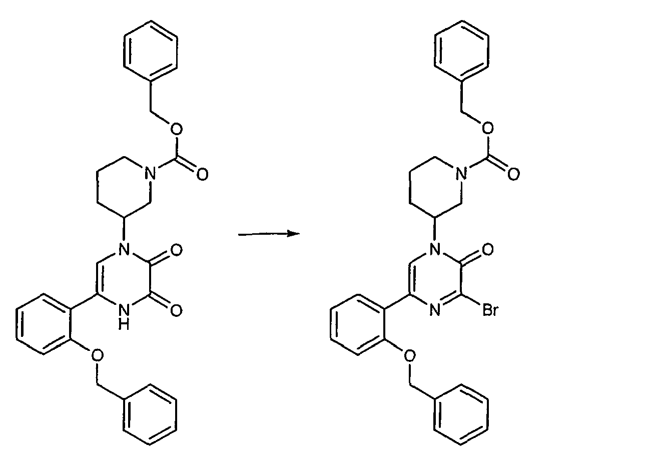

To a suspension of benzyl 3-[5-[2-(benzyloxy)phenyl]-2,3-dioxo-3,4-dihydropyrazin-1(2H)-yl]piperidine-1-carboxylate from Step 5 (0.49 g) and N,N-diisopropylethylamine (0.83 mL) in α,α,α-trifluorotoluene (8 mL) under N₂ was added a solution of POBr₃ in α,α,α-trifluorotoluene (2 M, 1.3 mL). The resulting mixture was heated 1.75 h at 120°C in the microwave. The reaction was quenched by addition of saturated aqueous sodium bicarbonate (25 mL) and diluted with dichloromethane (50 mL) and saturated aqueous sodium bicarbonate (25 mL). Phases were separated and the aqueous phase extracted three times with dichloromethane. The combined organic phases were dried (MgSO₄) and concentrated under reduced pressure. The title compound was purified by flash chromatography (2.5:1 to 2:1 hexanes:ethyl acetate) to give a yellow powder.

Step 7: Preparation of benzyl 3-[5-[2-(benzyloxy)phenyl]-3-[(2,4-dimethoxybenzyl)amino]-2-oxopyrazin-1 (2H)-yl]piperidine-1-carboxylate

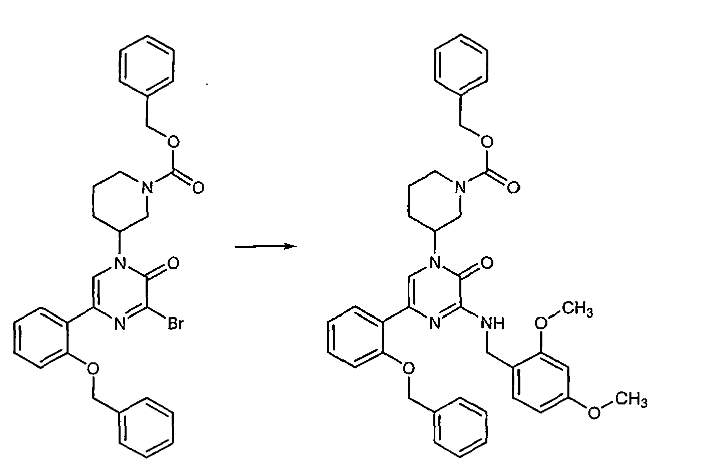

A solution of benzyl 3-[5-[2-(benzyloxy)phenyl]-3-bromo-2-oxopyrazin-1 (2H)-yl]piperidine-1-carboxylate from Step 6 (0.1294 g) and 2,4-dimethoxybenzylamine (0.34 mL) in acetonitrile (5 mL) was heated at 110°C for 1 h in a microwave. The title compound was isolated by flash chromatography (2:1 hexanes:ethyl acetate) to give a light yellow solid.

Step 8: Preparation of 5-[2-(benzyloxy)phenyl]-3-[(2,4-dimethoxybenzyl)amino]-1-piperidin-3-ylpyrazin-2(1H)-one

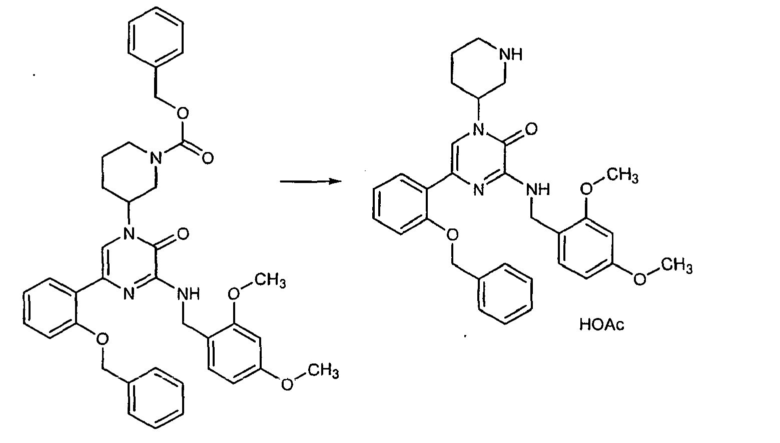

To a solution of benzyl 3-[5-[2-(benzyloxy)phenyl]-3-[(2,4-dimethoxybenzyl)amino]-2-oxopyrazin-1(2H)-yl]piperidine-1-carboxylate from Step 7 (0.1182 g) and 1,3-cyclohexadiene (0.17 mL) in ethanol:acetic acid (3 mL:0.3 mL) was added Pd/C (0.1115 g, 10 wt. % (dry basis), wet, Degussa type). The resulting heterogeneous mixture was heated to 75°C and stirred vigorously for 30 min. The reaction mixture was then filtered through a plug of celite, washing with ethanol and methanol. Concentration under reduced pressure gave the title compound as a beige solid.

Step 9: Preparation of 3-amino-5-(2-hydroxyphenyl)-1-piperidin-3-ylpyrazin-2(1H)-one

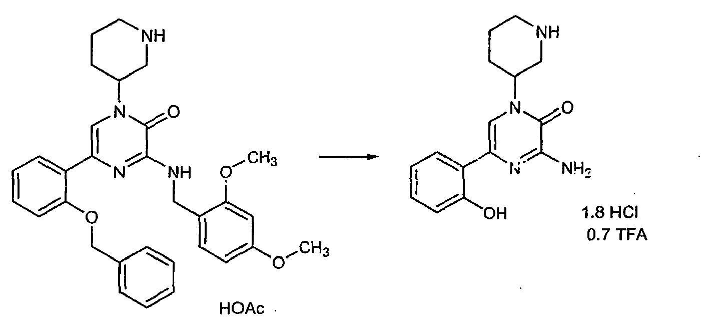

A solution of 5-[2-(benzyloxy)phenyl]-3-[(2,4-dimethoxybenzyl)amino]-1-piperidin-3-ylpyrazin-2(1 H)-one from Step 8 (0.0716 g) in trifluoroacetic acid (18 mL) was heated at 60-69°C for 20 min under N₂ then cooled to room temperature. The reaction was then concentrated under a stream of N₂. Ether was added and then removed under reduced pressure. The resulting solid was triturated with ether and dried. The crude material was purified by reverse-phase HPLC (gradient elution from 1:99 to 50:50 acetonitrile:water, containing trace HCl). Concentration under a stream of N₂ afforded the title compound as a mustard-colored fine powder. The elemental analysis was consistent with a mixed salt containing 1.8 and 0.7 molar equivalents of HCl and trifluoroacetic acid, respectively along with 1.8 H₂O. Calc. C 42.43, H 5.23, N 12.07, Cl 13.75, Found C 42.50, H 5.19, N 11.92, Cl 13.55. ¹H NMR (400 MHz, DMSO-d₆) δ 1.75-2.20 (m, 4H), 2.80-2.94 (m, 1 H), 3.24-3.44 (m, 3H), 5.05-5.17 (m, 1 H), 6.83-6.92 (m, 2H), 7.16-7.23 (m, 1 H), 7.48 (s, 1 H), 7.60 (br d, 1 H), 7.80-8.50 (br s, 2H), 9.02-9.17 (m, 1 H), 9.61 (br d, 1 H). m/z = 287.1 (M+1).

Example 2: 3-amino-5-(5-fluoro-2-hydroxyphenyl)-1-piperidin-3-ylpyrazin-2(1 H)-one

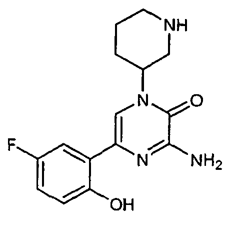

Step 1: Preparation of benzyl 3-[(cyanomethyl)amino]piperidine-1-carboxylate

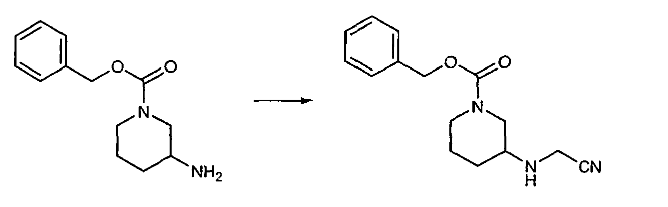

A suspension of 1-benzyloxycarbonyl-3-aminopiperidine (1.1 g), 4 A molecular sieves (3.2 g), and CsOH• H₂O (3.0 g) in DMF (10 mL) was stirred 1 h at room temperature under N₂. Bromoacetonitrile (0.36 mL) was then added by syringe, and the reaction was stirred overnight. Additional bromoacetonitrile (0.35 mL) was then added and the reaction stirred 30 min. The mixture was then diluted with ethyl acetate (75 mL) and filtered. NaOH (1 M, 25 mL), brine (25 mL), and H₂O were added and the phases separated. The aqueous phase was extracted three times with ethyl acetate. The combined organic phases were washed with 1 M NaOH and brine, dried with MgSO₄, and concentrated under reduced pressure. The title compound was purified by flash chromatography to give a brown oil.

Step 2: Preparation of benzyl 3-(3,5-dichloro-2-oxopyrazin-1(2H)-yl)piperidine-1-carboxylate

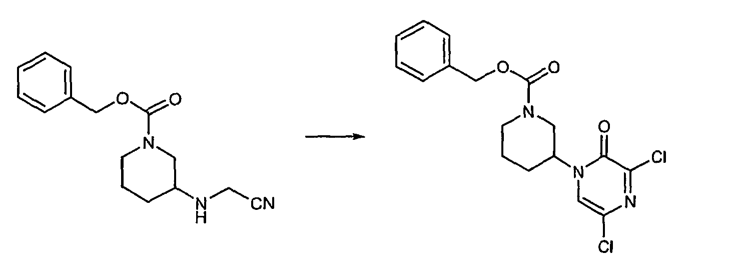

A solution of oxalyl chloride in dichloromethane (2.0 M, 0.60 mL) was added to benzyl 3-[(cyanomethyl)amino]piperidine-1-carboxylate from Step 1 (0.0659 g). N,N-diisopropylethylamine (0.1 mL) was added, resulting in some exothermicity and blackening of the reaction mixture. The resulting mixture was heated in a microwave at 100°C for 15 min then concentrated under a stream of N₂ (see Vekemans et al. (1983) J. Heterocyclic Chem. **20,** 919-923). The title compound was then isolated by flash chromatography (20:1 dichloromethane:ethyl acetate) to give a light brown-pink solid.

Step 3: Preparation of benzyl 3-[5-chloro-3-[(2,4-dimethoxybenzyl)amino]-2-oxopyrazin-1 (2H)-yl]piperidine-1-carboxylate

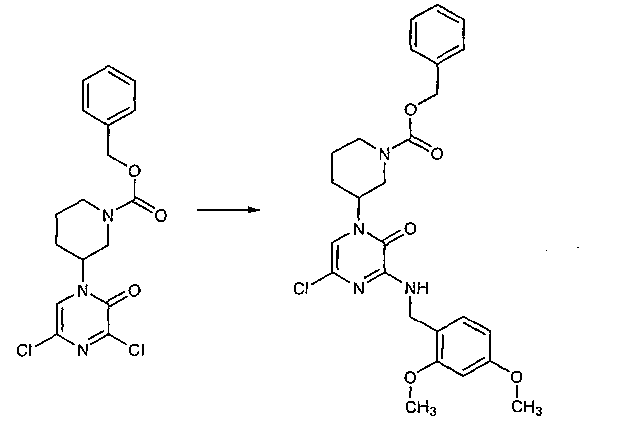

A solution of benzyl 3-(3,5-dichloro-2-oxopyrazin-1 (2H)-yl)piperidine-1-carboxylate from Step 2 (0.0428 g) and 2,4-dimethoxybenzylamine (0.17 mL) in acetonitrile (1 mL) was heated at 110°C for 30 min in a microwave. The desired product was then isolated by flash chromatography (2:1 hexanes:ethyl acetate) to give the title compound as a white solid.

Step 4: Preparation of benzyl 3-[3-[(2,4-dimethoxybenzyl)amino]-5-(5-fluoro-2-hydroxyphenyl)-2-oxopyrazin-1 (2H)-yl]piperidine-1-carboxylate

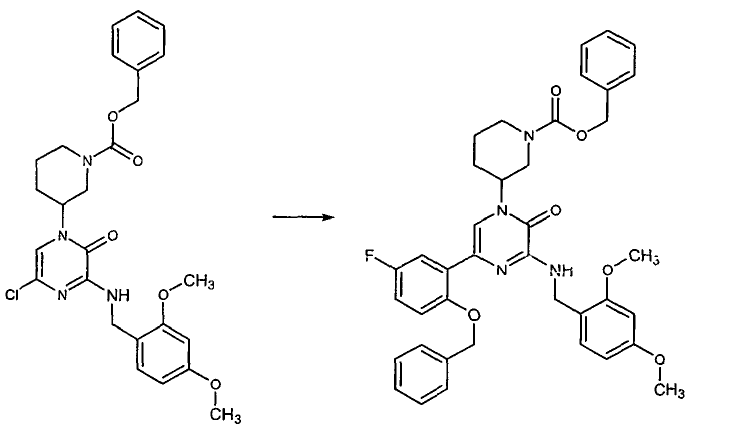

Benzyl 3-[5-chloro-3-[(2,4-dimethoxybenzyl)amino]-2-oxopyrazin-1(2H)-yl]piperidine-1-carboxylate from the previous step (0.513 g, 1 mmol), 2-(benzyloxy)-5-fluorophenylboronic acid (1.4 mmol), dichloro[1,1'-bis(diphenylphosphino)ferrocene]palladium (II) (0.073 g, 0.1 mmol), 1,1'-bis(diphenylphosphino)ferrocene (0.055 g, 0.1 mmol) and cesium carbonate (0.85 g, 2.6 mmol) were added to a scintillation vial with a Teflon-lined cap and three glass beads. The reaction vessel was evacuated and flushed with argon, acetonitrile (3-5 mL) that had been sparged with argon was added, and the reaction was agitated in an orbit shaker at 90°C and followed by HPLC. Upon completion, the reaction was cooled, filtered through celite and evaporated. The resulting oil was partitioned between EtOAc and 5% NaHCO₃, the layers separated and the EtOAc layer dried over MgSO₄ and filtered to give the coupled product. The product was purified using preparative reverse-phase chromatography by running a linear gradient from 15% to 90% acetonitrile/water with 0.1 % TFA buffer.

Step 5: Preparation of 3-[(2,4-dimethoxybenzyl)amino]-5-(5-fluoro-2-hydroxyphenyl)-1-piperidin-3-ylpyrazin-2(1 H)-one

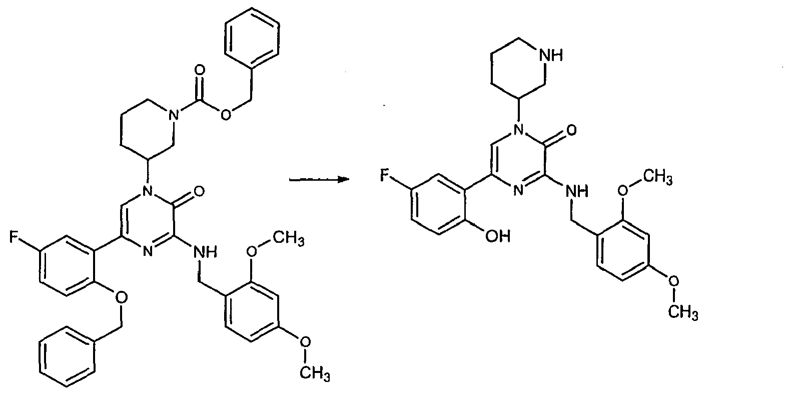

The benzyloxycarbonyl-protecting group was removed via phase-transfer hydrogenolysis (overnight at 50°C) using 10% Pd/C in methanol with ammonium formate (2 equiv.) as the hydrogen source. Reactions were filtered through celite, evaporated and partitioned between EtOAc and 5% NaHCO₃. The EtOAc layer was separated and evaporated to give the title compound.

Step 6: Preparation of 3-amino-5-(5-fluoro-2-hydroxyphenyl)-1-piperidin-3-ylpyrazin-2(1H)-one

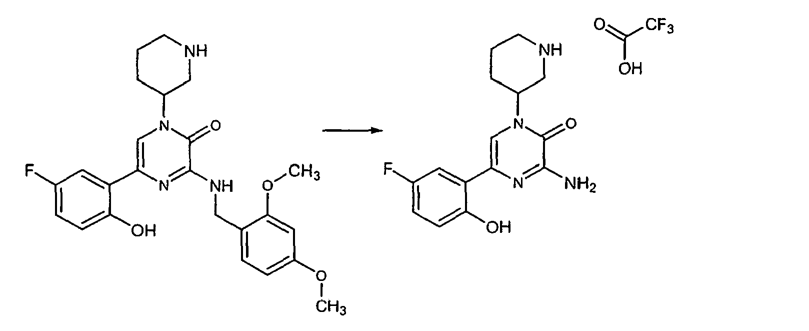

The 2,4-dimethoxybenzyl protecting group was removed by stirring the material in neat TFA at 60°C for two hours. The reaction was evaporated and the title compound was isolated as the TFA salt by purifying using preparative reverse-phase chromatography by running a linear gradient from 5% to 90% acetonitrile/water with 0.1 % TFA buffer. ¹H NMR (300 MHz, DMSO-*d*₆) δ 1.76-2.18 (m, 4 H), 2.83 (m, 1 H), 3.35 (m, 3 H), 5.09 (m, 1 H), 6.78 (m, 1 H), 6.94 (m, 1 H), 7.55 (m, 4 H), 8.83 (br s, 1 H), 9.23 (m, 1 H); MS (ESI⁺) for C₁₅H₁₇FN₄O₂ m/z 305.2 (M+H)⁺.

Example 3: 3-amino-5-(4-fluoro-2-hydroxyphenyl)-1-piperidin-3-ylpyrazin-2(1H)-one

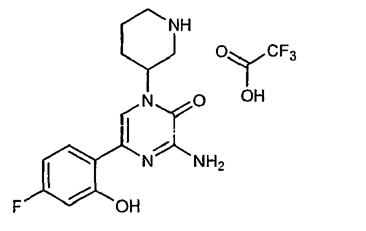

Prepared according to the procedure of Example 2. ¹H NMR (300 MHz, DMSO-*d*₆) δ 1.76-2.18 (m, 4 H), 2.83 (m, 1 H), 3.35 (m, 3 H), 5.09 (m, 1 H), 6.62 (m, 2 H), 7.46 (s, 1 H), 7.53 (br s, 2 H), 7.73 (m, 1 H), 8.83 (br s, 1 H), 9.23 (m, 1 H); MS (ESI⁺) for C₁₅H₁₇FN₄O₂ m/z 305.1 (M+H)⁺.

Example 4: IKK-2 IC₅₀ determination

Materials

SAM^{2™} 96 Biotin capture plates were from Promega. Anti-FLAG affinity resin, FLAG-peptide, NP-40 (Nonidet P-40), BSA, ATP, ADP, AMP, LPS (*E. coli* serotype 0111:B4), and dithiothreitol were obtained from Sigma Chemicals. Antibodies specific for NEMO (IKK-γ) (FL-419), IKK-1 (H-744), IKK-2(H-470) and IκBα(C-21) were purchased from Santa Cruz Biotechnology. Ni-NTA resin was purchased from Qiagen. Peptides were purchased from American Peptide Company. Protease inhibitor cocktail tablets were from Boehringer Mannheim. Sephacryl S-300 column was from Pharmacia LKB Biotechnology. Centriprep-10 concentrators with a molecular weight cutoff of 10 kDa and membranes with molecular weight cut-off of 30 kDa were obtained from Amicon. [γ-³³P] ATP (2500 Ci/mmol) and [γ-³²P] ATP (6000 Ci/mmol) were purchased from Amersham. The other reagents used were of the highest grade commercially available.

Cloning and Expression

cDNAs of human IKK-1 and IKK-2 were amplified by reverse transcriptase-polymerase chain reaction from human placental RNA (Clonetech). hIKK-1 was subcloned into pFastBac HTa (Life Technologies) and expressed as N-terminal His₆-tagged fusion protein. The hIKK-2 cDNA was amplified using a reverse oligonucleotide primer which incorporated the peptide sequence for a FLAG-epitope tag at the C-terminus of the IKK-2 coding region (DYKDDDDKD). The hIKK-2:FLAG cDNA was subcloned into the baculovirus vector pFastBac. The rhIKK-2 (S177S, E177E) mutant was constructed in the same vector used for wild type rhIKK-2 using a QuikChange^{™} mutagenesis kit (Stratagene). Viral stocks of each construct were used to infect insect cells grown in 40L suspension culture. The cells were lysed at a time that maximal expression and rhlKK activity were demonstrated. Cell lysates were stored at - 80°C until purification of the recombinant proteins was undertaken as described below.

Enzyme Isolation

All purification procedures were carried out at 4°C unless otherwise noted. Buffers used are: buffer A: 20 mM Tris-HCl, pH 7.6, containing 50 mM NaCl, 20 mM NaF, 20 mM β-Glycerophosphate, 500 uM sodium orthovanadate, 2.5 mM metabisulfite, 5 mM benzamidine, 1 mM EDTA, 0.5 mM EGTA, 10% glycerol, 1 mM DTT, 1X Complete^{™} protease inhibitors; buffer B: same as buffer A, except 150 mM NaCl, and buffer C: same as buffer A, except 500 mM NaCl.

Isolation of rhIKK-1 homodimer

Cells from an 8-liter fermentation of baculovirus-expressed IKK-1 tagged with His peptide were centrifuged and the cell pellet (MOI 0.1, 1=72 hr) was re-suspended in 100 ml of buffer C. The cells were microfluidized and centrifuged at 100,000 X g for 45 min. The supernatant was collected, imidazole added to the final concentration of 10 mM and incubated with 25 ml of Ni-NTA resin for 2 hrs. The suspension was poured into a 25 ml column and washed with 250 ml of buffer C and then with 125 ml of 50 mM imidazole in buffer C. rhIKK-1 homodimer was eluted using 300 mM imidazole in buffer C. BSA and NP-40 were added to the enzyme fractions to the final concentration of 0.1 %. The enzyme was dialyzed against buffer B, aliquoted and stored at -80°C.

Isolation of rhIKK-2 homodimer

A 10-liter culture of baculovirus-expressing IKK-2 tagged with FLAG peptide was centrifuged and the cell pellet (MOI=0.1 and I=72 hrs) was re-suspended in buffer A. These cells were microfluidized, and centrifuged at 100,000 X g for 45 min. Supernatant was passed over a G-25 column equilibrated with Buffer A. Protein peak was collected and incubated with anti-FLAG affinity resin on a rotator overnight in buffer B. The resin was washed in batch with 10-15 bed volumes of buffer C. Washed resin was poured into a column and rhIKK-2 homodimer was eluted using 5 bed volumes of buffer B containing FLAG peptide. 5 mM DTT, 0.1 % NP-40 and BSA (concentrated to 0.1 % in final amount) was added to the eluted enzyme before concentrating in using an Amicon membrane with a molecular weight cut-off of 30 kDa. Enzyme was aliquoted and stored at -80°C.

Isolation of rhIKK-1/IKK-2 heterodimer

The heterodimer enzyme was produced by coinfection in a baculovirus system (FLAG IKK-2/IKK-1 His; MOI=0.1 and 1=72 hrs). Infected cells were centrifuged and the cell pellet (10.0 g) was suspended in 50 ml of buffer A. The protein suspension was microfluidized and centrifuged at 100,000 X g for 45 min. Imidazole was added to the supernatant to a final concentration of 10 mM. The protein was allowed to bind 25 ml of Ni-NTA resin by mixing for 2 hrs. The protein-resin slurry was poured into a 25 ml column and washed with 250 ml of buffer A containing 10 mM imidazole followed by 125 ml of buffer A containing 50 mM imidazole. Buffer A, containing 300 mM imidazole, was then used to elute the protein. A 75 ml pool was collected and NP-40 was added to a final concentration of 0.1%. The protein solution was then dialyzed against buffer B. The dialyzed heterodimer enzyme was then allowed to bind to 25 ml of anti-FLAG M2 agarose affinity gel overnight with constant mixing. The protein-resin slurry was then centrifuged for 5 min at 2,000 rpm. The supernatant was collected and the resin re-suspended in 100 ml of buffer C containing 0.1 % NP-40. The resin was washed with 375 ml of buffer C containing 0.1 % NP-40. The protein-resin was poured into a 25 ml column and the enzyme eluted using buffer B containing FLAG peptide. Enzyme fractions (100 ml) were collected and concentrated to 20 ml using an Amicon membrane with molecular weight cut-off of 30 kDa. Bovine serum albumin was added to the concentrated enzyme to final concentration of 0.1 %. The enzyme was then aliquoted and stored at-80°C.

Cell Culture

The wild type (wt) human pre-B cell line, 70Z/3, and its mutant; 1.3E2, were generously provided by Dr. Carol Sibley. Wt 70Z/3 and 1.3E2 cells were grown in RPMI 1640 (Gibco) supplemented with 7 % defined bovine serum (Hyclone) and 50 µM 2-mercaptoethanol. Human monocytic leukemia THP-1 cells, obtained from ATCC, were cultured in RPMI 1640 supplemented with 10% defined bovine serum, 10 mM HEPES, 1.0 mM sodium pyruvate and 50 µM 2-mercaptoethanol. For experiments, cells were plated in 6 well plates at 1x10⁶ cells/ml in fresh media. Pre-B cells were stimulated by the addition of 10 µg/ml LPS for varying lengths of time ranging from 0-4 hr. THP-1 cells were stimulated by the addition of 1 µg/ml LPS for 45 minutes. Cells were pelleted, washed with cold 50 mM sodium phosphate buffer, pH 7.4 containing 0.15 M NaCl and lysed at 4°C in 20 mM Hepes buffer, pH 7.6 containing 50 mM NaCl, 1 mM EDTA, 1 mM EGTA, 1 mM sodium orthovanadate, 10 mM β-glycerophosphate, 1 mM NaF, 1 mM PMSF, 1 mM DTT and 0.5 % NP40 (lysis buffer). The cytosolic fractions obtained following centrifugation at 10,000 X g were stored at -80°C until used.

Immunoprecipitation and Western Blotting

SF9 cells paste containing rhIKKs were centrifuged (100,000 X g, 10 min) to remove debris. rhIKKs were immunoprecipitated (100 µg of cell paste) from the cell supernatant using 3 µg of anti-NEMO antibody (FL-419), followed by coupling to protein A sepharose beads. rhIKKs were also immunoprecipitated from affinity chromatography purified protein preparations (1 µg) using anti-FLAG, anti-His or anti-NEMO antibodies (1-4 µg) followed by protein A sepharose coupling. The native, human IKK complex was immunoprecipitated from THP-1 cell homogenates (300 µg/condition) using the anti-NEMO antibody. Immune complexes were pelleted and washed 3 times with 1 ml cold lysis buffer. Immunoprecipitated rhIKKs were chromatographed by SDS-PAGE (8% Tris-glycine) and transferred to nitrocellulose membranes (Novex) and detected by chemiluminescense (SuperSignal) using specific anti-IKK antibodies (IKK-2 H-470, IKK-1 H-744). Native IKK-2, IκBα, and NEMO proteins from cytosolic lysates (20-80 µg) were separated by SDS-PAGE and visualized by chemiluminescense using specific antibodies.

Phosphatase Treatment

Immunoprecipitated rhIKKs were washed 2 times in 50 mM Tris-HCl, pH 8.2 containing 0.1 mM EDTA, 1 mM DTT, 1 mM PMSF and 2 mM MnCl₂ and resuspended in 50 µl. Phosphatase (APPase, 1000 U) was prediluted in the same buffer and added to the IKK samples. Following incubation at room temperature for 30 minutes with intermittent mixing, cold lysis buffer was added to the tubes to stop the reaction. After several washes, 10 % of the beads were removed for Western analysis, and the remaining material was pelleted and resuspended in 100 µl of the buffer used for the *in vitro* kinase assay.

IKK-1 SAM Enzyme Assay

IKK-1 kinase activity was measured using a biotinylated IκBα peptide (Gly-Leu-Lys-Lys-Glu-Arg-Leu-Leu-Asp-Asp-Arg-His-Asp-Ser₃₂-Gly-Leu-Asp-Ser₃₆-Met-Lys-Asp-Glu-Glu), a SAM^{2™} 96 Biotin capture plate and a vacuum system. The standard reaction mixture contained 5 µM biotinylated IκBα peptide, 1 µM [γ-³³P] ATP (about 1 X 10⁵ cpm), 1 mM DTT, 50 mM KCI, 2 mM MgCl₂, 2 mM MnCl₂, 10 mM NaF, 25 mM Hepes buffer, pH. 7.6 and enzyme solution (1-10 µl) in a final volume of 50 µl. After incubation at 25°C for 30 min, 25 µl of the reaction mixture was withdrawn and added to a SAM^{2™} 96 Biotin capture 96-well plate. Each well was then washed successively with 800 µl 2 M NaCl, 1.2 ml of NaCl containing 1% H₃PO₄, 400 µl H₂O, and 200 µl 95% ethanol. The plate was allowed to dry in a hood at 25°C for 1 hr and then 25 µl of scintillation fluid (Microscint 20) was added to each well. Incorporation of [γ-³³P] ATP was measured using a Top-Count NXT (Packard). Under each assay condition, the degree of phosphorylation of IκBα peptide substrate was linear with time and concentration for all purified enzymes. Results from the biotinylated peptide assay were confirmed by SDS-PAGE analysis of kinase reaction utilizing a GST-IκBα₁₋₅₄ and [γ-³²P] ATP. The resulting radiolabeled substrate was quantitated by Phosphoimager (Molecular Dynamics). An ion exchange resin assay was also employed using [γ-³³P] ATP and GST-IκBα₁₋₅₄ fusion protein as the substrates. Each assay system yielded consistent results in regard to Kₘ and specific activities for each of the purified kinase isoforms. One unit of enzyme activity was defined as the amount required to catalyze the transfer of 1 nmole of phosphate from ATP to IκBα peptide per min. Specific activity was expressed as units per mg of protein. For experiments related to Kₘ determination of purified enzymes, various concentrations of ATP or IκBα peptide were used in the assay at either a fixed IκBα or ATP concentration. For IκBα peptide Kₘ, assays were carried out with 0.1 µg of enzyme, 5 µM ATP and IκBα peptide from 0.5 to 20µM. For ATP Kₘ, assays were carried out with 0.1 µg of enzyme, 10 µM IκBα peptide and ATP from 0.1 to 10µM. For Kₘ determination of rhlKK-1 homodimer, due to its low activity and higher Kₘ for IκBα peptide, rhlKK-1 homodimer (0.3 µg) was assayed with 125 µM IκBα peptide and a 5-fold higher specific activity of ATP (from 0.1 to 10 µM) for ATP Kₘ experiments and a 5-fold higher specific activity of 5 µM ATP and IκBα peptide (from 5 to 200 µM) for IκBα peptide Kₘ experiments.

IKK heterodimer Resin Enzyme Assay

IKK heterodimer kinase activity was measured using a biotinylated IκBα peptide (Gly-Leu-Lys-Lys-Glu-Arg-Leu-Leu-Asp-Asp-Arg-His-Asp-Ser₃₂-Gly-Leu-Asp-Ser₃₆-Met-Lys-Asp-Glu-Glu) (American Peptide Co.). 20 ul of the standard reaction mixture contained 5 µM biotinylated IκBα peptide, 0.1 µCi/reaction [γ-³³P] ATP (Amersham) (about 1 X 10⁵ cpm), 1 µM ATP (Sigma), 1 mM DTT (Sigma), 2 mM MgCl₂ (Sigma), 2 mM MnCl₂ (Sigma), 10 mM NaF (Sigma), 25 mM Hepes (Sigma) buffer, pH 7.6 and 20 µl enzyme solution and 10 µl inhibitor in a final volume of 50 µl. After incubation at 25°C for 30 min, 150 µl resin (Dowex anion-exchange resin AG1X8 200-400 mesh) in 900 mM formate, pH 3.0 was added to each well to stop the reaction. Resin was allowed to settle for one hour and 50 ul of supernatant was removed to a Micolite-2 flat bottom plate (Dynex). 150 µl of scintillation fluid (Microscint 40) (Packard) was added to each well. Incorporation of [γ-³³P] ATP was measured using a Top-Count NXT (Packard).

IKK-2 Resin Enzyme Assay

IKK-2 kinase activity was measured using a biotinylated IκBα peptide (Gly-Leu-Lys-Lys-Glu-Arg-Leu-Leu-Asp-Asp-Arg-His-As p-Ser₃₂-Gly-Leu-Asp-Ser₃₆-Met-Lys-Asp-Glu-Glu) (American Peptide Co.). 20 ul of the standard reaction mixture contained 5 µM biotinylated IκBα peptide, 0.1 µCi/reaction [γ-³³P] ATP (Amersham) (about 1 X 10⁵ cpm), 1 µM ATP (Sigma), 1 mM DTT (Sigma), 2 mM MgCl₂ (Sigma), 2 mM MnCl₂ (Sigma), 10 mM NaF (Sigma), 25 mM Hepes (Sigma) buffer, pH 7.6 and 20 µl enzyme solution and 10 ul inhibitor in a final volume of 50 µl. After incubation at 25°C for 30 min, 150 µl resin (Dowex anion-exchange resin AG1X8 200-400 mesh) in 900 mM formate, pH 3.0 was added to each well to stop the reaction. Resin was allowed to settle for one hour and 50 ul of supernatant was removed to a Micolite-2 flat bottom plate (Dynex). 150 µl of scintillation fluid (Microscint 40) (Packard) was added to each well. Incorporation of [γ-³³P] ATP was measured using a Top-Count NXT (Packard).

IKK-2 IC₅₀ values obtained from the assay described above are shown in the table below.

| **Example** | **IKK-2 IC₅₀ (µM)** |
|---|---|
| 1 | 1.08 |
| 2 | 0.903 |
| 3 | 1.79 |

## Claims

1. A compound of Formula I : 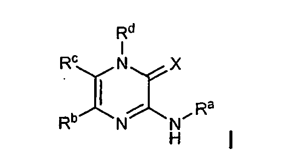 wherein X is selected from the group consisting of O, S, and NR^{5a};
wherein R^{a} and R^{c} are independently selected from the group consisting of hydrido, hydroxyl, alkoxy, alkyl, haloalkyl, aryl, and heteroaryl;
wherein R^{b} is a 3- to 12-membered cyclic moiety selected from the group consisting of cycloalkyl, cycloalkenyl, aryl, heterocycloalkyl, heterocycloalkenyl, and heteroaryl, wherein R^{b} is optionally substituted by one or more substituents selected from the group consisting of R², cycloalkyl, and cycloalkylalkyl;
wherein R^{d} is selected from the group consisting of -(CH₂)_{q}NH₂, -(CH₂)_{q}NHR², and a 5- to 7-membered heterocycloalkyl having ring members selected from the group consisting of carbon and nitrogen, wherein said heterocycloalkyl may be optionally substituted by one or more substituents selected from the group consisting of R²;
wherein R² is selected from the group consisting of halo, alkylsulfinyl, alkylsulfonyl, cyano, alkoxycarbonyl, alkyl, haloalkyl, hydroxyalkyl, haloalkoxy, nitro, acylamino, R⁷, -OR³, -(CH₂)ₘOR³, -(CH₂)ₚCO₂R³, -SR³, -SO₂N(R^{4a})R^{4b}, -NR^{5a}R^{5b}, -NR^{5a}COR^{5b}, -NR^{5a}CO(OR^{5b}), -NR^{5a}SO₂R⁶, -NR^{5a}SO₂N(R^{6a})R^{6b}, -NR^{5a}CON(R^{6a})R^{6b}, -COR^{5a}, and -CON(R^{4a})R^{4b};
wherein R³, R^{4a}, and R^{4b} are independently selected from the group consisting of hydrido, aryl, heteroaryl, heteroaralkyl, alkyl, haloalkyl, alkenyl, alkynyl, hydroxyalkyl, aminoalkyl, alkylaminoalkyl, N,N-dialkylaminoalkyl, alkoxy, alkoxyalkyl, heterocycloalkyl, heterocycloalkenyl, cycloalkyl, cycloalkylalkyl, aralkyl, and aralkylamino wherein said aryl is optionally substituted with one or more radicals selected from the group consisting of alkyl, aminoalkyl, alkoxy and halo, wherein R^{4a} and R^{4b} may be taken together to form a 3- to 7-membered heterocyclic ring having from 1 to 3 heteroatoms selected from S, SO, SO₂, O, N, and NR^{5a};
wherein R^{5a} and R^{5b} are independently selected from the group consisting of hydrido, alkyl, aryl, heteroaryl, aralkyl, heterocycloalkenyl, cycloalkyl, heterocycloalkyl, haloalkyl, aralkylamino, amino, aminoalkyl, aminoacyl, nitro, azido, and heteroaralkyl, wherein said alkyl, aryl, heteroaryl, aminoalkyl, and aralkyl moieties are optionally substituted with one or more substituents selected from the group consisting of alkylsulfonamido, sulfamyl, alkyl, alkylthio, alkylsulfinyl, alkylsulfonyl, N-alkylamino, aminoalkyl, alkylaminoalkyl, alkoxy, halo, acyloxy, oxy, formyl, haloalkyl, cyano, haloalkoxy, acyl, carboxyl, hydroxy, hydroxyalkoxy, phenoxy, nitro, azido, benzyloxy, N,N-dialkylaminoacyl, thioalkyl, aminoacyloxy, thiocyanato, isothiocyanato, alkyldioxy, hydroxyalkyl, N-alkylamino, alkoxycarbonyl, alkoxyalkyl, alkenylamino, alkynylamino, alkenyl, alkynyl, N,N-dialkylaminoalkoxy, heterocycloalkyl, heterocycloalkenyl, and heteroaryl;
wherein R^{6a} and R^{6b} are independently selected from the group consisting of hydrido, alkyl, heteroaryl, heterocycloalkenyl, haloalkyl, aralkylamino, heteroaralkyl, aryl, and aralkyl, wherein said aryl, heteroaryl, heterocycloalkenyl, and aralkyl moieties are optionally substituted with one or more substituents selected from alkyl, alkoxy, halo, haloalkyl, cyano, haloalkoxy, acyl, carboxyl, hydroxy, hydroxyalkoxy, phenoxy, benzyloxy, N,N-dialkylaminoalkoxy, heterocycloalkyl, heterocycloalkenyl, and heteroaryl, wherein R^{6a} and R^{6b} may be taken together to form a 3- to 7-membered heterocyclic ring having 1 to 3 heteroatoms selected from S, SO, SO₂, O, N, and NR^{5a};
wherein R⁷ is selected from the group consisting of aryl, heterocycloalkenyl, heteroaryl, and alkenyl, wherein R⁷ is optionally substituted with one or more substituents selected from the group consisting of R^{5a};
wherein n is 1, 2, or 3
wherein m is 1, 2, or 3;
wherein p is 0, 1, or 2;
wherein q is an integer between 0 and 9;
or a pharmaceutically acceptable salt thereof.

2. A compound according to claim 1:
wherein R^{a} and R^{c} are independently selected from the group consisting of hydrido, hydroxyl, C₁₋₆ alkoxy, C₁₋₆ alkyl, C₁₋₆ haloalkyl, C₃₋₁₂ aryl, and 3- to 12-membered heteroaryl
wherein R^{b} is a 3- to 12-membered cyclic moiety selected from the group consisting of C₃₋₁₂cycloalkyl, C₃₋₁₂ cycloalkenyl, C₃₋₁₂ aryl, 3- to 12-membered heterocycloalkyl, 3- to 12-membered 3- to 12-membered heterocycloalkenyl, and 3- to 12-membered heteroaryl, wherein R^{b} is optionally substituted by one or more substituents selected from the group consisting of R², C₃₋₁₂ cycloalkyl, and C₄₋₁₈ cycloalkylalkyl;
wherein R^{d} is selected from the group consisting of -(CH₂)_{q}NH₂, -(CH₂)_{q}NHR², and a 5- to 7-membered heterocycloalkyl having ring members selected from the group consisting of carbon and nitrogen, wherein said heterocycloalkyl may be optionally substituted by one or more substituents selected from the group consisting of R²;
wherein R² is selected from the group consisting of halo, C₁₋₆ alkylsulfinyl, C₁₋₆ alkylsulfonyl, cyano, C₂₋₇ alkoxycarbonyl, C₁₋₆ alkyl, C₁₋₆ haloalkyl, C₁₋₆ hydroxyalkyl, C₁₋₆ haloalkoxy, nitro, C₂₋₁₀ acylamino, R⁷, -OR³, -(CH₂)ₘOR³, -(CH₂)ₚCO₂R³, -SR³, -SO₂N(R^{4a})R^{4b}, -NR^{5a}R^{5b}, -NR^{5a}COR^{5b}, -NR^{5a}CO(OR^{5b}), -NR^{5a}SO₂R⁶, -NR^{5a}SO₂N(R^{6a})R^{6b}, -NR^{5a}CON(R^{6a})R^{6b}, -COR^{5a}, and -CON(R^{4a})R^{4b};
wherein R³, R^{4a}, and R^{4b} are independently selected from the group consisting of hydrido, C₃₋₁₂ aryl, 3- to 12-membered heteroaryl, 4- to 18-membered heteroaralkyl, C₁₋₆ alkyl, C₁₋₆ haloalkyl, C₂₋₆ alkenyl, C₂₋₆ alkynyl, C₁₋₆ hydroxyalkyl, C₁₋₆ aminoalkyl, C₂₋₁₂ alkylaminoalkyl, N-N-di(C₁₋₆alkyl)amino(C₁₋₆ alkyl), C₁₋₆ alkoxy, C₂₋₁₂ alkoxyalkyl, 3- to 12-membered heterocycloalkyl, 3- to 12-membered heterocycloalkenyl, C₃₋₁₂ cycloalkyl, C₄₋₁₈ cycloalkylalkyl, C₄₋₁₈ aralkyl, and C₄₋₁₈ aralkylamino, wherein said aryl is optionally substituted with one or more radicals selected from the group consisting of C₁₋₆alkyl, C₁₋₆ aminoalkyl, C₁₋₆ alkoxy and halo, wherein R^{4a} and R^{4b} may be taken together to form a 3- to 7-membered heterocyclic ring having from 1 to 3 heteroatoms selected from S, SO,SO₂, O, N, and NR^{5a};
wherein R^{5a} and R^{5b} are independently selected from the group consisting of hydrido, C₁₋₆ alkyl, C₃₋₁₂ aryl, 3- to 12-membered heteroaryl, C₄₋₁₈ aralkyl, 3- to 12-membered heterocycloalkenyl, C₃₋₁₂ cycloalkyl, 3- to 12-membered heterocycloalkyl, C₁₋₆ haloalkyl, C₄₋₁₈ aralkylamino, amino, C₁₋₆ aminoalkyl, C₂₋₁₀ aminoacyl, nitro, azido, and 4- to 18-membered heteroaralkyl, wherein said alkyl, aryl, heteroaryl, aminoalkyl, and aralkyl moieties are optionally substituted with one or more substituents selected from the group consisting of C₁₋₆ alkylsulfonamido, sulfamyl, C₁₋₆ alkyl, C₁₋₆ alkylthio, C₁₋₆ alkylsulfinyl, C₁₋₆ alkylsulfonyl, N-(C₁₋₆ alkyl)amino, C₁₋₆ aminoalkyl, C₂₋₁₂ alkylaminoalkyl, C₁₋₆ alkoxy, halo, C₂₋₁₀ acyloxy, oxy, formyl, C₁₋₆ haloalkyl, cyano, C₁₋₆ haloalkoxy, C₂₋₁₀ acyl, carboxyl, hydroxy, C₁₋₆ hydroxyalkoxy, phenoxy, nitro, azido, benzyloxy, N,N-di(C₁₋₆ alkyl)amino(C₂₋₁₀ acyl), C₁₋₆ thioalkyl, C₂₋₁₀ aminoacyloxy, thiocyanato, isothiocyanato, C₁₋₆ alkyldioxy, C₁₋₆ hydroxyalkyl, N-(C₁₋₆ alkyl)amino, C₂₋₇ alkoxycarbonyl, C₂₋₁₂ alkoxyalkyl, C₂₋₆ alkenylamino, C₂₋₆ alkynylamino, C₂₋₆ alkenyl, C₂₋₆ alkynyl, N,N-di(C₁₋₆ alkyl)amino(C₁₋₆ alkoxy), 3- to 12-membered heterocycloalkenyl, 3- to 12-membered heterocycloalkyl, and 3- to 12-membered heteroaryl;
wherein R^{6a} and R^{6b} are independently selected from the group consisting of hydrido, C₁₋₆ alkyl, 3- to 12-membered heteroaryl, 3- to 12-membered heterocycloalkenyl, C₁₋₆ haloalkyl, C₄₋₁₈ aralkylamino, 4- to 18-membered heteroaralkyl, C₃₋₁₂ aryl, and C₄₋₁₈ aralkyl, wherein said aryl, heteroaryl, heterocycloalkenyl, and aralkyl moieties are optionally substituted with one or more substituents selected from C₁₋₆ alkyl, C₁₋₆ alkoxy, halo, C₁₋₆ haloalkyl, cyano, C₁₋₆ haloalkoxy, C₂₋₁₀ acyl, carboxyl, hydroxy, C₁₋₆ hydroxyalkoxy, phenoxy, benzyloxy, N,N-di(C₁₋₆ alkyl)amino(C₁₋₆ alkoxy), 3- to 12-membered heterocycloalkenyl, 3- to 12-membered heterocycloalkyl, and 3- to 12-membered heteroaryl, wherein R^{6a} and R^{6b} may be taken together to form a 3- to 7-membered heterocyclic ring having 1 to 3 heteroatoms selected from S, SO, SO₂, O, N, and NR^{5a}; and
wherein R⁷ is selected from the group consisting of C₃₋₁₂ aryl, 3- to 12-membered heterocycloalkenyl, 3- to 12-membered heteroaryl, and C₂₋₆ alkenyl,
wherein R⁷ is optionally substituted with one or more substituents selected from the group consisting of R^{5a};
or a pharmaceutically acceptable salt thereof.

3. A compound according to either of claims 1 or 2:
wherein R^{a} and R^{c} are independently selected from the group consisting of hydrido, hydroxyl, methoxy, ethoxy, propoxy, butoxy, methyl, ethyl, propyl, butyl, pentyl, hexyl, chloromethyl, dichloromethyl, trichloromethyl, fluoromethyl, difluoromethyl, trifluoromethyl, phenyl, biphenyl, naphthyl, indenyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl;
wherein R^{b} is a 3- to 12-membered cyclic moiety selected from the group consisting of cyclopropyl, cyclobutyl, cyclopentyl, cyclohexyl, cyclopropenyl, cyclobutenyl, cyclopentenyl, cyclohexenyl, phenyl, biphenyl, naphthyl, indenyl, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl, wherein R^{b} is optionally substituted by one or more substituents selected from the group consisting of R², cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl, and cyclohexylethyl;
wherein R^{d} is selected from the group consisting of -(CH₂)_{q}NH₂, -(CH₂)_{q}NHR², piperidinyl, pyrrolidinyl, pyrazolidinyl, and imidazolidinyl, wherein said piperidinyl, pyrrolidinyl, pyrazolidinyl, or imidazolidinyl may be optionally substituted by one or more substituents selected from the group consisting of R²;
wherein R² is selected from the group consisting of chloro, fluoro, bromo, iodo, methylsulfinyl, ethylsulfinyl, propylsulfinyl, butylsulfinyl, methylsulfonyl, ethylsulfonyl, propylsulfonyl, butylsulfonyl, cyano, methoxycarbonyl, ethoxycarbonyl, propoxycarbonyl, butoxycarbonyl, methyl, ethyl, propyl, butyl, pentyl, hexyl, chloromethyl, dichloromethyl, trichloromethyl, fluoromethyl, difluoromethyl, trifluoromethyl, hydroxymethyl, hydroxyethyl, hydroxypropyl, hydroxybutyl, hydroxypentyl, hydroxyhexyl, chloromethoxy, dichloromethoxy, trichloromethoxy, fluoromethoxy, difluoromethoxy, trifluoromethoxy, nitro, methylcarbonylamino, ethylcarbonylamino, propylcarbonylamino, butylcarbonylamino, pentylcarbonylamino, hexylcarbonylamino, phenylcarbonylamino, benzylcarbonylamino, R⁷, -OR³, -(CH₂)ₘOR³, -(CH₂)ₚCO₂R³, -SR³, -SO₂N(R^{4a})R^{4b}, -NR^{5a}R^{5b}, -NR^{5a}COR^{5b}, -NR^{5a}CO(OR^{5b}), -NR^{5a}SO₂R⁶, -NR^{5a}SO₂N(R^{6a})R^{6b}, -NR^{5a}CON(R^{6a})R^{6b}, -COR^{5a}, and -CON(R^{4a})R^{4b};
wherein R³, R^{4a}, and R^{4b} are independently selected from the group consisting of hydrido, phenyl, biphenyl, naphthyl, indenyl, pyridinyl, benzothiophenyl, indolyl, isoquinolinyl, quinolinyl, thienyl, pyrrolyl, furyl, pyrazolyl, imidazolyl, isoxazolyl, oxazolyl, isoindoledionyl, pyridinylmethyl, pyridinylethyl, benzothiophenylmethyl, benzothiophenylethyl, indolylmethyl, indolylethyl, isoquinolinylmethyl, isoquinolinylethyl, quinolinylmethyl, quinolinylethyl, thienylmethyl, thienylethyl, pyrrolylmethyl, pyrrolylethyl, furylmethyl, furylethyl, pyrazolylmethyl, pyrazolylethyl, imidazolylmethyl, imidazolylethyl, isoxazolylmethyl, isoxazolylethyl, oxazolylmethyl, oxazolylethyl, isoindoledionylmethyl, isoindoledionylethyl, methyl, ethyl, propyl, butyl, pentyl, hexyl, chloromethyl, dichloromethyl, trichloromethyl, fluoromethyl, difluoromethyl, trifluoromethyl, ethenyl, propenyl, butenyl, pentenyl, ethynyl, propynyl, butynyl, pentynyl, hydroxymethyl, hydroxyethyl, hydroxypropyl, hydroxybutyl, hydroxypentyl, hydroxyhexyl, aminomethyl, aminoethyl, aminopropyl, aminobutyl, aminopentyl, aminohexyl, methylaminomethyl, ethylaminomethyl, propylaminomethyl, methylaminoethyl, ethylaminoethyl, propylaminoethyl, methylaminopropyl, ethylaminopropyl, propylaminopropyl, methylaminobutyl, ethylaminobutyl, propylaminobutyl, methylaminopentyl, ethylaminopentyl, propylaminopentyl, methylaminohexyl, ethylaminohexyl, propylaminohexyl, N,N-dimethylaminomethyl, N,N-dimethylaminoethyl, N-methyl-N-ethylaminomethyl, N-methyl-N-ethylaminoethyl, N-methyl-N-propylaminomethyl, N-methyl-N-propylaminoethyl, N,N-diethylaminomethyl, N,N-diethylaminoethyl, N-ethyl-N-propylaminomethyl, N-ethyl-N-propylaminoethyl, N,N-dipropylaminomethyl, N,N-dipropylaminoethyl, methoxy, ethoxy, propoxy, butoxy, methoxymethyl, methoxyethyl, methoxypropyl, ethoxymethyl, ethoxyethyl, ethoxypropyl, propoxymethyl, propoxyethyl, propoxypropyl, butoxymethyl, butoxyethyl, butoxypropyl, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl, cyclohexylethyl, benzyl, phenylethyl, benzylamino, and phenylethylamino,
wherein said phenyl, biphenyl, naphthyl, or indenyl moiety is optionally substituted with one or more radicals selected from the group consisting of methyl, ethyl, propyl, butyl, pentyl, hexyl, aminomethyl, aminoethyl, aminopropyl, aminobutyl, aminopentyl, aminohexyl, methoxy, ethoxy, propoxy, butoxy, chloro, fluoro, bromo, and iodo, wherein R^{4a} and R^{4b} may be taken together to form a moiety selected from the group consisting of piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl;
wherein R^{5a} and R^{5b} are independently selected from the group consisting of hydrido, methyl, ethyl, propyl, butyl, pentyl, hexyl, phenyl, biphenyl, naphthyl, indenyl, pyridinyl, benzothiophenyl, indolyl, isoquinolinyl, quinolinyl, thienyl, pyrrolyl, furyl, pyrazolyl, imidazolyl, isoxazolyl, oxazolyl, isoindoledionyl, benzyl, phenylethyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, cyclopropyl, cyclobutyl, cyclopentyl, cyclohexyl, piperidinyl, pyrrolidinyl, pyrazolidinyl, imidazolidinyl, isoxazolidinyl, oxazolidinyl, chloromethyl, dichloromethyl, trichloromethyl, fluoromethyl, difluoromethyl, trifluoromethyl, benzylamino, phenylethylamino, amino, aminomethyl, aminoethyl, aminopropyl, aminobutyl, -aminopentyl, aminohexyl, aminomethylcarbonyl, aminoethylcarbonyl, aminopropylcarbonyl, aminobutylcarbonyl, aminopentylcarbonyl, aminohexylcarbonyl, aminophenylcarbonyl, aminobenzylcarbonyl, nitro, azido, pyridinylmethyl, pyridinylethyl, benzothiophenylmethyl, benzothiophenylethyl, indolylmethyl, indolylethyl, isoquinolinylmethyl, isoquinolinylethyl, quinolinylmethyl, quinolinylethyl, thienylmethyl, thienylethyl, pyrrolylmethyl, pyrrolylethyl, furylmethyl, furylethyl, pyrazolylmethyl, pyrazolylethyl, imidazolylmethyl, imidazolylethyl, isoxazolylmethyl, isoxazolylethyl, oxazolylmethyl, oxazolylethyl, isoindoledionylmethyl, and isoindoledionylethyl, wherein said methyl, ethyl, propyl, butyl, pentyl, hexyl, phenyl, biphenyl, naphthyl, indenyl, pyridinyl, benzothiophenyl, indolyl, isoquinolinyl, quinolinyl, thienyl, pyrrolyl, furyl, pyrazolyl, imidazolyl, isoxazolyl, oxazolyl, isoindoledionyl, aminomethyl, aminoethyl, aminopropyl, aminobutyl, aminopentyl, aminohexyl, benzyl, and phenylethyl moieties are optionally substituted with one or more substituents selected from the group consisting of methylsulfonamido, ethylsulfonamido, propylsulfonamido, butylsulfonamido, sulfamyl, methyl, ethyl, propyl, butyl, pentyl, hexyl, methylthio, ethylthio, propylthio, butylthio, methylsulfinyl, ethylsulfinyl, propylsulfinyl, butylsulfinyl, methylsulfonyl, ethylsulfonyl, propylsulfonyl, butylsulfonyl, N-methylamino, N-ethylamino, N-propylamino, aminomethyl, aminoethyl, aminopropyl, aminobutyl, aminopentyl, aminohexyl, methylaminomethyl, ethylaminomethyl, propylaminomethyl, methylaminoethyl, ethylaminoethyl, propylaminoethyl, methylaminopropyl, ethylaminopropyl, propylaminopropyl, methylaminobutyl, ethylaminobutyl, propylaminobutyl; methylaminopentyl, ethylaminopentyl, propylaminopentyl, methylaminohexyl, ethylaminohexyl, propylaminohexyl, methoxy, ethoxy, propoxy, butoxy, chloro, fluoro, bromo, iodo, acyloxy, oxy, formyl, chloromethyl, dichloromethyl, trichloromethyl, fluoromethyl, difluoromethyl, trifluoromethyl, cyano, chloromethoxy, dichloromethoxy, trichloromethoxy, fluoromethoxy, difluoromethoxy, trifluoromethoxy, methylcarbonyl, ethylcarbonyl, propylcarbonyl, butylcarbonyl, pentylcarbonyl, hexylcarbonyl, phenylcarbonyl, benzylcarbonyl, carboxyl, hydroxy, hydroxymethoxy, hydroxyethoxy, hydroxypropoxy, hydroxybutoxy, phenoxy, nitro, azido, benzyloxy, N,N-dimethylaminomethylcarbonyl, N,N-dimethylaminoethylcarbonyl, N,N-dimethylaminophenylcarbonyl, N-methyl-N-ethylaminomethylcarbonyl, N-methyl-N-ethylaminoethylcarbonyl, N-methyl-N-ethylaminophenylcarbonyl, N-methyl-N-propylaminomethylcarbonyl, N-methyl-N-propylaminoethylcarbonyl, N-methyl-N-propylaminophenylcarbonyl, N,N-diethylaminomethylcarbonyl, N,N-diethylaminoethylcarbonyl, N,N-diethylaminophenylcarbonyl, N-ethyl-N-propylaminomethylcarbonyl, N-ethyl-N-propylaminoethylcarbonyl, N-ethyl-N-propylaminophenylcarbonyl, N,N-dipropylaminomethylcarbonyl, N,N-dipropylaminoethylcarbonyl, N,N-dipropylaminophenylcarbonyl, thiomethyl, thioethyl, thiopropyl, thiobutyl, thiopentyl, thiohexyl, aminomethylcarbonyloxy, aminoethylcarbonyloxy, aminopropylcarbonyloxy, aminobutylcarbonyloxy, aminopentylcarbonyloxy, aminohexylcarbonyloxy, aminophenylcarbonyloxy, aminobenzylcarbonyloxy, thiocyanato, isothiocyanato, methyldioxy, ethyldioxy, propyldioxy, butyldioxy, pentyldioxy, hexyldioxy, hydroxymethyl, hydroxyethyl, hydroxypropyl, hydroxybutyl, hydroxypentyl, hydroxyhexyl, N-methylamino, N-ethylamino, N-propylamino, methoxycarbonyl, ethoxycarbonyl, propoxycarbonyl, butoxycarbonyl, methoxymethyl, methoxyethyl, methoxypropyl, ethoxymethyl, ethoxyethyl, ethoxypropyl, propoxymethyl, propoxyethyl, propoxypropyl, butoxymethyl, butoxyethyl, butoxypropyl, ethenylamino, propenylamino, butenylamino, pentenylamino, ethynylamino, propynylamino, butynylamino, pentynylamino, ethenyl, propenyl, butenyl, pentenyl, ethynyl, propynyl, butynyl, pentynyl, N,N-dimethylaminomethoxy, N,N-dimethylaminoethoxy, N-methyl-N-ethylaminomethoxy, N-methyl-N-ethylaminoethoxy, N-methyl-N-propylaminomethoxy, N-methyl-N-propylaminoethoxy, N,N-diethylaminomethoxy, N,N-diethylaminoethoxy, N-ethyl-N-propylaminomethoxy, N-ethyl-N-propylaminoethoxy, N,N-dipropylaminomethoxy, N,N-dipropylaminoethoxy, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl;
wherein R^{6a} and R^{6b} are independently selected from the group consisting of hydrido, methyl, ethyl, propyl, butyl, pentyl, hexyl, pyridinyl, benzothiophenyl, indolyl, isoquinolinyl, quinolinyl, thienyl, pyrrolyl, furyl, pyrazolyl, imidazolyl, isoxazolyl, oxazolyl, isoindoledionyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, chloromethyl, dichloromethyl, trichloromethyl, fluoromethyl, difluoromethyl, trifluoromethyl, benzylamino, phenylethylamino, pyridinylmethyl, pyridinylethyl, benzothiophenylmethyl, benzothiophenylethyl, indolylmethyl, indolylethyl, isoquinolinylmethyl, isoquinolinylethyl, quinolinylmethyl, quinolinylethyl, thienylmethyl, thienylethyl, pyrrolylmethyl, pyrrolylethyl, furylmethyl, furylethyl, pyrazolylmethyl, pyrazolylethyl, imidazolylmethyl, imidazolylethyl, isoxazolylmethyl, isoxazolylethyl, oxazolylmethyl, oxazolylethyl, isoindoledionylmethyl, isoindoledionylethyl, phenyl, biphenyl, naphthyl, indenyl, benzyl, and phenylethyl, wherein said phenyl, biphenyl, naphthyl, indenyl, pyridinyl, benzothiophenyl, indolyl, isoquinolinyl, quinolinyl, thienyl, pyrrolyl, furyl, pyrazolyl, imidazolyl, isoxazolyl, oxazolyl, isoindoledionyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, benzyl, and phenylethyl moieties are optionally substituted with one or more substituents selected from methyl, ethyl, propyl, butyl, pentyl, hexyl, methoxy, ethoxy, propoxy, butoxy, chloro, fluoro, bromo, iodo, chloromethyl, dichloromethyl, trichloromethyl, fluoromethyl, difluoromethyl, trifluoromethyl, cyano, chloromethoxy, dichloromethoxy, trichloromethoxy, fluoromethoxy, difluoromethoxy, trifluoromethoxy, methylcarbonyl, ethylcarbonyl, propylcarbonyl, butylcarbonyl, pentylcarbonyl, hexylcarbonyl, phenylcarbonyl, benzylcarbonyl, carboxyl, hydroxy, hydroxymethoxy, hydroxyethoxy, hydroxypropoxy, hydroxybutoxy, phenoxy, benzyloxy, N,N-dimethylaminomethoxy, N,N-dimethylaminoethoxy, N-methyl-N-ethylaminomethoxy, N-methyl-N-ethylaminoethoxy, N-methyl-N-propylaminomethoxy, N-methyl-N-propylaminoethoxy, N,N-diethylaminomethoxy, N,N-diethylaminoethoxy, N-ethyl-N-propylaminomethoxy, N-ethyl-N-propylaminoethoxy, N,N-dipropylaminomethoxy, N,N-dipropylaminoethoxy, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl, wherein R^{6a} and R^{6b} may be taken together to form a moiety selected from the group consisting of piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl: and
wherein R⁷ is selected from the group consisting of phenyl, biphenyl, naphthyl, indenyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, isoindoledionyl, ethenyl, propenyl, butenyl, and pentenyl, wherein R⁷ is optionally substituted with one or more substituents selected from the group consisting of R^{5a};
or a pharmaceutically acceptable salt thereof.

4. A compound according to claim 1 wherein the compound of Formula I is a compound of Formula II:
wherein s is 1, 2, or 3; 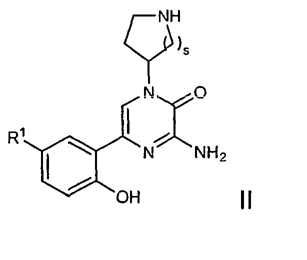 wherein R¹ is selected from the group consisting of hydrido, -OR³, C₁₋₆ alkyl, C₅₋₇ cycloalkyl, benzyl, -CH₂(C₃₋₇ cycloalkyl), aryl, halo, heterocycloalkyl, heterocycloalkenyl, and heteroaryl;
wherein R³ is selected from the group consisting of hydrido, C₁₋₆ alkyl, C₅₋₇ cycloalkyl, benzyl, -CH₂(C₃₋₇ cycloalkyl), and aryl;
or a pharmaceutically acceptable salt thereof.

5. A compound according to claim 4:
wherein R¹ is selected from the group consisting of hydrido, -OR³, C₁₋₆ alkyl, C₅₋₇ cycloalkyl, benzyl, -CH₂(C₃₋₇ cycloalkyl), C₃₋₁₂ aryl, halo, 3- to 12-membered heterocycloalkyl, 3- to 12-membered heterocycloalkenyl, and 3- to 12-membered heteroaryl; and
wherein R³ is selected from the group consisting of hydrido, C₁₋₆ alkyl, C₅₋₇ cycloalkyl, benzyl, -CH₂(C₃₋₇ cycloalkyl), and C₃₋₁₂ aryl;
or a pharmaceutically acceptable salt thereof.

6. A compound according to claim 5:
wherein R¹ is selected from the group consisting of hydrido, -OR³, methyl, ethyl, propyl, butyl, pentyl, hexyl, cyclopentyl, cyclohexyl, cycloheptyl, benzyl, methylcyclopropyl, methylcyclobutyl, methylcyclopentyl, methylcyclohexyl, phenyl, biphenyl, naphthyl, indenyl, chloro, fluoro, bromo, iodo, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl; and
wherein R³ is selected from the group consisting of hydrido, methyl, ethyl, propyl, butyl, pentyl, hexyl, cyclopentyl, cyclohexyl, cycloheptyl, benzyl, methylcyclopropyl, methylcyclobutyl, methylcyclopentyl, methylcyclohexyl, phenyl, biphenyl, naphthyl, and indenyl;
or a pharmaceutically acceptable salt thereof.

7. A compound according to claim 1 wherein the compound of Formula I is a compound of Formula III:
wherein s is 1, 2, or 3; 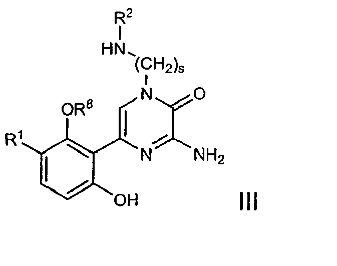 wherein R¹ is selected from the group consisting of hydrido, -OR³, C₁₋₆ alkyl, C₅₋₇ cycloalkyl, benzyl, -CH₂(C₃₋₇ cycloalkyl), aryl, halo, heterocycloalkyl, heterocycloalkenyl, and heteroaryl;
wherein R² is hydrido or C₁₋₆ alkyl;
wherein R³ is selected from the group consisting of C₁₋₆ alkyl, C₅₋₇ cycloalkyl, benzyl, -CH₂(C₃₋₇ cycloalkyl), and aryl; and
wherein R⁸ is selected from the group consisting of C₁₋₆ alkyl, C₅₋₇ cycloalkyl, benzyl, and -CH₂(C₃₋₇ cycloalkyl);
or a pharmaceutically acceptable salt thereof.

8. A compound according to claim 7:
wherein R¹ is selected from the group consisting of hydrido, -OR³, C₁₋₆ alkyl, C₅₋₇ cycloalkyl, benzyl, -CH₂(C₃₋₇ cycloalkyl), C₃₋₁₂ aryl, halo, 3- to 12-membered heterocycloalkyl, 3- to 12-membered heterocycloalkenyl, and 3- to 12-membered heteroaryl;
wherein R² is hydrido or C₁₋₆ alkyl;
wherein R³ is selected from the group consisting of C₁₋₆ alkyl, C₅₋₇ cycloalkyl, benzyl, -CH₂(C₃₋₇ cycloalkyl), and C₃₋₁₂ aryl; and
wherein R⁸ is selected from the group consisting of C₁₋₆ alkyl, C₅₋₇ cycloalkyl, benzyl, and -CH₂(C₃₋₇ cycloalkyl);
or a pharmaceutically acceptable salt thereof.

9. A compound according to claim 8:
wherein R¹ is selected from the group consisting of hydrido, -OR³, methyl, ethyl, propyl, butyl, pentyl, hexyl, cyclopentyl, cyclohexyl, cycloheptyl, benzyl, methylcyclopropyl, methylcyclobutyl, methylcyclopentyl, methylcyclohexyl, phenyl, biphenyl, naphthyl, indenyl, chloro, fluoro, bromo, iodo, piperidinyl, pyrrolidinyl, pyrazolidinyl, imidazolidinyl, isoxazolidinyl, oxazolidinyl, isoindolyl, dihydroindolyl, isoindoline, dihydrothiophenyl, dihydropyrrolyl, dihydrofuryl, dihydropyrazolyl, dihydroimidazolyl, dihydroisoxazolyl, dihydrooxazolyl, pyridinyl, benzothiophenyl, indolyl, isoquinolinyl, quinolinyl, thienyl, pyrrolyl, furyl, pyrazolyl, imidazolyl, isoxazolyl, oxazolyl, and isoindoledionyl;
wherein R² is selected from the group consisting of hydrido, methyl, ethyl, propyl, butyl, pentyl, and hexyl;
wherein R³ is selected from the group consisting of methyl, ethyl, propyl, butyl, pentyl, hexyl, cyclopentyl, cyclohexyl, cycloheptyl, benzyl, methylcyclopropyl, methylcyclobutyl, methylcyclopentyl, methylcyclohexyl, phenyl, biphenyl, naphthyl, and indenyl; and
wherein R⁸ is selected from the group consisting of methyl, ethyl, propyl, butyl, pentyl, hexyl, cyclopentyl, cyclohexyl, cycloheptyl, benzyl, methylcyclopropyl, methylcyclobutyl, methylcyclopentyl, and methylcyclohexyl;
or a pharmaceutically acceptable salt thereof.

10. A compound according to claim 1 wherein the compound of Formula I is a compound of Formula IV: 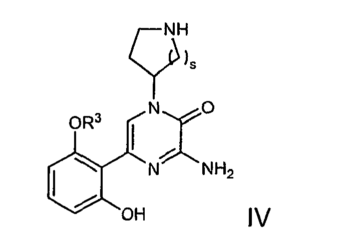 wherein n is 1, 2, or 3; and
wherein R³ is selected from the group consisting of hydrido, C₁₋₆ alkyl, C₅₋₇ cycloalkyl, benzyl, and -CH₂(C₃₋₇ cycloalkyl);
or a pharmaceutically acceptable salt thereof.

11. A compound according to claim 10.
wherein wherein R³ is selected from the group consisting of hydrido, methyl, ethyl, propyl, butyl, pentyl, hexyl, cyclopentyl, cyclohexyl, cycloheptyl, benzyl, methylcyclopropyl, methylcyclobutyl, methylcyclopentyl, and methylcyclohexyl;
or a pharmaceutically acceptable salt thereof.

12. A compound according to claim 1 selected from the group of compounds consisting of:
3-amino-5-(2-hydroxyphenyl)-1-piperidin-3-ylpyrazin-2(1H)-one;
3-amino-5-(5-fluoro-2-hydroxyphenyl)-1-piperidin-3-ylpyrazin-2(1H)-one;
3-amino-5-(4-fluoro-2-hydroxyphenyl)-1-piperidin-3-ylpyrazin-2(1H)-one;
3-amino-5-(3-fluoro-2-hydroxyphenyl)-1-piperidin-3-ylpyrazin-2(1H)-one;
3-amino-5-(2-fluoro-6-hydroxyphenyl)-1-piperidin-3-ylpyrazin-2(1H)-one;
3-amino-5-(2-bromo-6-hydroxyphenyl)-1-piperidin-3-ylpyrazin-2(1H)-one;
3-amino-5-(5-bromo-2-hydroxyphenyl)-1-piperidin-3-ylpyrazin-2(1H)-one;
3-amino-5-(4-bromo-2-hydroxyphenyl)-1-piperidin-3-ylpyrazin-2(1H)-one;
3-amino-5-(3-bromo-2-hydroxyphenyl)-1-piperidin-3-ylpyrazin-2(1H)-one;
3-amino-5-(2-chloro-6-hydroxyphenyl)-1-piperidin-3-ylpyrazin-2(1H)-one;
3-amino-5-(5-chloro-2-hydroxyphenyl)-1-piperidin-3-ylpyrazin-2(1H)-one;
3-amino-5-(4-chloro-2-hydroxyphenyl)-1-piperidin-3-ylpyrazin-2(1H)-one;
3-amino-5-(3-chloro-2-hydroxyphenyl)-1-piperidin-3-ylpyrazin-2(1H)-one;
3-amino-5-(2-hydroxy-6-methylphenyl)-1-piperidin-3-ylpyrazin-2(1H)-one;
3-amino-5-(2-hydroxy-5-methylphenyl)-1-piperidin-3-ylpyrazin-2(1H)-one;
3-amino-5-(2-hydroxy-4-methylphenyl)-1-piperidin-3-ylpyrazin-2(1H)-one;
3-amino-5-(2-hydroxy-3-methylphenyl)-1-piperidin-3-ylpyrazin-2(1H)-one;
3-amino-5-(5-cyclopentyl-2-hydroxyphenyl)-1-piperidin-3-ylpyrazin-2(1H)-one;
3-amino-5-(5-cyclohexyl-2-hydroxyphenyl)-1-piperidin-3-ylpyrazin-2(1H)-one;
3-amino-5-(5-cycloheptyl-2-hydroxyphenyl)-1-piperidin-3-ylpyrazin-2(1H)-one;
3-amino-5-(5-cyclopropylmethyl-2-hydroxyphenyl)-1-piperidin-3-ylpyrazin-2(1H)-one;
3-amino-5-(5-cyclobutylmethyl-2-hydroxyphenyl)-1-piperidin-3-ylpyrazin-2(1H)-one;
3-amino-5-(5-cyclopentylmethyl-2-hydroxyphenyl)-1-piperidin-3-ylpyrazin-2(1H)-one;
3-amino-5-(5-cyclohexylmethyl-2-hydroxyphenyl)-1-piperidin-3-ylpyrazin-2(1H)-one;
3-amino-5-(5-phenyl-2-hydroxyphenyl)-1-piperidin-3-ylpyrazin-2(1H)-one;
3-amino-5-[2-hydroxy-5-(1-naphthyl)phenyl]-1-piperidin-3-ylpyrazin-2(1H)-one;
3-amino-5-[2-hydroxy-5-(2-naphthyl)phenyl]-1-piperidin-3-ylpyrazin-2(1H)-one;
3-amino-5-(2-hydroxy-5-pyrrolidin-2-ylphenyl)-1-piperidin-3-ylpyrazin-2(1H)-one;
3-amino-5-(2-hydroxy-5-pyrrolidin-3-ylphenyl)-1-piperidin-3-ylpyrazin-2(1H)-one;
3-amino-5-(2-hydroxy-5-pyrrolidin-1-ylphenyl)-1-piperidin-3-ylpyrazin-2(1H)-one;
3-amino-5-(2-hydroxy-5-pyrazolidin-1-ylphenyl)-1-piperidin-3-ylpyrazin-2(1H)-one;
3-amino-5-(2-hydroxy-5-imidazolidin-1-ylphenyl)-1-piperidin-3-ylpyrazin-2(1H)-one;
3-amino-5-(2-hydroxy-5-pyrazolidin-3-ylphenyl)-1-piperidin-3-ylpyrazin-2(1H)-one;
3-amino-5-(2-hydroxy-5-pyrazolidin-4-ylphenyl)-1-piperidin-3-ylpyrazin-2(1H)-one;
3-amino-5-(2-hydroxy-5-imidazolidin-1-ylphenyl)-1-piperidin-3-ylpyrazin-2(1H)-one;
3-amino-5-(2-hydroxy-5-imidazolidin-4-ylphenyl)-1-piperidin-3-ylpyrazin-2(1H)-one;
3-amino-5-(2-hydroxy-5-tetrahydrofuran-2-ylphenyl)-1-piperidin-3-ylpyrazin-2(1H)-one;
3-amino-5-(2-hydroxy-5-tetrahydrofuran-3-ylphenyl)-1-piperidin-3-ylpyrazin-2(1H)-one;
3-amino-5-(2-hydroxy-5-tetrahydrothien-2-ylphenyl)-1-piperidin-3-ylpyrazin-2(1H)-one;
3-amino-5-(2-hydroxy-5-tetrahydrothien-3-ylphenyl)-1-piperidin-3-ylpyrazin-2(1H)-one;
3-amino-5-(2-hydroxy-5-isoxazolidin-2-ylphenyl)-1-piperidin-3-ylpyrazin-2(1H)-one;
3-amino-5-[2-hydroxy-5-(1,3-oxazolidin-3-yl)phenyl]-1-piperidin-3-ylpyrazin-2(1H)-one;
3-amino-5-(2-hydroxy-5-isoxazolidin-3-ylphenyl)-1-piperidin-3-ylpyrazin-2(1H)-one;
3-amino-5-[2-hydroxy-5-(1,3-oxazolidin-4-yl)phenyl]-1-piperidin-3-ylpyrazin-2(1H)-one;
3-amino-5-[2-hydroxy-5-(1,3-oxazolidin-2-yl)phenyl]-1-piperidin-3-ylpyrazin-2(1H)-one;
3-amino-5-(2-hydroxy-5-isoxazolidin-4-ylphenyl)-1-piperidin-3-ylpyrazin-2(1H)-one;
3-amino-5-[2-hydroxy-5-(1,3-oxazolidin-5-yl)phenyl]-1-piperidin-3-ylpyrazin-2(1H)-one;
3-amino-5-(2-hydroxy-5-isoxazolidin-5-ylphenyl)-1-piperidin-3-ylpyrazin-2(1H)-one;
3-amino-5-[2-hydroxy-5-(1H-pyrrol-1-yl)phenyl]-1-piperidin-3-ylpyrazin-2(1H)-one;
3-amino-5-[2-hydroxy-5-(1H-pyrrol-2-yl)phenyl]-1-piperidin-3-ylpyrazin-2(1H)-one;
3-amino-5-[2-hydroxy-5-(1 H-pyrrol-3-yl)phenyl]-l -piperidin-3-ylpyrazin-2(1H)-one;
3-amino-5-[2-hydroxy-5-(1H-pyrazol-1-yl)phenyl]-1-piperidin-3-ylpyrazin-2(1H)-one;
3-amino-5-[2-hydroxy-5-(1H-imidazol-1-yl)phenyl]-1-piperidin-3-ylpyrazin-2(1H)-one;
3-amino-5-[2-hydroxy-5-(1H-pyrazol-3-yl)phenyl]-1-piperidin-3-ylpyrazin-2(1H)-one;
3-amino-5-[2-hydroxy-5-(1H-imidazol-4-yl)phenyl]-1-piperidin-3-ylpyrazin-2(1H)-one;
3-amino-5-[2-hydroxy-5-(1H-imidazol-2-yl)phenyl]-1-piperidin-3-ylpyrazin-2(1H)-one;
3-amino-5-[5-(2-furyl)-2-hydroxyphenyl]-1-piperidin-3-ylpyrazin-2(1H)-one;
3-amino-5-[5-(3-furyl)-2-hydroxyphenyl]-1-piperidin-3-ylpyrazin-2(1H)-one;
3-amino-5-(2-hydroxy-5-thien-2-ylphenyl)-1-piperidin-3-ylpyrazin-2(1H)-one;
3-amino-5-(2-hydroxy-5-thien-3-ylphenyl)-1-piperidin-3-ylpyrazin-2(1H)-one;
3-amino-5-(2-hydroxy-5-isoxazolidin-2-ylphenyl)-1-piperidin-3-ylpyrazin-2(1H)-one;
3-amino-5-(2-hydroxy-5-isoxazol-3-ylphenyl)-1-piperidin-3-ylpyrazin-2(1H)-one;
3-amino-5-[2-hydroxy-5-(1,3-oxazol-4-yl)phenyl]-1-piperidin-3-ylpyrazin-2(1H)-one;
3-amino-5-[2-hydroxy-5-(1,3-oxazol-2-yl)phenyl]-1-piperidin-3-ylpyrazin-2(1H)-one;
3-amino-5-(2-hydroxy-5-isoxazol-4-ylphenyl)-1-piperidin-3-ylpyrazin-2(1H)-one;
3-amino-5-[2-hydroxy-5-(1,3-oxazol-5-yl)phenyl]-1-piperidin-3-ylpyrazin-2(1H)-one;
3-amino-5-(2-hydroxy-5-isoxazol-5-ylphenyl)-1-piperidin-3-ylpyrazin-2(1H)-one;
3-amino-5-(2-hydroxy-5-piperidin-1-ylphenyl)-1-piperidin-3-ylpyrazin-2(1H)-one;
3-amino-5-(2-hydroxy-5-piperidin-2-ylphenyl)-1-piperidin-3-ylpyrazin-2(1H)-one;
3-amino-5-(2-hydroxy-5-piperidin-3-ylphenyl)-1-piperidin-3-ylpyrazin-2(1H)-one;
3-amino-5-(2-hydroxy-5-piperidin-4-ylphenyl)-piperidin-3-ylpyrazin-2(1H)-one;
3-amino-5-(2-hydroxy-5-piperazin-1-ylphenyl)-1-piperid in-3-ylpyrazin-2(1H)-one;
3-amino-5-(2-hydroxy-5-piperazin-2-ylphenyl)-1-piperidin-3-ylpyrazin-2(1H)-one;
3-amino-5-(2-hydroxy-5-pyridin-2-ylphenyl)-1-piperidin-3-ylpyrazin-2(1H)-one;
3-amino-5-(2-hydroxy-5-pyridin-3-ylphenyl)-1-piperidin-3-ylpyrazin-2(1H)-one;
3-amino-5-(2-hydroxy-5-pyridin-4-ylphenyl)-1-piperidin-3-ylpyrazin-2(1H)-one;
3-amino-5-(2-hydroxy-5-pyridazin-3-ylphenyl)-1-piperidin-3-ylpyrazin-2(1H)-one;
3-amino-5-(2-hydroxy-5-pyridazin-4-ylphenyl)-1-piperidin-3-ylpyrazin-2(1H)-one;
3-amino-5-(2-hydroxy-5-pyrazin-2-ylphenyl)-1-piperidin-3-ylpyrazin-2(1H)-one;
3-amino-5-(5-benzyl-2-hydroxyphenyl)-1-piperidin-3-ylpyrazin-2(1H)-one;
3-amino-5-(2,5-dihydroxyphenyl)-1-piperidin-3-ylpyrazin-2(1H)-one;
3-amino-5-(2-hydroxy-5-methoxyphenyl)-1-piperidin-3-ylpyrazin-2(1H)-one;
3-amino-5-(5-ethoxy-2-hydroxyphenyl)-1-piperidin-3-ylpyrazin-2(1H)-one;
3-amino-5-(2-hydroxy-5-propoxyphenyl)-1-piperidin-3-ylpyrazin-2(1H)-one;
3-amino-5-(2-hydroxy-5-isopropoxyphenyl)-1-piperidin-3-ylpyrazin-2(1H)-one;
3-amino-5-(2-hydroxy-5-t-butoxyphenyl)-1-piperidin-3-ylpyrazin-2(1H)-one;
3-amino-5-(2-hydroxy-5-phenoxyphenyl)-1-piperidin-3-ylpyrazin-2(1H)-one;
3-amino-5-[2-hydroxy-5-(1-naphthyloxy)phenyl]-1-piperidin-3-ylpyrazin-2(1H)-one;
3-amino-5-[2-hydroxy-5-(2-naphthyloxy)phenyl]-1-piperidin-3-ylpyrazin-2(1H)-one;
3-amino-5-[5-(benzyloxy)-2-hydroxyphenyl]-1-piperidin-3-ylpyrazin-2(1H)-one;
3-amino-5-[5-(cyclopropylmethoxy)-2-hydroxyphenyl]-1-piperidin-3-ylpyrazin-2(1H)-one;
3-amino-5-[5-(cyclopentylmethoxy)-2-hydroxyphenyl]-1-piperidin-3-ylpyrazin-2(1H)-one;
3-amino-5-[5-(cyclohexylmethoxy)-2-hydroxyphenyl]-1-piperidin-3-ylpyrazin-2(1H)-one;
3-amino-5-[5-(cyclohexyloxy)-2-hydroxyphenyl]-1-piperidin-3-ylpyrazin-2(1H)-one; and
3-amino-5-[5-(cyclopentyloxy)-2-hydroxyphenyl]-1-piperidin-3-ylpyrazin-2(1H)-one.

13. A pharmaceutical composition comprising a compound according to any one of claims 1-12 or a pharmaceutically-acceptable salt thereof, and a pharmaceutically acceptable carrier, diluent, or adjuvant.

14. Use of a compound according to any one of claims 1-2 for the preparation of a medicament for the treatment of cancer, inflammation, or an inflammation-associated disorder in a subject.

15. A use according to claim 14 wherein medicament is for the treatment of arthritis, cancer, asthma, COPD, frailty, diabetes, atherosclerosis, pain, and/or dermatological disease.

## Patentansprüche

1. Verbindung der Formel I: 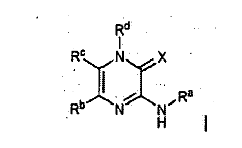 worin X ausgewählt ist aus der Gruppe, bestehend aus O, S, und NR^{5a};
worin R^{a} und R^{c} unabhängig ausgewählt sind aus der Gruppe, bestehend aus Hydrido, Hydroxyl, Alkoxy, Alkyl, Halogenalkyl, Aryl und Heteroaryl;
worin R^{b} eine 3- bis 12-gliedrige cyclische Gruppierung ist, ausgewählt aus der Gruppe, bestehend aus Cycloalkyl, Cycloalkenyl, Aryl, Heterocycloalkyl, Heterocycloalkenyl und Heteroaryl, wobei R^{b} gegebenenfalls mit einem oder mehreren Substituenten, ausgewählt aus der Gruppe, bestehend aus R², Cycloalkyl und Cycloalkylalkyl, substituiert ist;
worin R^{d} ausgewählt ist aus der Gruppe, bestehend aus -(CH₂)_{q}NH₂, -(CH₂)_{q}NHR² und einem 5- bis 7-gliedrigen Heterocycloalkyl mit Ringgliedern, ausgewählt aus der Gruppe, bestehend aus Kohlenstoff und Stickstoff, wobei das Heterocycloalkyl gegebenenfalls mit einem oder mehreren Substituenten, ausgewählt aus der Gruppe, bestehend aus R², substituiert sein kann;
worin R² ausgewählt ist aus der Gruppe, bestehend aus Halogen, Alkylsulfinyl, Alkylsulfonyl, Cyano, Alkoxycarbonyl, Alkyl, Halogenalkyl, Hydroxyalkyl, Halogenalkoxy, Nitro, Acylamino, R⁷, - OR³ , (CH₂)ₘOR³, -(CH₂)ₚCO₂R³, -SR³, -SO₂N(R^{4a})R^{4b}, -NR^{5a}R^{5b}, -NR^{5a}COR^{5b}, -NR^{5a}CO (OR^{5b}), -NR^{5a}SO₂R⁶, -NR^{5a}SO₂N(R^{6a}) R^{6b}, -NR^{5a}CON (R^{6a}) R^{6b}, -COR^{5a} und -CON(R^{4a}) R^{4b};
worin R³, R^{4a} und R^{4b} unabhängig ausgewählt sind aus der Gruppe, bestehend aus Hydrido, Aryl, Heteroaryl, Heteroaralkyl, Alkyl, Halogenalkyl, Alkenyl, Alkinyl, Hydroxyalkyl, Aminoalkyl, Alkylaminoalkyl, N,N-Dialkylaminoalkyl, Alkoxy, Alkoxyalkyl, Heterocycloalkyl, Heterocycloalkenyl, Cycloalkyl, Cycloalkylalkyl, Aralkyl und Aralkylamino, wobei das Aryl gegebenenfalls mit einem oder mehreren Resten, ausgewählt aus der Gruppe, bestehend aus Alkyl, Aminoalkyl, Alkoxy und Halogen substituiert ist, wobei R^{4a} und R^{4b} unter Bildung eines 3- bis 7-gliedrigen heterocyclischen Rings mit 1 bis 3 Heteroatomen, ausgewählt aus S, SO, SO₂, O, N und NR^{5a}, zusammengenommen werden können;
worin R^{5a} und R^{5b} unabhängig ausgewählt sind aus der Gruppe, bestehend aus Hydrido, Alkyl, Aryl, Heteroaryl, Aralkyl, Heterocycloalkenyl, Cycloalkyl, Heterocycloalkyl, Halogenalkyl, Aralkylamino, Amino, Aminoalkyl, Aminoacyl, Nitro, Azido und Heteroaralkyl, wobei die Alkyl-, Aryl-, Heteroaryl-, Aminoalkyl- und Aralkylgruppierungen gegebenenfalls mit einem oder mehreren Substituenten, ausgewählt aus der Gruppe, bestehend aus Alkylsulfonamido, Sulfamyl, Alkyl, Alkylthio, Alkylsulfinyl, Alkylsulfonyl, N-Alkylamino, Aminoalkyl, Alkylaminoalkyl, Alkoxy, Halogen, Acyloxy, Oxy, Formyl, Halogenalkyl, Cyano, Halogenalkoxy, Acyl, Carboxyl, Hydroxy, Hydroxyalkoxy; Phenoxy, Nitro, Azido, Benzyloxy, N,N-Dialkylaminoacyl, Thioalkyl, Aminoacyloxy, Thiocyanato, Isothiocyanato, Alkyldioxy, Hydroxyalkyl, N-Alkylamino, Alkoxycarbonyl, Alkoxyalkyl, Alkenylamino, Alkinylamino, Alkenyl, Alkinyl, N,N-Dialkylaminoalkoxy, Heterocycloalkyl, Heterocycloalkenyl und Heteroaryl, substituiert sind;
worin R^{6a} und R^{6b} unabhängig ausgewählt sind aus der Gruppe, bestehend aus Hydrido, Alkyl, Heteroaryl, Heterocycloalkenyl, Halogenalkyl, Aralkylamino, Heteroaralkyl, Aryl und Aralkyl, wobei die Aryl-, Heteroaryl-, Heterocycloalkenyl- und Aralkylgruppierungen gegebenenfalls mit einem oder mehreren Substituenten, ausgewählt aus Alkyl, Alkoxy, Halogen, Halogenalkyl, Cyano, Halogenalkoxy, Acyl, Carboxyl, Hydroxy, Hydroxyalkoxy, Phenoxy, Benzyloxy, N,N-Dialkylaminoalkoxy, Heterocycloalkyl, Heterocycloalkenyl und Heteroaryl, substituiert sind, wobei R^{6a} und R^{6b} unter Bildung eines 3- bis 7-gliedrigen heterocyclischen Rings mit 1 bis 3 Heteroatomen, ausgewählt aus S, SO, SO₂, O, N und NR^{5a}, zusammengenommen werden können;
worin R⁷ ausgewählt ist aus der Gruppe, bestehend aus Aryl, Heterocycloalkenyl, Heteroaryl und Alkenyl, wobei R⁷ gegebenenfalls mit einem oder mehreren Substituenten, ausgewählt aus der Gruppe, bestehend aus R^{5a}, substituiert ist;
worin n 1, 2 oder 3 ist;
worin m 1, 2 oder 3 ist;
worin p 0, 1 oder 2 ist;
worin q eine ganze Zahl zwischen 0 und 9 ist;
oder ein pharmazeutisch annehmbares Salz davon.

2. Verbindung gemäß Anspruch 1:
worin R^{a} und R^{c} unabhängig ausgewählt sind aus der Gruppe, bestehend aus Hydrido, Hydroxyl, C₁₋₆-Alkoxy, C₁₋₆-Alkyl, C₁₋₆-Halogenalkyl, C₃₋₁₂-Aryl und 3- bis 12-gliedrigem Heteroaryl;
worin R^{b} eine 3- bis 12-gliedrige cyclische Gruppierung ist, ausgewählt aus der Gruppe, bestehend aus C₃₋₁₂-Cycloalkyl, C₃₋₁₂-Cycloalkenyl, C₃₋₁₂-Aryl, 3- bis 12-gliedrigem Heterocycloalkyl, 3- bis 12-gliedrigem Heterocycloalkenyl und 3- bis 12-gliedrigem Heteroaryl, wobei R^{b} gegebenenfalls mit einem oder mehreren Substituenten, ausgewählt aus der Gruppe, bestehend aus R², C₃₋₁₂-Cycloalkyl und C₄₋₁₈-Cycloalkylalkyl, substituiert ist;
worin R^{d} ausgewählt ist aus der Gruppe, bestehend aus -(CH₂)_{q}NH₂,- (CH₂)_{q}NHR² und einem 5- bis 7-gliedrigen Heterocycloalkyl mit Ringgliedern, ausgewählt aus der Gruppe, bestehend aus Kohlenstoff und Stickstoff, wobei das Heterocycloalkyl gegebenenfalls mit einem oder mehreren Substituenten, ausgewählt aus der Gruppe, bestehend aus R², substituiert sein kann;
worin R² ausgewählt ist aus der Gruppe, bestehend aus Halogen, C₁₋₆-Alkylsulfinyl, C₁₋₆-Alkylsulfonyl, Cyano, C₂₋₇-Alkoxycarbonyl, C₁₋₆-Alkyl, C₁₋₆-Halogenalkyl, C₁₋₆-Hydroxyalkyl, C₁₋₆-Halogenalkoxy, Nitro, C₂₋₁₀-Acylamino, R⁷, -OR³, - (CH₂)ₘOR³, -(CH₂)ₚCO₂R³, -SR³, -SO₂N(R^{4a})R^{4b}, NR^{5a}R^{5b}, -NR^{5a}COR^{5b}, -NR^{5a}CO(OR^{5b}), -NR^{5a}SO₂R⁶, -NR^{5a}SO₂N (R^{6a})R^{6b}, -NR^{5a}CON(R^{6a})R^{6b}, -COR^{5a} und -CON(R^{4a}) R^{4b};
worin R³, R^{4a} und R^{4b} unabhängig ausgewählt sind aus der Gruppe, bestehend aus Hydrido, C₃₋₁₂-Aryl, 3- bis 12-gliedrigem Heteroaryl, 4- bis 18-gliedrigem Heteroaralkyl, C₁₋₆-Alkyl, C₁₋₆-Halogenalkyl, C₂₋₆-Alkenyl, C₂₋₆-Alkinyl, C₁₋₆-Hydroxyalkyl, C₁₋₆-Aminoalkyl, C₂₋₁₂-Alkylaminoalkyl, N,N-Di (C₁₋₆-alkyl)amino(C₁₋₆-alkyl), C₁₋₆-Alkoxy, C₂₋₁₂-Alkoxyalkyl, 3- bis 12-gliedrigem Heterocycloalkyl, 3- bis 12-gliedrigem Heterocycloalkenyl, C₃₋₁₂-Cycloalkyl, C₄₋₁₈-Cycloalkylalkyl, C₄₋₁₈-Aralkyl und C₄₋₁₈-Aralkylamino, wobei das Aryl gegebenenfalls mit einem oder mehreren Resten, ausgewählt aus der Gruppe, bestehend aus C₁₋₆-Alkyl, C₁₋₆-Aminoalkyl, C₁₋₆-Alkoxy und Halogen, substituiert ist, wobei R^{4a} und R^{4b} unter Bildung eines 3- bis 7-gliedrigen heterocyclischen Rings mit 1 bis 3 Heteroatomen, ausgewählt aus S, SO, SO₂, O, N und NR^{5a}, zusammengenommen werden können;
worin R^{5a} und R^{5b} unabhängig ausgewählt sind aus der Gruppe, bestehend aus Hydrido, C₁₋₆-Alkyl, C₃₋₁₂-Aryl, 3- bis 12-gliedrigem Heteroaryl, C₄₋₁₈-Aralkyl, 3- bis 12-gliedrigem Heterocycloalkenyl, C₃₋₁₂-Cycloalkyl, 3- bis 12-gliedrigem Heterocycloalkyl, C₁₋₆-Halogenalkyl, C₄₋₁₈-Aralkylamino, Amino, C₁₋₆-Aminoalkyl, C₂-₁₀-Aminoacyl, Nitro, Azido und 4- bis 18-gliedrigem Heteroaralkyl, wobei die Alkyl-, Aryl-, Heteroaryl-, Aminoalkyl- und Aralkylgruppierungen gegebenenfalls mit einem oder mehreren Substituenten, ausgewählt aus der Gruppe, bestehend aus C₁₋₆-Alkylsulfonamido, Sulfamyl, C₁₋₆-Alkyl, C₁₋₆-Alkylthio, C₁₋₆-Alkylsulfinyl, C₁₋₆-Alkylsulfonyl, N-(C₁-₆-Alkyl)amino, C₁₋₆-Aminoalkyl, C₂₋₁₂-Alkylaminoalkyl, C₁₋₆-Alkoxy, Halogen, C₂₋₁₀-Acyloxy, Oxy, Formyl, C₁₋₆-Halogenalkyl, Cyano, C₁₋₆-Halogenalkoxy, C₂₋₁₀-Acyl, Carboxyl, Hydroxy, C₁₋₆-Hydroxyalkoxy, Phenoxy, Nitro, Azido, Benzyloxy, N,N-Di(C₁₋₆-alkyl)amino(C₂₋₁₀-acyl), C₁₋₆-Thioalkyl, C₂₋₁₀-Aminoacyloxy, Thiocyanato, Isothiocyanato, C₁₋₆-Alkyldioxy, C₁₋₆-Hydroxyalkyl, N-(C₁₋₆-Alkyl)amino, C₂₋₇-Alkoxycarbonyl, C₂₋₁₂-Alkoxyalkyl, C₂₋₆-Alkenylamino, C₂₋₆-Alkinylamino, C₂₋₆-Alkenyl, C₂₋₆-Alkinyl, N,N-Di(C₁₋₆-alkyl)amino(C₁₋₆-alkoxy), 3- bis 12-gliedrigem Heterocycloalkenyl, 3- bis 12-gliedrigem Heterocycloalkyl und 3- bis 12-gliedrigem Heteroaryl, substituiert sind;
worin R^{6a} und R^{6b} unabhängig ausgewählt sind aus der Gruppe, bestehend aus Hydrido, C₁₋₆-Alkyl, 3- bis 12-gliedrigem Heteroaryl, 3- bis 12-gliedrigem Heterocycloalkenyl, C₁₋₆-Halogenalkyl, C₄₋₁₈-Aralkylamino, 4- bis 18-gliedrigem Heteroaralkyl, C₃₋₁₂-Aryl und C₄₋₁₈-Aralkyl, wobei die Aryl-, Heteroaryl-, Heterocycloalkenyl- und Aralkylgruppierungen gegebenenfalls mit einem oder mehreren Substituenten, ausgewählt aus C₁₋₆-Alkyl, C₁₋₆-Alkoxy, Halogen, C₁₋₆-Halogenalkyl, Cyano, C₁₋₆-Halogenalkoxy, C₂₋₁₀-Acyl, Carboxyl, Hydroxy, C₁₋₆-Hydroxyalkoxy, Phenoxy, Benzyloxy, N,N-Di(C₁₋₆-alkyl)amino(C₁₋₆-alkoxy), 3- bis 12-gliedrigem Heterocycloalkenyl, 3- bis 12-gliedrigem Heterocycloalkyl und 3- bis 12-gliedrigem Heteroaryl, substituiert sind, wobei R^{6a} und R^{6b} unter Bildung eines 3- bis 7-gliedrigen heterocyclischen Rings mit 1 bis 3 Heteroatomen, ausgewählt aus S, SO, SO₂, O, N und NR^{5a}, zusammengenommen werden können; und
worin R⁷ ausgewählt ist aus der Gruppe, bestehend aus C₃₋₁₂-Aryl, 3- bis 12-gliedrigem Heterocycloalkenyl, 3- bis 12-gliedrigem Heteroaryl und C₂₋₆-Alkenyl, wobei R⁷ gegebenenfalls mit einem oder mehreren Substituenten, ausgewählt aus der Gruppe, bestehend aus R^{5a}, substituiert ist;
oder ein pharmazeutisch annehmbares Salz davon.

3. Verbindung gemäß Anspruch 1 oder 2:
worin R^{a} und R^{c} unabhängig ausgewählt sind aus der Gruppe, bestehend aus Hydrido, Hydroxyl, Methoxy, Ethoxy, Propoxy, Butoxy, Methyl, Ethyl, Propyl, Butyl, Pentyl, Hexyl, Chlormethyl, Dichlormethyl, Trichlormethyl, Fluormethyl, Difluormethyl, Trifluormethyl, Phenyl, Biphenyl, Naphthyl, Indenyl, Pyridinyl, Benzothiophenyl, Indolyl, Isochinolyl, Chinolyl, Thienyl, Pyrrolyl, Furyl, Pyrazolyl, Imidazolyl, Isoxazolyl, Oxazolyl und Isoindoldionyl;
worin R^{b} eine 3- bis 12-gliedrige cyclische Gruppierung ist, ausgewählt aus der Gruppe, bestehend aus Cyclopropyl, Cyclobutyl, Cyclopentyl, Cyclohexyl, Cyclopropenyl, Cyclobutenyl, Cyclopentenyl, Cyclohexenyl, Phenyl, Biphenyl, Naphthyl, Indenyl, Piperidinyl, Pyrrolidinyl, Pyrazolidinyl, Imidazolidinyl, Isoxazolidinyl, Oxazolidinyl, Isoindolyl, Dihydroindolyl, Isoindolin, Dihydrothiophenyl, Dihydropyrrolyl, Dihydrofuryl, Dihydropyrazolyl, Dihydroimidazolyl, Dihydroisoxazolyl, Dihydrooxazolyl, Pyridinyl, Benzothiophenyl, Indolyl, Isochinolinyl, Chinolinyl, Thienyl, Pyrrolyl, Furyl, Pyrazolyl, Imidazolyl, Isoxazolyl, Oxazolyl und Isoindoldionyl, wobei R^{b} gegebenenfalls mit einem oder mehreren Substituenten, ausgewählt aus der Gruppe, bestehend aus R², Cyclopropyl, Cyclobutyl, Cyclopentyl, Cyclohexyl, Cyclopropylmethyl, Cyclopropylethyl, Cyclobutylmethyl, Cyclobutylethyl, Cyclopentylmethyl, Cyclopentylethyl, Cyclohexylmethyl und Cyclohexylethyl, substituiert ist;
worin R^{d}, ausgewählt ist aus der Gruppe, bestehend aus - (CH₂)_{q}NH₂, - (CH₂)_{q}NHR², Piperidinyl, Pyrrolidinyl, Pyrazolidinyl und Imidazolidinyl, wobei das Piperidinyl, Pyrrolidinyl, Pyrazolidinyl oder Imidazolidinyl gegebenenfalls mit einem oder mehreren Substituenten, ausgewählt aus der Gruppe, bestehend aus R², substituiert sein kann;
worin R² ausgewählt ist aus der Gruppe, bestehend aus Chlor, Fluor, Brom, Iod, Methylsulfinyl, Ethylsulfinyl, Propylsulfinyl, Butylsulfinyl, Methylsulfonyl, Ethylsulfonyl, Propylsulfonyl, Butylsulfonyl, Cyano, Methoxycarbonyl, Ethoxycarbonyl, Propoxycarbonyl, Butoxycarbonyl, Methyl, Ethyl, Propyl, Butyl, Pentyl, Hexyl, Chlormethyl, Dichlormethyl, Trichlormethyl, Fluormethyl, Difluormethyl, Trifluormethyl, Hydroxymethyl, Hydroxyethyl, Hydroxypropyl, Hydroxybutyl, Hydroxypentyl, Hydroxyhexyl, Chlormethoxy, Dichlormethoxy, Trichlormethoxy, Fluormethoxy, Difluormethoxy, Trifluormethoxy, Nitro, Methylcarbonylamino, Ethylcarbonylamino, Propylcarbonylamino, Butylcarbonylamino, Pentylcarbonylamino, Hexylcarbonylamino, Phenylcarbonylamino, Benzylcarbonylamino, R⁷, -OR³, -(CH₂)ₘOR³, -(CH₂)ₚCO₂R³, -SR³, -SO₂N(R^{4a})R^{4b}, -NR^{5a}R^{5b}, -NR^{5a}COR^{5b}, -NR^{5a}CO(OR^{5b}), -NR^{5a}SO₂R⁶, -NR^{5a}SO₂N (R^{6a})R^{6b}, -NR^{5a}CON(R^{6a})R^{6b}, -COR^{5a} und -CON(R^{4a})R^{4b};
worin R³, R^{4a} und R^{4b} unabhängig ausgewählt sind aus der Gruppe, bestehend aus Hydrido, Phenyl, Biphenyl, Naphthyl, Indenyl, Pyridinyl, Benzothiophenyl, Indolyl, Isochinolinyl, Chinolinyl, Thienyl, Pyrrolyl, Furyl, Pyrazolyl, Imidazolyl, Isoxazolyl, Oxazolyl, Isoindoldionyl, Pyridinylmethyl, Pyridinylethyl, Benzothiophenylmethyl, Benzothiophenylethyl, Indolylmethyl, Indolylethyl, Isochinolinylmethyl, Isochinolinylethyl, Chinolinylmethyl, Chinolinylethyl, Thienylmethyl, Thienylethyl, Pyrrolylmethyl, Pyrrolylethyl, Furylmethyl, Furylethyl, Pyrazolylmethyl, Pyrazolylethyl, Imidazolylmethyl, Imidazolylethyl, Isoxazolylmethyl, Isoxazolylethyl, Oxazolylmethyl, Oxazolylethyl, Isoindoldionylmethyl, Isoindoldionylethyl, Methyl, Ethyl, Propyl, Butyl, Pentyl, Hexyl, Chlormethyl, Dichlormethyl, Trichlormethyl, Fluormethyl, Difluormethyl, Trifluormethyl, Ethenyl, Propenyl, Butenyl, Pentenyl, Ethinyl, Propinyl, Butinyl, Pentinyl, Hydroxymethyl, Hydroxyethyl, Hydroxypropyl, Hydroxybutyl, Hydroxypentyl, Hydroxyhexyl, Aminomethyl, Aminoethyl, Aminopropyl, Aminobutyl, Aminopentyl, Aminohexyl, Methylaminomethyl, Ethylaminomethyl, Propylaminomethyl, Methylaminoethyl, Ethylaminoethyl, Propylaminoethyl, Methylaminopropyl, Ethylaminopropyl, Propylaminopropyl, Methylaminobutyl, Ethylaminobutyl, Propylaminobutyl, Methylaminopentyl, Ethylaminopentyl, Propylaminopentyl, Methylaminohexyl, Ethylaminohexyl, Propylaminohexyl, N,N-Dimethylaminomethyl, N,N-Dimethylaminoethyl, N-Methyl-N-ethylaminomethyl, N-Methyl-N-ethylaminoethyl, N-Methyl-N-propylaminomethyl, N-Methyl-N-propylaminoethyl, N,N-Diethylaminomethyl, N,N-Diethylaminoethyl, N-Ethyl-N-propylaminomethyl, N-Ethyl-N-propylaminoethyl, N,N-Dipropylaminomethyl, N,N-Dipropylaminoethyl, Methoxy, Ethoxy, Propoxy, Butoxy, Methoxymethyl, Methoxyethyl, Methoxypropyl, Ethoxymethyl, Ethoxyethyl, Ethoxypropyl, Propoxymethyl, Propoxyethyl, Propoxypropyl, Butoxymethyl, Butoxyethyl, Butoxypropyl, Piperidinyl, Pyrrolidinyl, Pyrazolidinyl, Imidazolidinyl, Isoxazolidinyl, Oxazolidinyl, Isoindolyl, Dihydroindolyl, Isoindolin, Dihydrothiophenyl, Dihydropyrrolyl, Dihydrofuryl, Dihydropyrazolyl, Dihydroimidazolyl, Dihydroisoxazolyl, Dihydrooxazolyl, Cyclopropyl, Cyclobutyl, Cyclopentyl, Cyclohexyl, Cyclopropylmethyl, Cyclopropylethyl, Cyclobutylmethyl, Cyclobutylethyl, Cyclopentylmethyl, Cyclopentylethyl, Cyclohexylmethyl, Cyclohexylethyl, Benzyl, Phenylethyl, Benzylamino und Phenylethylamino, wobei die Phenyl-, Biphenyl-, Naphthyl-oder Indenylgruppierung gegebenenfalls mit einem oder mehreren Resten, ausgewählt aus der Gruppe, bestehend aus Methyl, Ethyl, Propyl, Butyl, Pentyl, Hexyl, Aminomethyl, Aminoethyl, Aminopropyl, Aminobutyl, Aminopentyl, Aminohexyl, Methoxy, Ethoxy, Propoxy, Butoxy, Chlor, Fluor, Brom und Iod, substituiert ist, wobei R^{4a} und R^{4b} unter Bildung einer Gruppierung ausgewählt aus der Gruppe, bestehend aus Piperidinyl, Pyrrolidinyl, Pyrazolidinyl, Imidazolidinyl, Isoxazolidinyl, Oxazolidinyl, Isoindolyl, Dihydroindolyl, Isoindolin, Dihydrothiophenyl, Dihydropyrrolyl, Dihydrofuryl, Dihydropyrazolyl, Dihydroimidazolyl, Dihydroisoxazolyl, Dihydrooxazolyl, Pyridinyl, Benzothiophenyl, Indolyl, Isochinolinyl, Chinolinyl, Thienyl, Pyrrolyl, Furyl, Pyrazolyl, Imidazolyl, Isoxazolyl, Oxazolyl und Isoindoldionyl, zusammengenommen werden können;
worin R^{5a} und R^{5b} unabhängig ausgewählt sind aus der Gruppe, bestehend aus Hydrido, Methyl, Ethyl, Propyl, Butyl, Pentyl, Hexyl, Phenyl, Biphenyl, Naphthyl, Indenyl, Pyridinyl, Benzothiophenyl, Indolyl, Isochinolinyl, Chinolinyl, Thienyl, Pyrrolyl, Furyl, Pyrazolyl, Imidazolyl, Isoxazolyl, Oxazolyl, Isoindoldionyl, Benzyl, Phenylethyl, Isoindolyl, Dihydroindolyl, Isoindolin, Dihydrothiophenyl, Dihydropyrrolyl, Dihydrofuryl, Dihydropyrazolyl, Dihydroimidazolyl, Dihydroisoxazolyl, Dihydrooxazolyl, Cyclopropyl, Cyclobutyl, Cyclopentyl, Cyclohexyl, Piperidinyl, Pyrrolidinyl, Pyrazolidinyl, Imidazolidinyl, Isoxazolidinyl, Oxazolidinyl, Chlormethyl, Dichlormethyl, Trichlormethyl, Fluormethyl, Difluormethyl, Trifluormethyl, Benzylamino, Phenylethylamino, Amino, Aminomethyl, Aminoethyl, Aminopropyl, Aminobutyl, Aminopentyl, Aminohexyl, Aminomethylcarbonyl, Aminoethylcarbonyl, Aminopropylcarbonyl, Aminobutylcarbonyl, Aminopentylcarbonyl, Aminohexylcarbonyl, Aminophenylcarbonyl, Aminobenzylcarbonyl, Nitro, Azido, Pyridinylmethyl, Pyridinylethyl, Benzothiophenylmethyl, Benzothiophenylethyl, Indolylmethyl, Indolylethyl, Isochinolinylmethyl, Isochinolinylethyl, Chinolinylmethyl, Chinolinylethyl, Thienylmethyl, Thienylethyl, Pyrrolylmethyl, Pyrrolylethyl, Furylmethyl, Furylethyl, Pyrazolylmethyl, Pyrazolylethyl, Imidazolylmethyl, Imidazolylethyl, Isoxazolylmethyl, Isoxazolylethyl, Oxazolylmethyl, Oxazolylethyl, Isoindoldionylmethyl und Isoindoldionylethyl, wobei die Methyl-, Ethyl-, Propyl-, Butyl-, Pentyl-, Hexyl-, Phenyl-, Biphenyl-, Naphthyl-, Indenyl-, Pyridinyl-, Benzothiophenyl-, Indolyl-, Isochinolinyl-, Chinolinyl-, Thienyl-, Pyrrolyl-, Furyl-, Pyrazolyl-, Imidazolyl-, Isoxazolyl-, Oxazolyl-, Isoindoldionyl, Aminomethyl-, Aminoethyl-, Aminopropyl-, Aminobutyl-, Aminopentyl-, Aminohexyl-, Benzyl- und Phenylethylgruppierungen gegebenenfalls mit einem oder mehreren Substituenten, ausgewählt aus der Gruppe, bestehend aus Methylsulfonamido, Ethylsulfonamido, Propylsulfonamido, Butylsulfonamido, Sulfamyl, Methyl, Ethyl, Propyl, Butyl, Pentyl, Hexyl, Methylthio, Ethylthio, Propylthio, Butylthio, Methylsulfinyl, Ethylsulfinyl, Propylsulfinyl, Butylsulfinyl, Methylsulfonyl, Ethylsulfonyl, Propylsulfonyl, Butylsulfonyl, N-Methylamino, N-Ethylamino, N-Propylamino, Aminomethyl, Aminoethyl, Aminopropyl, Aminobutyl, Aminopentyl, Aminohexyl, Methylaminomethyl, Ethylaminomethyl, Propylaminomethyl, Methylaminoethyl, Ethylaminoethyl, Propylaminoethyl, Methylaminopropyl, Ethylaminopropyl, Propylaminopropyl, Methylaminobutyl, Ethylaminobutyl, Propylaminobutyl, Methylaminopentyl, Ethylaminopentyl, Propylaminopentyl, Methylaminohexyl, Ethylaminohexyl, Propylaminohexyl, Methoxy, Ethoxy, Propoxy, Butoxy, Chlor, Fluor, Brom, Iod, Acyloxy, Oxy, Formyl, Chlormethyl, Dichlormethyl, Trichlormethyl, Fluormethyl, Difluormethyl, Trifluormethyl, Cyano, Chlormethoxy, Dichlormethoxy, Trichlormethoxy, Fluormethoxy, Difluormethoxy, Trifluormethoxy, Methylcarbonyl, Ethylcarbonyl, Propylcarbonyl, Butylcarbonyl, Pentylcarbonyl, Hexylcarbonyl, Phenylcarbonyl, Benzylcarbonyl, Carboxyl, Hydroxy, Hydroxymethoxy, Hydroxyethoxy, Hydroxypropoxy, Hydroxybutoxy, Phenoxy, Nitro, Azido, Benzyloxy, N,N-Dimethylaminomethylcarbonyl, N,N-Dimethylaminoethylcarbonyl, N,N-Dimethylaminophenylcarbonyl, N-Methyl-N-ethylaminomethylcarbonyl, N-Methyl-N-ethylaminoethylcarbonyl, N-Methyl-N-ethylaminophenylcarbonyl, N-Methyl-N-propylaminomethylcarbonyl, N-Methyl-N-propyläminoethylcarbonyl, N-Methyl-N-propylaminophenylcarbonyl, N,N-Diethylaminomethylcarbonyl, N,N-Diethylaminoethylcarbonyl, N,N-Diethylaminophenylcarbonyl, N-Ethyl-N-propylaminomethylcarbonyl, N-Ethyl-N-propylaminoethylcarbonyl, N-Ethyl-N-propylaminophenylcarbonyl, N,N-Dipropylaminomethylcarbonyl, N,N-Dipropylaminoethylcarbonyl, N,N-Dipropylaminophenylcarbonyl, Thiomethyl, Thioethyl, Thiopropyl, Thiobutyl, Thiopentyl, Thiohexyl, Aminomethylcarbonyloxy, Aminoethylcarbonyloxy, Aminopropylcarbonyloxy, Aminobutylcarbonyloxy, Aminopentylcarbonyloxy, Aminohexylcarbonyloxy, Aminophenylcarbonyloxy, Aminobenzylcarbonyloxy, Thiocyanato, Isothiocyanato, Methyldioxy, Ethyldioxy, Propyldioxy, Butyldioxy, Pentyldioxy, Hexyldioxy, Hydroxymethyl, Hydroxyethyl, Hydroxypropyl, Hydroxybutyl, Hydroxypentyl, Hydroxyhexyl, N-Methylamino, N-Ethylamino, N-Propylamino, Methoxycarbonyl, Ethoxycarbonyl, Propoxycarbonyl, Butoxycarbonyl, Methoxymethyl, Methoxyethyl, Methoxypropyl, Ethoxymethyl, Ethoxyethyl, Ethoxypropyl, Propoxymethyl, Propoxyethyl, Propoxypropyl, Butoxymethyl, Butoxyethyl, Butoxypropyl, Ethenylamino, Propenylamino, Butenylamino, Pentenylamino, Ethinylamino, Propinylamino, Butinylamino, Pentinylamino, Ethenyl, Propenyl, Butenyl, Pentenyl, Ethinyl, Propinyl, Butinyl, Pentinyl, N,N-Dimethylaminomethoxy, N,N-dimethylaminoethoxy, N-Methyl-N-ethylaminomethoxy, N-Methyl-N-ethylaminoethoxy, N-Methyl-N-propylaminomethoxy, N-Methyl-N-propylaminoethoxy, N,N-Diethylaminomethoxy, N,N-diethylaminoethoxy, N-Ethyl-N-propylaminomethoxy, N-Ethyl-N-propylaminoethoxy, N,N-Dipropylaminomethoxy, N,N-dipropylaminoethoxy, Piperidinyl, Pyrrolidinyl, Pyrazolidinyl, Imidazolidinyl, Isoxazolidinyl, Oxazolidinyl, Isoindolyl, Dihydroindolyl, Isoindolin, Dihydrothiophenyl, Dihydropyrrolyl, Dihydrofuryl, Dihydropyrazolyl, Dihydroimidazolyl, Dihydroisoxazolyl, Dihydrooxazolyl, Pyridinyl, Benzothiophenyl, Indolyl, Isochinolinyl, Chinolinyl, Thienyl, Pyrrolyl, Furyl, Pyrazolyl, Imidazolyl, Isoxazolyl, Oxazolyl und Isoindoldionyl substituiert sind;
worin R^{6a} und R^{6b} unabhängig ausgewählt sind aus der Gruppe, bestehend aus Hydrido, Methyl, Ethyl, Propyl, Butyl, Pentyl, Hexyl, Pyridinyl, Benzothiophenyl, Indolyl, Isochinolinyl, Chinolinyl, Thienyl, Pyrrolyl, Furyl, Pyrazolyl, Imidazolyl, Isoxazolyl, Oxazolyl, Isoindoldionyl, Isoindolyl, Dihydroindolyl, Isoindolin, Dihydrothiophenyl, Dihydropyrrolyl, Dihydrofuryl, Dihydropyrazolyl, Dihydroimidazolyl, Dihydroisoxazolyl, Dihydrooxazolyl, Chlormethyl, Dichlormethyl, Trichlormethyl, Fluormethyl, Difluormethyl, Trifluormethyl, Benzylamino, Phenylethylamino, Pyridinylmethyl, Pyridinylethyl, Benzothiophenylmethyl, Benzothiophenylethyl, Indolylmethyl, Indolylethyl, Isochinolinylmethyl, Isochinolinylethyl, Chinolinylmethyl, Chinolinylethyl, Thienylmethyl, Thienylethyl, Pyrrolylmethyl, Pyrrolylethyl, Furylmethyl, Furylethyl, Pyrazolylmethyl, Pyrazolylethyl, Imidazolylmethyl, Imidazolylethyl, Isoxazolylmethyl, Isoxazolylethyl, Oxazolylmethyl, Oxazolylethyl, Isoindoldionylmethyl, Isoindoldionylethyl, Phenyl, Biphenyl, Naphthyl, Indenyl, Benzyl und Phenylethyl, wobei die Phenyl-, Biphenyl-, Naphthyl-, Indenyl-, Pyridinyl-, Benzothiophenyl-, Indolyl-, Isochinolinyl-, Chinolinyl-, Thienyl-, Pyrrolyl-, Furyl-, Pyrazolyl-, Imidazolyl-, Isoxazolyl-, Oxazolyl-, Isoindoldionyl, Isoindolyl-, Dihydroindolyl-, Isoindolin, Dihydrothiophenyl-, Dihydropyrrolyl-, Dihydrofuryl-, Dihydropyrazolyl-, Dihydroimidazolyl-, Dihydroisoxazolyl-, Dihydrooxazolyl-, Benzyl- und Phenylethylgruppierungen gegebenenfalls mit einem oder mehreren Substituenten, ausgewählt aus Methyl, Ethyl, Propyl, Butyl, Pentyl, Hexyl, Methoxy, Ethoxy, Propoxy, Butoxy, Chlor, Fluor, Brom, Iod, Chlormethyl, Dichlormethyl, Trichlormethyl, Fluormethyl, Difluormethyl, Trifluormethyl, Cyano, Chlormethoxy, Dichlormethoxy, Trichlormethoxy, Fluormethoxy, Difluormethoxy, Trifluormethoxy, Methylcarbonyl, Ethylcarbonyl, Propylcarbonyl, Butylcarbonyl, Pentylcarbonyl, Hexylcarbonyl, Phenylcarbonyl, Benzylcarbonyl, Carboxyl, Hydroxy, Hydroxymethoxy, Hydroxyethoxy, Hydroxypropoxy, Hydroxybutoxy, Phenoxy, Benzyloxy, N,N-Dimethylaminomethoxy, N,N-dimethylaminoethoxy, N-Methyl-N-ethylaminomethoxy, N-Methyl-N-ethylaminoethoxy, N-Methyl-N-propylaminomethoxy, N-Methyl-N-propylaminoethoxy, N,N-Diethylaminomethoxy, N,N-Diethylaminoethoxy, N-Ethyl-N-propylaminomethoxy, N-Ethyl-N-propylaminoethoxy, N,N-Dipropylaminomethoxy, N,N-Dipropylaminoethoxy, Piperidinyl, Pyrrolidinyl, Pyrazolidinyl, Imidazolidinyl, Isoxazolidinyl, Oxazolidinyl, Isoindolyl, Dihydroindolyl, Isoindolin, Dihydrothiophenyl, Dihydropyrrolyl, Dihydrofuryl, Dihydropyrazölyl, Dihydroimidazolyl, Dihydroisoxazolyl, Dihydrooxazolyl, Pyridinyl, Benzothiophenyl, Indolyl, Isochinolinyl, ,Chinolinyl, Thienyl, Pyrrolyl, Furyl, Pyrazolyl, Imidazolyl, Isoxazolyl, Oxazolyl und Isoindoldionyl, substituiert sein können, wobei R^{6a} und R^{6b} unter Bildung einer Gruppierung, ausgewählt aus der Gruppe, bestehend aus Piperidinyl, Pyrrolidinyl, Pyrazolidinyl, Imidazolidinyl, Isoxazolidinyl, Oxazolidinyl, Isoindolyl, Dihydroindolyl, Isoindolin, Dihydrothiophenyl, Dihydropyrrolyl, Dihydrofuryl, Dihydropyrazolyl, Dihydroimidazolyl, Dihydroisoxazolyl, Dihydrooxazolyl, Pyridinyl, Benzothiophenyl, Indolyl, Isochinolinyl, Chinolinyl, Thienyl, Pyrrolyl, Furyl, Pyrazolyl, Imidazolyl, Isoxazolyl, Oxazolyl und Isoindoldionyl zusammengenommen werden können; und
worin R⁷ ausgewählt ist aus der Gruppe, bestehend aus Phenyl, Biphenyl, Naphthyl, Indenyl, Isoindolyl, Dihydroindolyl, Isoindolin, Dihydrothiophenyl, Dihydropyrrolyl, Dihydrofuryl, Dihydropyrazolyl, Dihydroimidazolyl, Dihydroisoxazolyl, Dihydrooxazolyl, Pyridinyl, Benzothiophenyl, Indolyl, Isochinolinyl, Chinolinyl, Thienyl, Pyrrolyl, Furyl, Pyrazolyl, Imidazolyl, Isoxazolyl, Oxazolyl, Isoindoldionyl, Ethenyl, Propenyl, Butenyl und Pentenyl, wobei R⁷ gegebenenfalls mit einem oder mehreren Substituenten, ausgewählt aus der Gruppe, bestehend aus R^{5a}, substituiert ist;
oder ein pharmazeutisch annehmbares Salz davon.

4. Verbindung gemäß Anspruch 1, wobei die Verbindung der Formel I eine Verbindung der Formel II ist: 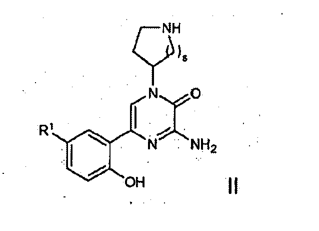 worin s 1, 2 oder 3 ist;
worin R¹ ausgewählt ist aus der Gruppe, bestehend aus Hydrido, -OR ³ C₁₋₆-Alkyl, C₅₋₇-Cycloalkyl; Benzyl, -CH₂(C₃₋₇-Cycloalkyl), Aryl, Halogen, Heterocycloalkyl, Heterocycloalkenyl und Heteroaryl;
worin R³ ausgewählt ist aus der Gruppe, bestehend aus Hydrido, C₁₋₆-Alkyl, C₅₋₇-Cycloalkyl, Benzyl, -CH₂(C₃₋₇-Cycloalkyl) und Aryl;
oder ein pharmazeutisch annehmbares Salz davon.

5. Verbindung gemäß Anspruch 4:
worin R¹ ausgewählt ist aus der Gruppe, bestehend aus Hydrido, -OR³, C₁₋₆-Alkyl, C₅₋₇-Cycloalkyl, Benzyl, -CH₂ (C₃₋₇Cycloalkyl), C₃₋₁₂-Aryl, Halogen, 3- bis 12- gliedrigem Heterocycloalkyl, 3-bis 12-gliedrigem Heterocycloalkenyl und 3- bis 12-gliedrigem Heteroaryl; und
worin R³ ausgewählt ist aus der Gruppe, bestehend aus Hydrido, C₁₋₆-Alkyl, C₅₋₇-Cycloalkyl, Benzyl, -CH₂(C₃₋₇Cycloalkyl) und C₃₋₁₂-Aryl;
oder ein pharmazeutisch annehmbares Salz davon.

6. Verbindung gemäß Anspruch 5:
worin R¹ ausgewählt ist aus der Gruppe, bestehend aus Hydrido, -OR³, Methyl, Ethyl, Propyl, Butyl, Pentyl, Hexyl, Cyclopentyl, Cyclohexyl, Cycloheptyl, Benzyl, Methylcyclopropyl, Methylcyclobutyl, Methylcyclopentyl, Methylcyclohexyl, Phenyl, Biphenyl, Naphthyl, Indenyl, Chlor, Fluor, Brom, Iod, Piperidinyl, Pyrrolidinyl, Pyrazolidinyl, Imidazolidinyl, Isoxazolidinyl, Oxazolidinyl, Isoindolyl, Dihydroindolyl, Isoindolin, Dihydrothiophenyl, Dihydropyrrolyl, Dihydrofuryl, Dihydropyrazolyl, Dihydroimidazolyl, Dihydroisoxazolyl, Dihydrooxazolyl, Pyridinyl, Benzothiophenyl, Indolyl, Isochinolinyl, Chinolinyl, Thienyl, Pyrrolyl, Furyl, Pyrazolyl, Imidazolyl, Isoxazolyl, Oxazolyl und Isoindoldionyl; und
worin R³ ausgewählt ist aus der Gruppe, bestehend aus Hydrido, Methyl, Ethyl, Propyl, Butyl, Pentyl, Hexyl, Cyclopentyl, Cyclohexyl, Cycloheptyl, Benzyl, Methylcyclopropyl, Methylcyclobutyl, Methylcyclopentyl, Methylcyclohexyl, Phenyl, Biphenyl, Naphthyl und Indenyl;
oder ein pharmazeutisch annehmbares Salz davon.

7. Verbindung gemäß Anspruch 1, wobei die Verbindung der Formel I eine Verbindung der Formel III ist: 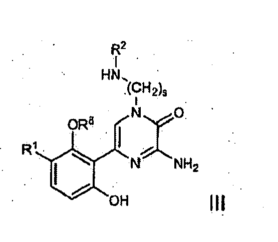 worin s 1, 2 oder 3 ist;
worin R¹ ausgewählt ist aus der Gruppe, bestehend aus Hydrido, -OR³, C₁₋₆-Alkyl, C₅₋₇-Cycloalkyl, Benzyl, -CH₂(C₃₋₇-Cycloalkyl), Aryl, Halogen, Heterocycloalkyl, Heterocycloalkenyl und Heteroaryl;
worin R² Hydrido oder C₁₋₆-Alkyl ist;
worin R³ ausgewählt ist aus der Gruppe, bestehend aus C₁₋₆-Alkyl, C₅₋₇-Cycloalkyl, Benzyl, -CH₂(C₃₋₇-Cycloalkyl) und Aryl; und
worin R⁸ ausgewählt ist aus der Gruppe, bestehend aus C₁₋₆-Alkyl, C₅₋₇-Cycloalkyl, Benzyl und -CH₂(C₃₋₇-Cycloalkyl)
oder ein pharmazeutisch annehmbares Salz davon.

8. Verbindung gemäß Anspruch 7:
worin R¹ ausgewählt ist aus der Gruppe, bestehend aus Hydrido, -OR³, C₁₋₆-Alkyl, C₅₋₇-Cycloalkyl, Benzyl, -CH₂(C₃₋₇-Cycloalkyl), C₃₋₁₂-Aryl, Halogen, 3- bis 12-gliedrigem Heterocycloalkyl, 3-bis 12- gliedrigem Heterocycloalkenyl und 3- bis 12- gliedrigem Heteroaryl;
worin R² Hydrido oder C₁₋₆-Alkyl ist;
worin R³ ausgewählt ist aus der Gruppe, bestehend aus C₁₋₆-Alkyl, C₅₋₇-Cycloalkyl, Benzyl, -CH₂(C₃₋₇-Cycloalkyl), und C₃₋₁₂-Aryl; und
worin R⁸ ausgewählt ist aus der Gruppe, bestehend aus C₁₋₆-Alkyl, C₅₋₇-Cycloalkyl, Benzyl und -CH₂(C₃₋₇-Cycloalkyl) ;
oder ein pharmazeutisch annehmbares Salz davon.

9. Verbindung gemäß Anspruch 8:
worin R¹ ausgewählt ist aus der Gruppe, bestehend aus Hydrido, -OR³, Methyl, Ethyl, Propyl, Butyl, Pentyl, Hexyl, Cyclopentyl, Cyclohexyl, Cycloheptyl, Benzyl, Methylcyclopropyl, Methylcyclobutyl, Methylcyclopentyl, Methylcyclohexyl, Phenyl, Biphenyl, Naphthyl, Indenyl, Chlor, Fluor, Brom, Iod, Piperidinyl, Pyrrolidinyl, Pyrazolidinyl, Imidazolidinyl, Isoxazolidinyl, Oxazolidinyl, Isoindolyl, Dihydroindolyl, Isoindolin, Dihydrothiophenyl, Dihydropyrrolyl, Dihydrofuryl, Dihydropyrazolyl, Dihydroimidazolyl, Dihydroisoxazolyl, Dihydrooxazolyl, Pyridinyl, Benzothiophenyl, Indolyl, Isochinolinyl, Chinolinyl, Thienyl, Pyrrolyl, Furyl, Pyrazolyl, Imidazolyl, Isoxazolyl, Oxazolyl und Isoindoldionyl;
worin R² ausgewählt ist aus der Gruppe, bestehend aus Hydrido, Methyl, Ethyl, Propyl, Butyl, Pentyl und Hexyl;
worin R³ ausgewählt ist aus der Gruppe, bestehend aus Methyl, Ethyl, Propyl, Butyl, Pentyl, Hexyl, Cyclopentyl, Cyclohexyl, Cycloheptyl, Benzyl, Methylcyclopropyl, Methylcyclobutyl, Methylcyclopentyl, Methylcyclohexyl, Phenyl, Biphenyl, Naphthyl und Indenyl; und
worin R⁸ ausgewählt ist aus der Gruppe, bestehend aus Methyl, Ethyl, Propyl, Butyl, Pentyl, Hexyl, Cyclopentyl, Cyclohexyl, Cycloheptyl, Benzyl, Methylcyclopropyl, Methylcyclobutyl, Methylcyclopentyl und Methylcyclohexyl;
oder ein pharmazeutisch annehmbares Salz davon.

10. Verbindung gemäß Anspruch 1, wobei die Verbindung der Formel I eine Verbindung der Formel IV ist: 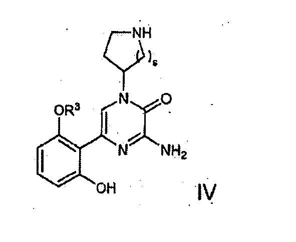 worin n 1, 2 oder 3 ist; und
worin R³ ausgewählt ist aus der Gruppe, bestehend aus Hydrido, C₁₋₆-Alkyl, C₅₋₇-Cycloalkyl, Benzyl und -CH₂(C₃₋₇-Cycloalkyl) ;
oder ein pharmazeutisch annehmbares Salz davon.

11. Verbindung gemäß Anspruch 10,
worin R³ ausgewählt ist aus der Gruppe, bestehend aus Hydrido, Methyl, Ethyl, Propyl, Butyl, Pentyl, Hexyl, Cyclopentyl, Cyclohexyl, Cycloheptyl, Benzyl, Methylcyclopropyl, Methylcyclobutyl, Methylcyclopentyl und Methylcyclohexyl;
oder ein pharmazeutisch annehmbares Salz davon.

12. Verbindung gemäß Anspruch 1, ausgewählt aus der Gruppe von Verbindungen, bestehend aus:
3-Amino-5-(2-hydroxyphenyl)-1-piperidin-3-ylpyrazin-2(1H)-on;
3-Amino-5-(5-fluor-2-hydroxyphenyl)-1-piperidin-3-ylpyrazin-2(1H)-on;
3-Amino-5-(4-fluor-2-hydroxyphenyl)-1-piperidin-3-ylpyrazin-2(1H)-on;
3-Amino-5-(3-fluor-2-hydroxyphenyl)-1-piperidin-3-ylpyrazin-2(1H)-on;
3-Amino-5-(2-fluor-6-hydroxyphenyl)-1-piperidin-3-ylpyrazin-2(1H)-on;
3-Amino-5-(2-brom-6-hydroxyphenyl)-1-piperidin-3-ylpyrazin-2(1H)-on;
3-Amino-5-(5-brom-2-hydroxyphenyl)-1-piperidin-3-ylpyrazin-2(1H)-on;
3-Amino-5-(4-brom-2-hydroxyphenyl)-1-piperidin-3-ylpyrazin-2(1H)-on;
3-Amino-5-(3-brom-2-hydroxyphenyl)-1-piperidin-3-ylpyrazin-2(1H)-on;
3-Amino-5-(2-chlor-6-hydroxyphenyl)-1-piperidin-3-ylpyrazin-2(1H)-on;
3-Amino-5-(5-chlor-2-hydroxyphenyl)-1-piperidin-3-ylpyrazin-2(1H)-on;
3-Amino-5-(4-chlor-2-hydroxyphenyl)-1-piperidin-3-ylpyrazin-2(1H)-on;
3-Amino-5-(3-chlor-2-hydroxyphenyl)-1-piperidin-3-ylpyrazin-2(1H)-on;
3-Amino-5-(2-hydroxy-6-methylphenyl)-1-piperidin-3-ylpyrazin-2(1H)-on;
3-Amino-5-(2-hydroxy-5-methylphenyl)-1-piperidin-3-ylpyrazin-2(1H)-on;
3-Amino-5-(2-hydroxy-4-methylphenyl)-1-piperidin-3-ylpyrazin-2(1H)-on;
3-Amino-5-(2-hydroxy-3-methylphenyl)-1-piperidin-3-ylpyrazin-2(1H)-on;
3-Amino-5-(5-cyclopentyl-2-hydroxyphenyl)-1-piperidin-3-ylpyrazin-2(1H)-on;
3-Amino-5-(5-cyclohexyl-2-hydroxyphenyl)-1-piperidin-3-ylpyrazin-2(1H)-on;
3-Amino-5-(5-cycloheptyl-2-hydroxyphenyl)-1-piperidin-3-ylpyrazin-2(1H)-on;
3-Amino-5-(5-cyclopropylmethyl-2-hydroxyphenyl)-1-piperidin-3-ylpyrazin-2(1H)-on;
3-Amino-5-(5-cyclobutylmethyl-2-hydroxyphenyl)-1-piperidin-3-ylpyrazin-2(1H)-on;
3-Amino-5-(5-cyclopentylmethyl-2-hydroxyphenyl)-1-piperidin-3-ylpyrazin-2(1H)-on;
3-Amino-5-(5-cyclohexylmethyl-2-hydroxyphenyl)-1-piperidin-3-ylpyrazin-2(1H)-on;
3-Amino-5-(5-phenyl-2-hydroxyphenyl)-1-piperidin-3-ylpyrazin-2(1H)-on;
3-Amino-5-[2-hydroxy-5-(1-naphthyl)phenyl]-1-piperidin-3-ylpyrazin-2(1H)-on;
3-Amino-5-[2-hydroxy-5-(2-naphthyl)phenyl]-1-piperidin-3-ylpyrazin-2(1H)-on;
3-Amino-5-(2-hydroxy-5-pyrrolidin-2-ylphenyl)-1-piperidin-3-ylpyrazin-2(1H)-on;
3-Amino-5-(2-hydroxy-5-pyrrolidin-3-ylphenyl)-1-piperidin-3-ylpyrazin-2(1H)-on;
3-Amino-5-(2-hydroxy-5-pyrrolidin-1-ylphenyl)-1-piperidin-3-ylpyrazin-2(1H)-on;
3-Amino-5-(2-hydroxy-5-pyrazolidin-1-ylphenyl)-1-piperidin-3-ylpyrazin-2(1H)-on;
3-Amino-5-(2-hydroxy-5-imidazolidin-1-ylphenyl)-1-piperidin-3-ylpyrazin-2(1H)-on;
3-Amino-5-(2-hydroxy-5-pyrazolidin-3-ylphenyl)-1-piperidin-3-ylpyrazin-2(1H)-on;
3-Amino-5-(2-hydroxy-5-pyrazolidin-4-ylphenyl)-1-piperidin-3-ylpyrazin-2(1H)-on;
3-Amino-5-(2-hydroxy-5-imidazolidin-1-ylphenyl)-1-piperidin-3-ylpyrazin-2(1H)-on;
3-Amino-5-(2-hydroxy-5-imidazolidin-4-ylphenyl)-1-piperidin-3-ylpyrazin-2(1H)-on;
3-Amino-5-(2-hydroxy-5-tetrahydrofuran-2-ylphenyl)-1-piperidin-3-ylpyrazin-2(1H)-on;
3-Amino-5-(2-hydroxy-5-tetrahydrofuran-3-ylphenyl)-1-piperidin-3-ylpyrazin-2(1H)-on;
3-Amino-5-(2-hydroxy-5-tetrahydrothien-2-ylphenyl)-1-piperidin-3-ylpyrazin-2(1H)-on;
3-Amino-5-(2-hydroxy-5-tetrahydrothien-3-ylphenyl)-1-piperidin-3-ylpyrazin-2(1H)-on;
3-Amino-5-(2-hydroxy-5-isoxazolidin-2-ylphenyl)-1-piperidin-3-ylpyrazin-2(1H)-on;
3-Amino-5-[2-hydroxy-5-(1,3-oxazolidin-3-yl)phenyl]-1-piperidiri-3-ylpyrazin-2(1H)-on;
3-Amino-5-(2-hydroxy-5-isoxazolidin-3-ylphenyl)-1-piperidin-3-ylpyrazin-2(1H)-on;
3-Amino-5-[2-hydroxy-5-(1,3-oxazolidin-4-yl)phenyl]-1-piperidin-3-ylpyrazin-2(1H)-on;
3-Amino-5-[2-hydroxy-5-(1,3-oxazolidin-2-yl)phenyl]-1-piperidin-3-ylpyrazin-2(1H)-on;
3-Amino-5-(2-hydroxy-5-isoxazolidin-4-ylphenyl)-1-piperidin-3-ylpyrazin-2(1H)-on;
3-Amino-5-[2-hydroxy-5-(1,3-oxazolidin-5-yl)phenyl]-1-piperidin-3-ylpyrazin-2(1H)-on;
3-Amino-5-(2-hydroxy-5-isoxazolidin-5-ylphenyl)-1-piperidin-3-ylpyrazin-2(1H)-on;
3-Amino-5-[2-hydroxy-5-(1H-pyrrol-1-yl)phenyl]-1-piperidin-3-ylpyrazin-2(1H)-on;
3-Amino-5-[2-hydroxy-5-(1H-pyrrol-2-yl)phenyl]-1-piperidin-3-ylpyrazin-2(1H)-on;
3-Amino-5-[2-hydroxy-5-(1H-pyrrol-3-yl)phenyl]-1-piperidin-3-ylpyrazin-2(1H)-on;
3-Amino-5-[2-hydroxy-5-(1H-pyrazol-1-yl)phenyl]-1-piperidin-3-ylpyrazin-2(1H)-on;
3-Amino-5-[2-hydroxy-5-(1H-imidazol-1-yl)phenyl]-1-piperidin-3-ylpyrazin-2(1H)-on;
3-Amino-5-[2-hydroxy-5-(1H-pyrazol-3-yl)phenyl]-1-piperidin-3-ylpyrazin-2(1H)-on;
3-Amino-5-[2-hydroxy-5-(1H-imidazol-4-yl)phenyl]-1-piperidin-3-ylpyrazin-2(1H)-on;
3-Amino-5-[2-hydroxy-5-(1H-imidazol-2-yl)phenyl]-1-piperidin-3-ylpyrazin-2(1H)-on;
3-Amino-5-[5-(2-furyl)-2-hydroxyphenyl]-1-piperidin-3-ylpyrazin-2(1H)-on;
3-Amino-5-[5-(3-furyl)-2-hydroxyphenyl]-1-piperidin-3-ylpyrazin-2(1H)-on;
3-Amino-5-(2-hydroxy-5-thien-2-ylphenyl)-1-piperidin-3-ylpyrazin-2(1H)-on;
3-Amino-5-(2-hydroxy-5-thien-3-ylphenyl)-1-piperidin-3-ylpyrazin-2(1H)-on;
3-Amino-5-(2-hydroxy-5-isoxazolidin-2-ylphenyl)-1-piperidin-3-ylpyrazin-2(1H)-on;
3-Amino-5-(2-hydroxy-5-isoxazol-3-ylphenyl)-1-piperidin-3-ylpyrazin-2(1H)-on;
3-Amino-5-[2-hydroxy-5-(1,3-oxazol-4-yl)phenyl]-1-piperidin-3-ylpyrazin-2(1H)-on;
3-Amino-5-[2-hydroxy-5-(1,3-oxazol-2-yl)phenyl]-1-piperidin-3-ylpyrazin-2(1H)-on;
3-Amino-5-(2-hydroxy-5-isoxazol-4-ylphenyl)-1-piperidin-3-ylpyrazin-2(1H)-on;
3-Amino-5-[2-hydroxy-5-(1,3-oxazol-5-yl)phenyl]-1-piperidin-3-ylpyrazin-2(1H)-on;
3-Amino-5-(2-hydroxy-5-isoxazol-5-ylphenyl)-1-piperidin-3-ylpyrazin-2(1H)-on;
3-Amino-5-(2-hydroxy-5-piperidin-1-ylphenyl)-1-piperidin-3-ylpyrazin-2(1H)-on;
3-Amino-5-(2-hydroxy-5-piperidin-2-ylphenyl)-1-piperidin-3-ylpyrazin-2(1H)-on ;
3-Amino-5-(2-hydroxy-5-piperidin-3-ylphenyl)-1-piperidin-3-ylpyrazin-2(1H)-on ;
3-Amino-5-(2-hydroxy-5-piperidin-4-ylphenyl)-1-piperidin-3-ylpyrazin-2(1H)-on;
3-Amino-5-(2-hydroxy-5-piperazin-1-ylphenyl)-1-piperidin-3-ylpyrazin-2(1H)-on;
3-Amino-5-(2-hydroxy-5-piperazin-2-ylphenyl)-1-piperidin-3-ylpyrazin-2(1H)-on;
3-Amino-5-(2-hydroxy-5-pyridin-2-ylphenyl)-1-piperidin-3-ylpyrazin-2(1H)-on;
3-Amino-5-(2-hydroxy-5-pyridin-3-ylphenyl)-1-piperidin-3-ylpyrazin-2(1H)-on;
3-Amino-5-(2-hydroxy-5-pyridin-4-ylphenyl)-1-piperidin-3-ylpyrazin-2(1H)-on;
3-Amino-5-(2-hydroxy-5-pyridazin-3-ylphenyl)-1-piperidin-3-ylpyrazin-2(1H)-on;
3-Amino-5-(2-hydroxy-5-pyridazin-4-ylphenyl)-1-piperidin-3-ylpyrazin-2(1H)-on;
3-Amino-5-(2-hydroxy-5-pyrazin-2-ylphenyl)-1-piperidin-3-ylpyrazin-2(1H)-on;
3-Amino-5-(5-benzyl-2-hydroxyphenyl)-1-piperidin-3-ylpyrazin-2(1H)-on;
3-Amino-5-(2,5-dihydroxyphenyl)-1-piperidin-3-ylpyrazin-2(1H)-on;
3-Amino-5-(2-hydroxy-5-methoxyphenyl)-1-piperidin-3-ylpyrazin-2(1H)-on;
3-Amino-5-(5-ethoxy-2-hydroxyphenyl)-1-piperidin-3-ylpyrazin-2(1H)-on;
3-Amino-5-(2-hydroxy-5-propoxyphenyl)-1-piperidin-3-ylpyrazin-2(1H)-on;
3-Amino-5-(2-hydroxy-5-isopropoxyphenyl)-1-piperidin-3-ylpyrazin-2(1H)-on;
3-Amino-5-(2-hydroxy-5-t-butoxyphenyl)-1-piperidin-3-ylpyrazin-2(1H)-on;
3-Amino-5-(2-hydroxy-5-phenoxyphenyl)-1-piperidin-3-ylpyrazin-2(1H)-on;
3-Amino-5-[2-hydroxy-5-(1-naphthyloxy)phenyl]-1-piperidin-3-ylpyrazin-2(1H)-on;
3-Amino-5-[2-hydroxy-5-(2-naphthyloxy)phenyl]-1-piperidin-3-ylpyrazin-2(1H)-on;
3-Amino-5-[5-(benzyloxy)-2-hydroxyphenyl]-1-piperidin-3-ylpyrazin-2(1H)-on;
3-Amino-5-[5-(cyclopropylmethoxy)-2-hydroxyphenyl]-1-piperidin-3-ylpyrazin-2(1H)-on;
3-Amino-5-[5-(cyclopentylmethoxy)-2-hydroxyphenyl]-1-piperidin-3-ylpyrazin-2(1H)-on;
3-Amino-5-[5-(cyclohexylmethoxy)-2-hydroxyphenyl]-1-piperidin-3-ylpyrazin-2(1H)-on;
3-Amino-5-[5-(cyclohexyloxy)-2-hydroxyphenyl]-1-piperidin-3-ylpyrazin-2(1H)-on; und
3-Amino-5-[5-(cyclopentyloxy)-2-hydroxyphenyl]-1-piperidin-3-ylpyrazin-2(1H)-on.

13. Pharmazeutische Zusammensetzung, umfassend eine Verbindung gemäß einem der Ansprüche 1-12 oder ein pharmazeutisch annehmbares Salz davon und einen pharmazeutisch annehmbaren Träger, ein pharmazeutisch annehmbares Verdünnungsmittel oder Adjuvans.

14. Verwendung einer Verbindung gemäß einem der Ansprüche 1-2 zur Herstellung eines Medikamentes für die Behandlung von Krebs, Entzündung oder einer entzündungsassoziierten Störung bei einer Person.

15. Verwendung gemäß Anspruch 14, wobei das Medikament für die Behandlung von Arthritis, Krebs, Asthma, COPD, Gebrechlichkeit bzw. Schwäche, Diabetes, Atherosklerose, Schmerz und/oder dermatologischer Erkrankung ist.

## Revendications

1. Composé de Formule I : 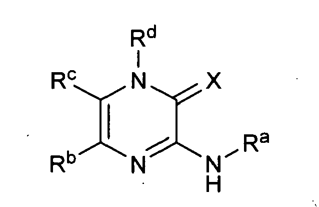 dans lequel :
X est sélectionné dans le groupe consistant en O, S et NR^{5a};
R^{a} et R^{c} sont indépendamment sélectionnés dans le groupe consistant en hydrido, hydroxyle, alcoxy, alkyle, halogénoalkyle, aryle et hétéroaryle ;
R^{b} représente un groupe fonctionnel cyclique ayant de 3 à 12 éléments, sélectionné dans le groupe consistant en cycloalkyle, cycloalkényle, aryle, hétérocycloalkyle, hétérocycloalkényle et hétéroaryle, R^{b} étant facultativement substitué par un ou plusieurs substituants sélectionné(s) dans le groupe consistant en R², cycloalkyle et cycloalkylalkyle ;
R^{d} est sélectionné dans le groupe consistant en -(CH₂)_{q}NH₂, -(CH₂)_{q}NHR² et un groupe hétérocycloalkyle ayant de 5 à 7 éléments dont des éléments de noyau sont sélectionnés dans le groupe consistant en le carbone et l'azote, ledit groupe hétérocycloalkyle pouvant être facultativement substitué par un ou plusieurs substituants sélectionné(s) dans le groupe consistant en R² ;
R² étant sélectionné dans le groupe consistant en halogéno, alkylsulfinyle, alkylsulfonyle, cyano, alcoxycarbonyle, alkyle, halogénoalkyle, hydroxyalkyle, halogénoalcoxy, nitro, acylamino, R⁷, -OR³, -(CH₂)ₘOR³, - (CH₂)ₚCO₂R³, -SR³, -SO₂N (R^{4a}) R^{4b}, -NR^{5a}R^{5b}, -NR^{5a}COR^{5b}, -NR^{5a}CO (OR^{5b}), -NR^{5a}SO₂R⁶, -NR^{5a}SO₂N (R^{6a}) R^{6b}, -NR^{5a}CON (R^{6a}) R^{6b}, -COR^{5a} et -CON(R^{4a})R^{4b} ;
R³, R^{4a} et R^{4b} étant indépendamment sélectionnés dans le groupe consistant en hydrido, aryle, hétéroaryle, hétéroaralkyle, alkyle, halogénoalkyle, alkényle, alkynyle, hydroxyalkyle, aminoalkyle, alkylaminoalkyle, N,N-dialkylaminoalkyle, alcoxy, alcoxyalkyle, hétérocycloalkyle, hétérocycloalkényle, cycloalkyle, cycloalkylalkyle, aralkyle et aralkylamino, ledit groupe aryle étant facultativement substitué par un ou plusieurs radicaux sélectionné(s) dans le groupe consistant en alkyle, aminoalkyle, alcoxy et halogéno, R^{4a} et R^{4b} pouvant être pris ensemble pour former un noyau hétérocyclique ayant de 3 à 7 éléments dont 1 à 3 hétéroatomes sélectionné(s) parmi S, SO, SO₂, O, N et NR^{5a};
R^{5a} et R^{5b} étant indépendamment sélectionnés dans le groupe consistant en hydrido, alkyle, aryle, hétéroaryle, aralkyle, hétérocycloalkényle, cycloalkyle, hétérocycloalkyle, halogénoalkyle, aralkylamino, amino, aminoalkyle, aminoacyle, nitro, azido et hétéroaralkyle, lesdits groupes fonctionnels alkyle, aryle, hétéroaryle,' aminoalkyle et aralkyle étant facultativement substitués par un ou plusieurs substituants sélectionné(s) dans le groupe consistant en alkylsulfonamido, sulfamyle, alkyle, alkylthio, alkylsulfinyle, alkylsulfonyle, N-alkylamino, aminoalkyle, alkylaminoalkyle, alcoxy, halogéno, acyloxy, oxy, formyle, halogénoalkyle, cyano, halogénoalcoxy, acyle, carboxyle, hydroxy, hydroxyalcoxy, phénoxy, nitro, azido, benzyloxy, N,N-dialkylaminoacyle, thioalkyle, aminoacyloxy, thiocyanato, isothiocyanato, alkyldioxy, hydroxyalkyle, N-alkylamino, alcoxycarbonyle, alcoxyalkyle, alkénylamino, alkynylamino, alkényle, alkynyle, N,N-dialkylaminoalcoxy, hétérocycloalkyle, hétérocycloalkényle et hétéroaryle ;
R^{6a} et R^{6b} étant indépendamment sélectionnés dans le groupe consistant en hydrido, alkyle, hétéroaryle, hétérocycloalkényle, halogénoalkyle, aralkylamino, hétéroaralkyle, aryle et aralkyle, lesdits groupes fonctionnels aryle, hétéroaryle, hétérocycloalkényle et aralkyle étant facultativement substitués par un ou plusieurs substituants sélectionné(s) parmi alkyle, alcoxy, halogéno, halogénoalkyle, cyano, halogénoalcoxy, acyle, carboxyle, hydroxy, hydroxyalcoxy, phénoxy, benzyloxy, N,N-dialkylaminoalcoxy, hétérocycloalkyle, hétérocycloalkényle et hétéroaryle, R^{6a} et R^{6b} pouvant être pris ensemble pour former un noyau hétérocyclique ayant de 3 à 7 éléments dont 1 à 3 hétéroatomes sélectionné(s) parmi S, SO, SO₂, O, N et NR^{5a};
R⁷ étant sélectionné dans le groupe consistant en aryle, hétérocycloalkényle, hétéroaryle et alkényle, R⁷ étant facultativement substitué par un ou plusieurs substituants sélectionné(s) dans le groupe consistant en R^{5a} ;
n représentant 1, 2 ou 3 ;
m représentant 1, 2 ou 3 ;
p représentant 0, 1 ou 2 ;
q étant un entier compris entre 0 et 9 ;
ou sel pharmaceutiquement acceptable de celui-ci.

2. Composé selon la revendication 1 dans lequel :
R^{a} et R^{c} sont indépendamment sélectionnés dans le groupe consistant en hydrido, hydroxyle, alcoxy en C₁₋₆, alkyle en C₁₋₆, halogénoalkyle en C₁₋₆, aryle en C₃₋₁₂ et hétéroaryle ayant de 3 à 12 éléments ;
R^{b} est un groupe fonctionnel cyclique ayant de 3 à 12 éléments sélectionné dans le groupe consistant en cycloalkyle en C₃₋₁₂, cycloalkényle en C₃₋₁₂, aryle en C₃₋₁₂, hétérocycloalkyle ayant de 3 à 12 éléments, hétérocycloalkényle ayant de 3 à 12 éléments et hétéroaryle ayant de 3 à 12 éléments, R^{b} étant facultativement substitué par un ou plusieurs substituants sélectionné(s) dans le groupe consistant en R², cycloalkyle en C₃₋₁₂ et cycloalkylalkyle en C₄₋₁₈ ;
R^{d} est sélectionné dans le groupe consistant en -(CH₂)_{q}NH₂,- -(CH₂)_{q}NHR² et un groupe hétérocycloalkyle ayant de 5 à 7 éléments dont des éléments de noyau sont sélectionnés dans le groupe consistant en le carbone et l'azote, ledit groupe hétérocycloalkyle pouvant être facultativement substitué par un ou plusieurs substituants sélectionné(s) dans le groupe consistant en R² ;
R² étant sélectionné dans le groupe consistant en halogéno, (alkyle en C₁₋₆)sulfinyle, (alkyle en C₁₋₆)sulfonyle, cyano, (alcoxy en C₂₋₇)carbonyle, alkyle en C₁₋₆, halogénoalkyle en C₁₋₆, hydroxyalkyle en C₁₋₆, halogénoalcoxy en C₁₋₆, nitro, acylamino en C₂₋₁₀, R⁷, -OR³, - (CH₂)ₘOR³, -(CH₂)ₚCO₂R³, -SR³, -SO₂N(R^{4a})R^{4b}, -NR^{5a}R^{5b}, -NR^{5a}COR^{5b}, -NR^{5a}CO(OR^{5b}), -NR^{5a}SO₂R⁶, -NR^{5a}SO₂N(R^{6a})R^{6b}, -NR^{5a}CON(R^{6a})R^{6b}, -COR^{5a} et -CON(R^{4a})R^{4b} ;
R³, R^{4a} et R^{4b} étant indépendamment sélectionnés dans le groupe consistant en hydrido, aryle en C₃₋₁₂, hétéroaryle ayant de 3 à 12 éléments, hétéroaralkyle ayant de 4 à 18 éléments, alkyle en C₁₋₆, halogénoalkyle en C₁₋₆, alkényle en C₂₋₆, alkynyle en C₂₋₆, hydroxyalkyle en C₁₋₆, aminoalkyle en C₁₋₆, (alkyle en C₂₋₁₂) aminoalkyle, N-N-di (alkyle en C₁₋₆) amino (alkyle en C₁₋₆) , alcoxy en C₁₋₆, (alcoxy en C₂₋₁₂)alkyle, hétérocycloalkyle ayant de 3 à 12 éléments, hétérocycloalkényle ayant de 3 à 12 éléments, cycloalkyle en C₃₋₁₂, (cycloalkyle en C₄₋₁₈)alkyle, aralkyle en C₄₋₁₈ et (aralkyle en C₄₋₁₈)amino, ledit groupe aryle étant facultativement substitué par un ou plusieurs radicaux sélectionné (s) dans le groupe consistant en alkyle en C₁₋₆, amino (alkyle en C₁₋₆), alcoxy en C₁₋₆ et halogéno, R^{4a} et R^{4b} pouvant être pris ensemble pour former un noyau hétérocyclique ayant de 3 à 7 éléments dont 1 à 3 hétéroatomes sélectionné(s) parmi S, SO, SO₂, O, N et NR^{5a} ;
R^{5a} et R^{5b} étant indépendamment sélectionnés dans le groupe consistant en hydrido, alkyle en C₁₋₆, aryle en C₃₋₁₂, hétéroaryle ayant de 3 à 12 éléments, aralkyle en C₄₋₁₈, hétérocycloalkényle ayant de 3 à 12 éléments, cycloalkyle en C₃₋₁₂, hétérocycloalkyle ayant de 3 à 12 éléments, halogénoalkyle en C₁₋₆, (aralkyle en C₄₋₁₈)amino, amino, aminoalkyle en C₁₋₆, aminoacyle en C₂₋₁₀, nitro, azido et hétéroaralkyle ayant de 4 à 18 éléments, lesdits groupes fonctionnels alkyle, aryle, hétéroaryle, aminoalkyle et aralkyle étant facultativement substitués par un ou plusieurs substituants sélectionné(s) dans le groupe consistant en (alkyle en C₁₋₆)sulfonamido, sulfamyle, alkyle en C₁₋₆, (alkyle en C₁₋₆)thio, (alkyle en C₁₋₆)sulfinyle, (alkyle en C₁₋₆) sulfonyle, N-(alkyle en C₁₋₆)amino, aminoalkyle en C₁₋₆, (alkyle en C₂₋₁₂) aminoalkyle, alcoxy en C₁₋₆, halogéno, acyloxy en C₂₋₁₀, oxy, formyle, halogénoalkyle en C₁₋₆, cyano, halogénoalcoxy en C₁₋₆, acyle en C₂₋₁₀, carboxyle, hydroxy, hydroxyalcoxy en C₁₋₆, phénoxy, nitro, azido, benzyloxy, N,N-di (alkyle en C₁₋₆)amino (acyle en C₂₋₁₀), thioalkyle en C₁₋₆, aminoacyloxy en C₂₋₁₀, thiocyanato, isothiocyanato, (alkyle en C₁₋₆)dioxy, hydroxyalkyle en C₁₋₆, N-(alkyle en C₁₋₆)amino, (alcoxy en C₂₋₇)carbonyle, (alcoxy en C₂₋₁₂) alkyle, (alkényle en C₂₋₆) amino, (alkynyle en C₂₋₆)amino, alkényle en C₂₋₆, alkynyle en C₂₋₆, N,N-di(alkyle en C₁₋₆)amino(alcoxy en C₁₋₆), hétérocycloalkényle ayant de 3 à 12 éléments, hétérocycloalkyle ayant de 3 à 12 éléments et hétéroaryle ayant de 3 à 12 éléments ;
R^{6a} et R^{6b} étant indépendamment sélectionnés dans le groupe consistant en hydrido, alkyle en C₁₋₆, hétéroaryle ayant de 3 à 12 éléments, hétérocycloalkényle ayant de 3 à 12 éléments, halogénoalkyle en C₁₋₆, aralkylamino en C₄₋₁₈, hétéroaralkyle ayant de 4 à 18 éléments, aryle en C₃₋₁₂ et aralkyle en C₄₋₁₈, lesdits groupes fonctionnels aryle, hétéroaryle, hétérocycloalkényle et aralkyle étant facultativement substitués par un ou plusieurs substituants sélectionné (s) parmi alkyle en C₁₋₆, alcoxy en C₁₋₆, halogéno, halogénoalkyle en C₁₋₆, cyano, halogénoalcoxy en C₁₋₆, acyle en C₂₋₁₀, carboxyle, hydroxy, hydroxyalcoxy en C₁₋₆, phénoxy, benzyloxy, N,N-di(alkyle en C₁₋₆)amino(alcoxy en C₁₋₆), hétérocycloalkényle ayant de 3 à 12 éléments, hétérocycloalkyle ayant de 3 à 12 éléments et hétéroaryle ayant de 3 à 12 éléments, R^{6a} et R^{6b} pouvant être pris ensemble pour former un noyau hétérocyclique ayant de 3 à 7 éléments dont 1 à 3 hétéroatomes sélectionné(s) parmi S, SO, SO₂, O, N et NR^{5a} ; et
R⁷ étant sélectionné dans le groupe consistant en aryle en C₃₋₁₂, hétérocycloalkényle ayant de 3 à 12 éléments, hétéroaryle ayant de 3 à 12 éléments et alkényle en C₂₋₆, R⁷ étant facultativement substitué par un ou plusieurs substituants sélectionné(s) dans le groupe consistant en R^{5a} ;
ou sel pharmaceutiquement acceptable de celui-ci.

3. Composé selon l'une des revendications 1 ou 2 dans lequel :
R^{a} et R^{c} sont indépendamment sélectionnés dans le groupe consistant en hydrido, hydroxyle, méthoxy, éthoxy, propoxy, butoxy, méthyle, éthyle, propyle, butyle, pentyle, hexyle, chlorométhyle, dichlorométhyle, trichlorométhyle, fluorométhyle, difluorométhyle, trifluorométhyle, phényle, biphényle, naphtyle, indényle, pyridinyle, benzothiophényle, indolyle, isoquinolinyle, quinolinyle, thiényle, pyrrolyle, furyle, pyrazolyle, imidazolyle, isoxazolyle, oxazolyle et isoindoledionyle ;
R^{b} est un groupe fonctionnel cyclique ayant de 3 à 12 éléments sélectionnés dans le groupe consistant en cyclopropyle, cyclobutyle, cyclopentyle, cyclohexyle, cyclopropényle, cyclobutényle, cyclopentényle, cyclohexényle, phényle, biphényle, naphtyle, indényle, pipéridinyle, pyrrolidinyle, pyrazolidinyle, imidazolidinyle, isoxazolidinyle, oxazolidinyle, isoindolyle, dihydroindolyle, isoindoline, dihydrothiophényle, dihydropyrrolyle, dihydrofuryle, dihydropyrazolyle, dihydroimidazolyle, dihydroisoxazolyle, dihydrooxazolyle, pyridinyle, benzothiophényle, indolyle, isoquinolinyle, quinolinyle, thiényle, pyrrolyle, furyle, pyrazolyle, imidazolyle, isoxazolyle, oxazolyle et isoindoledionyle, R^{b} étant facultativement substitué par un ou plusieurs substituants sélectionné(s) dans le groupe consistant en R², cyclopropyle, cyclobutyle, cyclopentyle, cyclohexyle, cyclopropylméthyle, cyclopropyléthyle, cyclobutylméthyle, cyclobutyléthyle, cyclopentylméthyle, cyclopentyléthyle, cyclohexylméthyle et cyclohexyléthyle,
R^{d} est sélectionné dans le groupe consistant en -(CH₂)_{q}NH₂, - (CH₂)_{q}NHR², pipéridinyle, pyrrolidinyle, pyrazolidinyle et imidazolidinyle, ledit groupe pipéridinyle, pyrrolidinyle, pyrazolidinyle ou imidazolidinyle pouvant être facultativement substitué par un ou plusieurs substituants sélectionné(s) dans le groupe consistant en R² ;
R² étant sélectionné dans le groupe consistant en chloro, fluoro, bromo, iodo, méthylsulfinyle, éthylsulfinyle, propylsulfinyle, butylsulfinyle, méthyl-sulfonyle, éthylsulfonyle, propylsulfonyle, butylsulfonyle, cyano, méthoxycarbonyle, éthoxycarbonyle, propoxycarbonyle, butoxycarbonyle, méthyle, éthyle, propyle, butyle, pentyle, hexyle, chlorométhyle, dichlorométhyle, trichlorométhyle, fluorométhyle, difluorométhyle, trifluorométhyle, hydroxyméthyle, hydroxyéthyle, hydroxypropyle, hydroxybutyle, hydroxypentyle, hydroxyhexyle, chlorométhoxy, dichlorométhoxy, trichlorométhoxy, fluorométhoxy, difluorométhoxy, trifluorométhoxy, nitro, méthylcarbonylamino, éthyl-carbonylamino, propyl-carbonylamino, butylcarbonylamino, pentylcarbonylamino, hexylcarbonylamino, phénylcarbonylamino, benzylcarbonylamino, R⁷, -OR³, (CH₂)ₘOR³, - (CH₂)ₚCO₂R³, -SR³, -SO₂N(R^{4a}) R^{4b}, -NR^{5a}R^{5b}, -NR^{5a}COR^{5b}, -NR^{5a}CO (OR^{5b}), -NR^{5a}SO₂R⁶, -NR^{5a}SO₂N (R^{6a}) R^{6b}, -NR^{5a} CON (R^{6a}) R^{6b}, -COR^{5a} et -CON (R^{4a})R^{4b} ;
R³, R^{4a} et R^{4b} étant indépendamment sélectionnés dans le groupe consistant en hydrido, phényle, biphényle, naphtyle, indényle, pyridinyle, benzothiophényle, indolyle, isoquinolinyle, quinolinyle, thiényle, pyrrolyle, furyle, pyrazolyle, imidazolyle, isoxazolyle, oxazolyle, isoindoledionyle, pyridinylméthyle, pyridinyléthyle, benzothiophénylméthyle, benzothiophényléthyle, indolylméthyle, indolyléthyle, isoquinolinylméthyle, isoquinolinyléthyle, quinolinylméthyle, quinolinyléthyle, thiénylméthyle, thiényléthyle, pyrrolylméthyle, pyrrolyléthyle, furylméthyle, furyléthyle, pyrazolylméthyle, pyrazolyléthyle, imidazolylméthyle, imidazolyléthyle, isoxazolylméthyle, isoxazolyléthyle, oxazolylméthyle, oxazolyléthyle, isoindoledionylméthyle, isoindoledionyléthyle, méthyle, éthyle, propyle, butyle, pentyle, hexyle, chlorométhyle, dichlorométhyle, trichlorométhyle, fluorométhyle, difluorométhyle, trifluorométhyle, éthényle, propényle, butényle, pentényle, éthynyle, propynyle, butynyle, pentynyle, hydroxyméthyle, hydroxyéthyle, hydroxypropyle, hydroxybutyle, hydroxypentyle, hydroxyhexyle, aminométhyle, aminoéthyle, aminopropyle, aminobutyle, aminopentyle, aminohexyle, méthylaminométhyle, éthylaminométhyle, propylaminométhyle, méthylaminoéthyle, éthylaminoéthyle, propylaminoéthyle, méthylaminopropyle, éthylaminopropyle, propylaminopropyle, méthylaminobutyle, éthylaminobutyle, propylaminobutyle, méthylaminopentyle, éthylaminopentyle, propylaminopentyle, méthylaminohexyle, éthylaminohexyle, propylaminohexyle, N,N-diméthylaminométhyle, N,N-diméthyl-aminoéthyle, N-méthyl-N-éthylaminométhyle, N-méthyl-N-éthylaminoéthyle, N-méthyl-N-propylaminométhyle, N-méthyl-N-propylamino-éthyle, N,N-diéthylaminométhyle, N,N-diéthyl-aminoéthyle, N-éthyl-N-propylaminométhyle, N-éthyl-N-propylaminoéthyle, N,N-dipropylaminométhyle, N,N-dipropyl-aminoéthyle, méthoxy, éthoxy, propoxy, butoxy, méthoxyméthyle, méthoxyéthyle, méthoxypropyle, éthoxy-méthyle, éthoxyéthyle, éthoxypropyle, propoxyméthyle, propoxyéthyle, propoxypropyle, butoxyméthyle, butoxyéthyle, butoxypropyle, pipéridinyle, pyrrolidinyle, pyrazolidinyle, imidazolidinyle, isoxazolidinyle, oxazolidinyle, isoindolyle, dihydro-indolyle, isoindoline, dihydrothiophényle, dihydro-pyrrolyle, dihydrofuryle, dihydropyrazolyle, dihydro-imidazolyle, dihydroisoxazolyle, dihydrooxazolyle, cyclopropyle, cyclobutyle, cyclopentyle, cyclohexyle, cyclopropylméthyle, cyclopropyléthyle, cyclobutylméthyle, cyclobutyléthyle, cyclopentylméthyle, cyclopentyléthyle, cyclohexylméthyle, cyclohexyléthyle, benzyle, phényléthyle, benzylamino et phényléthylamino, ledit groupe fonctionnel phényle, biphényle, naphtyle ou indényle étant facultativement substitué par un ou plusieurs radicaux sélectionné(s) dans le groupe consistant en méthyle, éthyle, propyle, butyle, pentyle, hexyle, aminométhyle, aminoéthyle, aminopropyle, aminobutyle, aminopentyle, aminohexyle, méthoxy, éthoxy, propoxy, butoxy, chloro, fluoro, bromo et iodo, R^{4a} et R^{4b} pouvant être pris ensemble pour former un groupe fonctionnel sélectionné dans le groupe consistant en pipéridinyle, pyrrolidinyle, pyrazolidinyle, imidazolidinyle, isoxazolidinyle, oxazolidinyle, isoindolyle, dihydroindolyle, isoindoline, dihydrothiophényle, dihydropyrrolyle, dihydrofuryle, dihydropyrazolyle, dihydroimidazolyle, dihydroisoxazolyle, dihydrooxazolyle, pyridinyle, benzothiophényle, indolyle, isoquinolinyle, quinolinyle, thiényle, pyrrolyle, furyle, pyrazolyle, imidazolyle, isoxazolyle, oxazolyle et isoindoledionyle ;
R^{5a} et R^{5b} étant indépendamment sélectionnés dans le groupe consistant en hydrido, méthyle, éthyle, propyle, butyle, pentyle, hexyle, phényle, biphényle, naphtyle, indényle, pyridinyle, benzothiophényle, indolyle, isoquinolinyle, quinolinyle, thiényle, pyrrolyle, furyle, pyrazolyle, imidazolyle, isoxazolyle, oxazolyle, isoindoledionyle, benzyle, phényléthyle, isoindolyle, dihydroindolyle, isoindoline, dihydrothiophényle, dihydropyrrolyle, dihydrofuryle, dihydropyrazolyle, dihydroimidazolyle, dihydroisoxazolyle, dihydrooxazolyle, cyclopropyle, cyclobutyle, cyclopentyle, cyclohexyle, pipéridinyle, pyrrolidinyle, pyrazolidinyle, imidazolidinyle, isoxazolidinyle, oxazolidinyle, chlorométhyle, dichlorométhyle, trichlorométhyle, fluorométhyle, difluorométhyle, trifluorométhyle, benzylamino, phényléthylamino, amino, aminométhyle, aminoéthyle, aminopropyle, aminobutyle, aminopentyle, aminohexyle, aminométhylcarbonyle, aminoéthylcarbonyle, aminopropylcarbonyle, aminobutylcarbonyle, aminopentylcarbonyle, aminohexylcarbonyle, aminophénylcarbonyle, aminobenzylcarbonyle, nitro, azido, pyridinylméthyle, pyridinyléthyle, benzothiophénylméthyle, benzothiophényléthyle, indolylméthyle, indolyléthyle, isoquinolinyl-méthyle, isoquinolinyléthyle, quinolinylméthyle, quinolinyléthyle, thiénylméthyle, thiényléthyle, pyrrolylméthyle, pyrrolyléthyle, furylméthyle, furyléthyle, pyrazolylméthyle, pyrazolyléthyle, imidazolylméthyle, imidazolyléthyle, isoxazolylméthyle, isoxazolyléthyle, oxazolylméthyle, oxazolyléthyle, isoindoledionylméthyle et isoindoledionyléthyle, lesdits groupes fonctionnels méthyle, éthyle, propyle, butyle, pentyle, hexyle, phényle, biphényle, naphtyle, indényle, pyridinyle, benzothiophényle, indolyle, isoquinolinyle, quinolinyle, thiényle, pyrrolyle, furyle, pyrazolyle, imidazolyle, isoxazolyle, oxazolyle, isoindoledionyle, aminométhyle, aminoéthyle, aminopropyle, aminobutyle, aminopentyle, aminohexyle, benzyle et phényléthyle étant facultativement substitués par un ou plusieurs substituants sélectionnés dans le groupe consistant en méthylsulfonamido, éthylsulfonamido, propylsulfonamido, butylsulfonamido, sulfamyle, méthyle, éthyle, propyle, butyle, pentyle, hexyle, méthylthio, éthylthio, propylthio, butylthio, méthylsulfinyle, éthylsulfinyle, propylsulfinyle, butylsulfinyle, méthylsulfonyle, éthylsulfonyle, propylsulfonyle, butylsulfonyle, N-méthylamino, N-éthylamino, N-propylamino, aminométhyle, aminoéthyle, aminopropyle, aminobutyle, aminopentyle, aminohexyle, méthylaminométhyle, éthylaminométhyle, propylaminométhyle, méthylaminoéthyle, éthylaminoéthyle, propylaminoéthyle, méthylaminopropyle, éthylaminopropyle, propylaminopropyle, méthylaminobutyle, éthylaminobutyle, propylaminobutyle, méthylaminopentyle, éthylaminopentyle, propylaminopentyle, méthylaminohexyle, éthylaminohexyle, propylaminohexyle, méthoxy, éthoxy, propoxy, butoxy, chloro, fluoro, bromo, iodo, acyloxy, oxy, formyle, chlorométhyle, dichlorométhyle, trichlorométhyle, fluorométhyle, difluorométhyle, trifluorométhyle, cyano, chlorométhoxy, dichlorométhoxy, trichlorométhoxy, fluorométhoxy, difluorométhoxy, trifluorométhoxy, méthylcarbonyle, éthylcarbonyle, propylcarbonyle, butylcarbonyle, pentylcarbonyle, hexylcarbonyle, phénylcarbonyle, benzylcarbonyle, carboxyle, hydroxy, hydroxyméthoxy, hydroxyéthoxy, hydroxypropoxy, hydroxybutoxy, phénoxy, nitro, azido, benzyloxy, N,N-diméthylaminométhylcarbonyle, N,N-diméthylaminoéthylcarbonyle, N,N-diméthylaminophénylcarbonyle, N-méthyl-N-éthylaminométhylcarbonyle, N-méthyl-N-éthylaminoéthylcarbonyle, N-méthyl-N-éthylaminophénylcarbonyle, N-méthyl-N-propylaminométhylcarbonyle, N-méthyl-N-propylaminoéthylcarbonyle, N-méthyl-N-propylaminophénylcarbonyle, N,N-diéthylaminométhylcarbonyle, N,N-diéthylaminoéthylcarbonyle, N,N-diéthylamino-phénylcarbonyle, N-éthyl-N-propylaminométhylcarbonyle, N-éthyl-N-propylaminoéthylcarbonyle, N-éthyl-N-propylamino-phénylcarbonyle, N,N-dipropylaminométhylcarbonyle, N,N-dipropylaminoéthylcarbonyle, N,N-dipropylamino-phénylcarbonyle, thiométhyle, thioéthyle, thiopropyle, thiobutyle, thiopentyle, thiohexyle, aminométhyl-carbonyloxy, aminoéthylcarbonyloxy, aminopropylcarbonyloxy, aminobutylcarbonyloxy, aminopentylcarbonyloxy, aminohexylcarbonyloxy, aminophénylcarbonyloxy, aminobenzylcarbonyloxy, thiocyanato, isothiocyanato, méthyldioxy, éthyldioxy, propyldioxy, butyldioxy, pentyldioxy, hexyldioxy, hydroxyméthyle, hydroxyéthyle, hydroxypropyle, hydroxybutyle, hydroxypentyle, hydroxyhexyle, N-méthylamino, N-éthylamino, N-propylamino, méthoxycarbonyle, éthoxycarbonyle, propoxycarbonyle, butoxycarbonyle, méthoxyméthyle, méthoxyéthyle, méthoxypropyle, éthoxyméthyle, éthoxyéthyle, éthoxypropyle, propoxyméthyle, propoxyéthyle, propoxypropyle, butoxyméthyle, butoxyéthyle, butoxypropyle, éthénylamino, propénylamino, buténylamino, penténylamino, éthynylamino, propynylamino, butynylamino, pentynylamino, éthényle, propényle, butényle, pentényle, éthynyle, propynyle, butynyle, pentynyle, N,N-diméthylaminométhoxy, N,N-diméthylaminoéthoxy, N-méthyl-N-éthylaminométhoxy, N-méthyl-N-éthylaminoéthoxy, N-méthyl-N-propylaminométhoxy, N-méthyl-N-propylaminoéthoxy, N,N-diéthylaminométhoxy, N,N-diéthylaminoéthoxy, N-éthyl-N-propylaminométhoxy, N-éthyl-N-propylaminoéthoxy, N,N-dipropylaminométhoxy, N,N-dipropylaminoéthoxy, pipéridinyle, pyrrolidinyle, pyrazolidinyle, imidazolidinyle, isoxazolidinyle, oxazolidinyle, isoindolyle, dihydroindolyle, isoindoline, dihydrothiophényle, dihydropyrrolyle, dihydrofuryle, dihydropyrazolyle, dihydroimidazolyle, dihydroisoxazolyle, dihydrooxazolyle, pyridinyle, benzothiophényle, indolyle, isoquinolinyle, quinolinyle, thiényle, pyrrolyle, furyle, pyrazolyle, imidazolyle, isoxazolyle, oxazolyle et isoindoledionyle ;
R^{6a} et R^{6b} étant indépendamment sélectionnés dans le groupe consistant en hydrido, méthyle, éthyle, propyle, butyle, pentyle, hexyle, pyridinyle, benzothiophényle, indolyle, isoquinolinyle, quinolinyle, thiényle, pyrrolyle, furyle, pyrazolyle, imidazolyle, isoxazolyle, oxazolyle, isoindoledionyle, isoindolyle, dihydroindolyle, isoindoline, dihydrothiophényle, dihydropyrrolyle, dihydrofuryle, dihydropyrazolyle, dihydroimidazolyle, dihydroisoxazolyle, dihydrooxazolyle, chlorométhyle, dichlorométhyle, trichlorométhyle, fluorométhyle, difluorométhyle, trifluorométhyle, benzylamino, phényléthylamino, pyridinylméthyle, pyridinyléthyle, benzothiophénylméthyle, benzothiophényléthyle, indolylméthyle, indolyléthyle, isoquinolinylméthyle, isoquinolinyléthyle, quinolinylméthyle, quinolinyléthyle, thiénylméthyle, thiényléthyle, pyrrolylméthyle, pyrrolyléthyle, furylméthyle, furyléthyle, pyrazolylméthyle, pyrazolyléthyle, imidazolylméthyle, imidazolyléthyle, isoxazolylméthyle, isoxazolyléthyle, oxazolylméthyle, oxazolyléthyle, isoindoledionylméthyle, isoindoledionyléthyle, phényle, biphényle, naphtyle, indényle, benzyle et phényléthyle, lesdits groupes fonctionnels phényle, biphényle, naphtyle, indényle, pyridinyle, benzothiophényle, indolyle, isoquinolinyle, quinolinyle, thiényle, pyrrolyle, furyle, pyrazolyle, imidazolyle, isoxazolyle, oxazolyle, isoindoledionyle, isoindolyle, dihydroindolyle, isoindoline, dihydrothiophényle, dihydropyrrolyle, dihydrofuryle, dihydropyrazolyle, dihydroimidazolyle, dihydroisoxazolyle, dihydrooxazolyle, benzyle et phényléthyle étant facultativement substitués par un ou plusieurs substituants sélectionné(s) parmi méthyle, éthyle, propyle, butyle, péntyle, hexyle, méthoxy, éthoxy, propoxy, butoxy, chloro, fluoro, bromo, iodo, chlorométhyle, dichlorométhyle, trichlorométhyle, fluorométhyle, difluorométhyle, trifluorométhyle, cyano, chlorométhoxy, dichlorométhoxy, trichlorométhoxy, fluorométhoxy, difluorométhoxy, trifluorométhoxy, méthylcarbonyle, éthylcarbonyle, propylcarbonyle, butylcarbonyle, pentylcarbonyle, hexylcarbonyle, phénylcarbonyle, benzylcarbonyle, carboxyle, hydroxy, hydroxyméthoxy, hydroxyéthoxy, hydroxypropoxy, hydroxybutoxy, phénoxy, benzyloxy, N,N-diméthylaminométhoxy, N,N-diméthylaminoéthoxy, N-méthyl-N-éthylaminométhoxy, N-méthyl-N-éthylaminoéthoxy, N-méthyl-N-propylaminométhoxy, N-méthyl-N-propylamino-éthoxy, N,N-diéthylaminométhoxy, N,N-diéthylaminoéthoxy, N-éthyl-N-propylaminométhoxy, N-éthyl-N-propylaminoéthoxy, N,N-dipropylaminométhoxy, N,N-dipropylaminoéthoxy, pipéridinyle, pyrrolidinyle, pyrazolidinyle, imidazolidinyle, isoxazolidinyle, oxazolidinyle, isoindolyle, dihydroindolyle, isoindoline, dihydrothiophényle, dihydropyrrolyle, dihydrofuryle, dihydropyrazolyle, dihydroimidazolyle, dihydroisoxazolyle, dihydrooxazolyle, pyridinyle, benzothiophényle, indolyle, isoquinolinyle, quinolinyle, thiényle, pyrrolyle, furyle, pyrazolyle, imidazolyle, isoxazolyle, oxazolyle et isoindoledionyle, R^{6a} et R^{6b} pouvant être pris ensemble pour former un groupe fonctionnel sélectionné dans le groupe consistant en pipéridinyle, pyrrolidinyle, pyrazolidinyle, imidazolidinyle, isoxazolidinyle, oxazolidinyle, isoindolyle, dihydroindolyle, isoindoline, dihydrothiophényle, dihydropyrrolyle, dihydrofuryle, dihydropyrazolyle, dihydroimidazolyle, dihydroisoxazolyle, dihydrooxazolyle, pyridinyle, benzothiophényle, indolyle, isoquinolinyle, quinolinyle, thiényle, pyrrolyle, furyle, pyrazolyle, imidazolyle, isoxazolyle, oxazolyle et isoindoledionyle ; et
R⁷ étant sélectionné dans le groupe consistant en phényle, biphényle, naphtyle, indényle, isoindolyle, dihydroindolyle, isoindoline, dihydrothiophényle, dihydropyrrolyle, dihydrofuryle, dihydropyrazolyle, dihydroimidazolyle, dihydroisoxazolyle, dihydrooxazolyle, pyridinyle, benzothiophényle, indolyle, isoquinolinyle, quinolinyle, thiényle, pyrrolyle, furyle, pyrazolyle, imidazolyle, isoxazolyle, oxazolyle, isoindoledionyle, éthényle, propényle, butényle et pentényle, R⁷ étant facultativement substitué par un ou plusieurs substituants sélectionné(s) dans le groupe' consistant en R^{5a};
ou sel pharmaceutiquement acceptable de celui-ci.

4. Composé selon la revendication 1, dans lequel le composé de Formule I est un composé de Formule II : 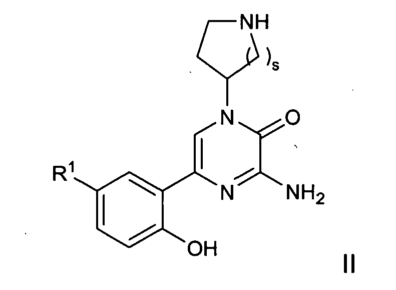 dans lequel :
s représente 1, 2 ou 3 ;
R¹ est sélectionné dans le groupe consistant en hydrido, -OR³, alkyle en C₁₋₆, cycloalkyle en C₅₋₇, benzyle, -CH₂(cycloalkyle en C₃₋₇), aryle, halogéno, hétérocycloalkyle, hétérocycloalkényle et hétéroaryle ;
R³ étant sélectionné dans le groupe consistant en hydrido, alkyle en C₁₋₆, cycloalkyle en C₅₋₇, benzyle, -CH₂(cycloalkyle en C₃₋₇) et aryle,
ou sel pharmaceutiquement acceptable de celui-ci.

5. Composé selon la revendication 4 dans lequel:
R¹ est sélectionné dans le groupe consistant en hydrido, -OR³, alkyle en C₁₋₆, cycloalkyle en C₅₋₇, benzyle, -CH₂(cycloalkyle en C₃₋₇), aryle en C₃₋₁₂, halogéno, hétérocycloalkyle ayant de 3 à 12 éléments, hétérocycloalkényle ayant de 3 à 12 éléments et hétéroaryle ayant de 3 à 12 éléments ; et
R³ est sélectionné dans le groupe consistant en hydrido, alkyle en C₁₋₆, cycloalkyle en C₅₋₇, benzyle, -CH₂(cycloalkyle en C₃₋₇) et aryle en C₃₋₁₂,
ou sel pharmaceutiquement acceptable de celui-ci.

6. Composé selon la revendication 5 :
dans lequel R¹ est sélectionné dans le groupe consistant en hydrido, -OR³, méthyle, éthyle, propyle, butyle, pentyle, hexyle, cyclopentyle, cyclohexyle, cycloheptyle, benzyle, méthylcyclopropyle, méthylcyclobutyle, méthylcyclopentyle, méthylcyclohexyle, phényle, biphényle, naphtyle, indényle, chloro, fluoro, bromo, iodo, pipéridinyle, pyrrolidinyle, pyrazolidinyle, imidazolidinyle, isoxazolidinyle, oxazolidinyle, isoindolyle, dihydroindolyle, isoindoline, dihydrothiophényle, dihydropyrrolyle, dihydrofuryle, dihydropyrazolyle, dihydroimidazolyle, dihydroisoxazolyle, dihydrooxazolyle, pyridinyle, benzothiophényle, indolyle, isoquinolinyle, quinolinyle, thiényle, pyrrolyle, furyle, pyrazolyle, imidazolyle, isoxazolyle, oxazolyle et isoindoledionyle ; et
dans lequel R³ est sélectionné dans le groupe consistant en hydrido, méthyle, éthyle, propyle, butyle, pentyle, hexyle, cyclopentyle, cyclohexyle, cycloheptyle, benzyle, méthylcyclopropyle, méthylcyclobutyle, méthylcyclopentyle, méthylcyclohexyle, phényle, biphényle, naphtyle et indényle ;
ou sel pharmaceutiquement acceptable de celui-ci.

7. Composé selon la revendication 1, dans lequel le composé de Formule I est un composé de Formule III : 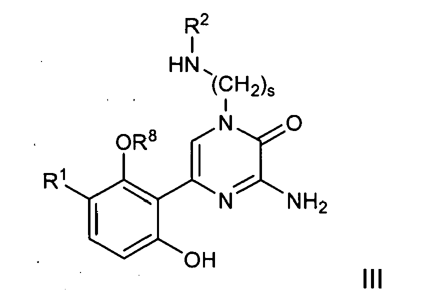 dans lequel :
s représente 1, 2 ou 3 ;
R¹ est sélectionné dans le groupe consistant en hydrido, -OR³, alkyle en C₁₋₆, cycloalkyle en C₅₋₇, benzyle, -CH₂ (cycloalkyle en C₃₋₇), aryle, halogéno, hétérocycloalkyle, hétérocycloalkényle et hétéroaryle ;
R² représente hydrido ou alkyle en C₁₋₆ ;
R³ étant sélectionné dans le groupe consistant en alkyle en C₁₋₆, cycloalkyle en C₅₋₇, benzyle, -CH₂(cycloalkyle en C₃₋₇) et aryle ; et
R⁸ est sélectionné dans le groupe consistant en alkyle en C₁₋₆, cycloalkyle en C₅₋₇, benzyle et -CH₂ (cycloalkyle en C₃₋₇) ;
ou sel pharmaceutiquement acceptable de celui-ci.

8. Composé selon la revendication 7 dans lequel :
R¹ est sélectionné dans le groupe consistant en hydrido, -OR³, alkyle en C₁₋₆, cycloalkyle en C₅₋₇, benzyle, -CH₂ (cycloalkyle en C₃₋₇), aryle en C₃₋₁₂. halogéno, hétérocycloalkyle ayant de 3 à 12 éléments, hétérocycloalkényle ayant de 3 à 12 éléments et hétéroaryle ayant de 3 à 12 éléments ;
R² représente hydrido ou alkyle en C₁₋₆ ;
R³ étant sélectionné dans le groupe consistant en alkyle en C₁₋₆, cycloalkyle en C₅₋₇, benzyle, -CH₂ (cycloalkyle en C₃₋₇) et aryle en C₃₋₁₂ ; et
R⁸ est sélectionné dans le groupe consistant en alkyle en C₁₋₆, cycloalkyle en C₅₋₇, benzyle et -CH₂(cycloalkyle en C₃₋₇);
ou sel pharmaceutiquement acceptable de celui-ci.

9. Composé selon la revendication 8 dans lequel :
R¹ est sélectionné dans le groupe consistant en hydrido, -OR³, méthyle, éthyle, propyle, butyle, pentyle, hexyle, cyclopentyle, cyclohexyle, cycloheptyle, benzyle, méthylcyclopropyle, méthylcyclobutyle, méthylcyclopentyle, méthylcyclohexyle, phényle, biphényle, naphtyle, indényle, chloro, fluoro, bromo, iodo, pipéridinyle, pyrrolidinyle, pyrazolidinyle, imidazolidinyle, isoxazolidinyle, oxazolidinyle, isoindolyle, dihydroindolyle, isoindoline, dihydrothiophényle, dihydropyrrolyle, dihydrofuryle, dihydropyrazolyle, dihydroimidazolyle, dihydroisoxazolyle, dihydrooxazolyle, pyridinyle, benzothiophényle, indolyle, isoquinolinyle, quinolinyle, thiényle, pyrrolyle, furyle, pyrazolyle, imidazolyle, isoxazolyle, oxazolyle et isoindoledionyle ;
R² est sélectionné dans le groupe consistant en hydrido, méthyle, éthyle, propyle, butyle, pentyle et hexyle;
R³ étant sélectionné dans le groupe consistant en méthyle, éthyle, propyle, butyle, pentyle, hexyle, cyclopentyle, cyclohexyle, cycloheptyle, benzyle, méthylcyclopropyle, méthylcyclobutyle, méthylcyclopentyle, méthylcyclohexyle, phényle, biphényle, naphtyle et indényle ; et
R⁸ est sélectionné dans le groupe consistant en méthyle, éthyle, propyle, butyle, pentyle, hexyle, cyclopentyle, cyclohexyle, cycloheptyle, benzyle, méthylcyclopropyle, méthylcyclobutyle, méthylcyclopentyle et méthylcyclohexyle ;
ou sel pharmaceutiquement acceptable de celui-ci.

10. Composé selon la revendication 1, dans lequel le composé de Formule I est un composé de Formule IV : 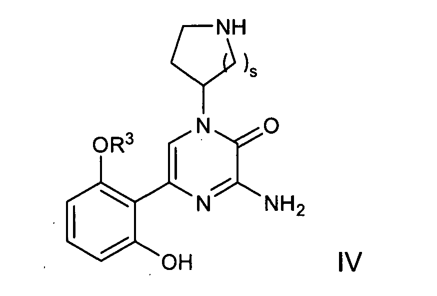 dans lequel n représente 1, 2 ou 3 ; et
dans lequel R³ est sélectionné dans le groupe consistant en hydrido, alkyle en C₁₋₆, cycloalkyle en C₅₋₇, benzyle et -CH₂ (cycloalkyle en C₃₋₇);
ou sel pharmaceutiquement acceptable de celui-ci.

11. Composé selon la revendication 10 :
dans lequel R³ est sélectionné dans le groupe consistant en hydrido, méthyle, éthyle, propyle, butyle, pentyle, hexyle, cyclopentyle, cyclohexyle, cycloheptyle, benzyle, méthylcyclopropyle, méthylcyclobutyle, méthyl-(alkyle en C₁₋₆)cyclopentyle et méthylcyclohexyle ;
ou sel pharmaceutiquement acceptable de celui-ci.

12. Composé selon la revendication 1, sélectionné dans le groupe de composés consistant en :
• la 3-amino-5-(2-hydroxyphényl)-1-pipéridin-3-ylpyrazin-2(1H)-one ;
• la 3-amino-5-(5-fluoro-2-hydroxyphényl)-1-pipéridin-3-ylpyrazin-2(1H)-one ;
• la 3-amino-5-(4-fluoro-2-hydroxyphényl)-1-pipéridin-3-ylpyrazin-2(1H)-one ;
• la 3-amino-5-(3-fluoro-2-hydroxyphényl)-1-pipéridin-3-ylpyrazin-2(1H)-one ;
• la 3-amino-5-(2-fluoro-6-hydroxyphényl)-1-pipéridin-3-ylpyrazin-2(1H)-one ;
• la 3-amino-5-(2-bromo-6-hydroxyphényl)-1-pipéridin-3-ylpyrazin-2(1H)-one ;
• la 3-amino-5-(5-bromo-2-hydroxyphényl)-1-pipéridin-3-ylpyrazin-2(1H)-one ;
• la 3-amino-5-(4-bromo-2-hydroxyphényl)-1-pipéridin-3-ylpyrazin-2(1H)-one ;
• la 3-amino-5-(3-bromo-2-hydroxyphényl)-1-pipéridin-3-ylpyrazin-2(1H)-one ;
• la 3-amino-5-(2-chloro-6-hydroxyphényl)-1-pipéridin-3-ylpyrazin-2(1H)-one ;
• la 3-amino-5-(5-chloro-2-hydroxyphényl)-1-pipéridin-3-ylpyrazin-2(1H)-one ;
• la 3-amino-5-(4-chloro-2-hydroxyphényl)-1-pipéridin-3-ylpyrazin-2(1H)-one ;
• la 3-amino-5-(3-chloro-2-hydroxyphényl)-1-pipéridin-3-ylpyrazin-2(1H)-one ;
• la 3-amino-5-(2-hydroxy-6-méthylphényl)-1-pipéridin-3-ylpyrazin-2(1H)-one ;
• la 3-amino-5-(2-hydroxy-5-méthylphényl)-1-pipéridin-3-ylpyrazin-2(1H)-one ;
• la 3-amino-5-(2-hydroxy-4-méthylphényl)-1-pipéridin-3-ylpyrazin-2(1H)-one ;
• la 3-amino-5-(2-hydroxy-3-méthylphényl)-1-pipéridin-3-ylpyrazin-2(1H)-one ;
• la 3-amino-5-(5-cyclopentyl-2-hydroxyphényl)-1-pipéridin-3-ylpyrazin-2(1H)-one ;
• la 3-amino-5-(5-cyclohexyl-2-hydroxyphényl)-1-pipéridin-3-ylpyrazin-2(1H)-one ;
• la 3-amino-5-(5-cycloheptyl-2-hydroxyphényl)-1-pipéridin-3-ylpyrazin-2(1H)-one ;
• la 3-amino-5-(5-cyclopropylméthyl-2-hydroxyphényl)-1-pipéridin-3-ylpyrazin-2(1H)-one ;
• la 3-amino-5-(5-cyclobutylméthyl-2-hydroxyphényl)-1-pipéridin-3-ylpyrazin-2(1H)-one;
• la 3-amino-5-(5-cyclopentylméthyl-2-hydroxyphényl)-1-pipéridin-3-ylpyrazin-2(1H)-one;
• la 3-amino-5-(5-cyclohexylméthyl-2-hydroxyphényl)-1-pipéridin-3-ylpyrazin-2(1H)-one;
• la 3-amino-5-(5-phényl-2-hydroxyphényl)-1-pipéridin-3-ylpyrazin-2(1H)-one ;
• la 3-amino-5-[2-hydroxy-5-(1-naphtyl)phényl]-1-pipéridin-3-ylpyrazin-2(1H)-one;
• la 3-amino-5-[2-hydroxy-5-(2-naphtyl)phényl]-1-pipéridin-3-ylpyrazin-2(1H)-one ;
• la 3-amino-5-(2-hydroxy-5-pyrrolidin-2-ylphényl)-1-pipéridin-3-ylpyrazin-2(1H)-one ;
• la 3-amino-5-(2-hydroxy-5-pyrrolidin-3-ylphényl)-1-pipéridin-3-ylpyrazin-2(1H)-one ;
• la 3-amino-5-(2-hydroxy-5-pyrrolidin-1-ylphényl)-1-pipéridin-3-ylpyrazin-2(1H)-one ;
• la 3-amino-5-(2-hydroxy-5-pyrazolidin-1-ylphényl)-1-pipéridin-3-ylpyrazin-2(1H)-one ;
• la 3-amino-5-(2-hydroxy-5-imidazolidin-1-ylphényl)-1-pipéridin-3-ylpyrazin-2(1H)-one ;
• la 3-amino-5-(2-hydroxy-5-pyrazolidin-3-ylphényl)-1-pipéridin-3-ylpyrazin-2(1H)-one ;
• la 3-amino-5-(2-hydroxy-5-pyrazolidin-4-ylphényl)-1-pipéridin-3-ylpyrazin-2(1H)-one ;
• la 3-amino-5-(2-hydroxy-5-imidazolidin-1-ylphényl)-1-pipéridin-3-ylpyrazin-2(1H)-one ;
• la 3-amino-5-(2-hydroxy-5-imidazolidin-4-ylphényl)-1-pipéridin-3-ylpyrazin-2(1H)-one ;
• la 3-amino-5-(2-hydroxy-5-tétrahydrofuran-2-ylphényl)-1-pipéridin-3-ylpyrazin-2(1H)-one ;
• la 3-amino-5-(2-hydroxy-5-tétrahydrofuran-3-ylphényl)-1-pipéridin-3-ylpyrazin-2(1H)-one ;
• la 3-amino-5-(2-hydroxy-5-tétrahydrothién-2-ylphényl)-1-pipéridin-3-ylpyrazin-2(1H)-one ;
• la 3-amino-5-(2-hydroxy-5-tétrahydrothién-3-ylphényl)-1-pipéridin-3-ylpyrazin-2(1H)-one ;
• la 3-amino-5-(2-hydroxy-5-isoxazolidin-2-ylphényl)-1-pipéridin-3-ylpyrazin-2(1H)-one ;
• la 3-amino-5-[2-hydroxy-5-(1,3-oxazolidin-3-yl) phényl]-1-pipéridin-3-ylpyrazin-2(1H)-one ;
• la 3-amino-5-(2-hydroxy-5-isoxazolidin-3-ylphényl)-1-pipéridin-3-ylpyrazin-2 (1H)-one ;
• la 3-amino-5-[2-hydroxy-5-(1,3-oxazolidin-4-yl)phényl]-1-pipéridin-3-ylpyrazin-2(1H)-one ;
• la 3-amino-5-[2-hydroxy-5-(1,3-oxazolidin-2-yl)phényl]-1-pipéridin-3-ylpyrazin-2(1H)-one ;
• la 3-amino-5-(2-hydroxy-5-isoxazolidin-4-ylphényl)-1-pipéridin-3-ylpyrazin-2(1H)-one ;
• la 3-amino-5-[2-hydroxy-5-(1,3-oxazolidin-5-yl)phényl]-1-pipéridin-3-ylpyrazin-2(1H)-one ;
• la 3-amino-5-(2-hydroxy-5-isoxazolidin-5-ylphényl)-1-pipéridin-3-ylpyrazin-2(1H)-one ;
• la 3-amino-5-[2-hydroxy-5-(1H-pyrrol-1-yl)phényl]-1-pipéridin-3-ylpyrazin-2(1H)-one ;
• la 3-amino-5-[2-hydroxy-5-(1H-pyrrol-2-yl)phényl]-1-pipéridin-3-ylpyrazin-2(1H)-one ;
• la 3-amino-5-[2-hydroxy-5-(1H-pyrrol-3-yl)phényl]-1-pipéridin-3-ylpyrazin-2(1H)-one ;
• la 3-amino-5-[2-hydroxy-5-(1H-pyrazol-1-yl)phényl]-1-pipéridin-3-ylpyrazin-2(1H)-one ;
• la 3-amino-5-[2-hydroxy-5-(1H-imidazol-1-yl)phényl]-1-pipéridin-3-ylpyrazin-2(1H)-one ;
• la 3-amino-5-[2-hydroxy-5-(1H-pyrazol-3-yl)phényl]-1-pipéridin-3-ylpyrazin-2(1H)-one ;
• la 3-amino-5-[2-hydroxy-5-(1H-imidazol-4-yl)phényl]-1-pipéridin-3-ylpyrazin-2(1H)-one ;
• la 3-amino-5-[2-hydroxy-5-(1H-imidazol-2-yl)phényl]-1-pipéridin-3-ylpyrazin-2(1H)-one ;
• la 3-amino-5-[5-(2-furyl)-2-hydroxyphényl]-1-pipéridin-3-ylpyrazin-2(1H)-one ;
• la 3-amino-5-[5-(3-furyl)-2-hydroxyphényl]-1-pipéridin-3-ylpyrazin-2(1H)-one ;
• la 3-amino-5-(2-hydroxy-5-thién-2-ylphényl)-1-pipéridin-3-ylpyrazin-2(1H)-one ;
• la 3-amino-5-(2-hydroxy-5-thién-3-ylphényl)-1-pipéridin-3-ylpyrazin-2(1H)-one ;
• la 3-amino-5-(2-hydroxy-5-isoxazolidin-2-ylphényl)-1-pipéridin-3-ylpyrazin-2(1H)-one ;
• la 3-amino-5-(2-hydroxy-5-isoxazol-3-ylphényl)-1-pipéridin-3-ylpyrazin-2(1H)-one ;
• la 3-amino-5-[2-hydroxy-5-(1,3-oxazol-4-yl)phényl]-1-pipéridin-3-ylpyrazin-2(1H)-one ;
• la 3-amino-5-[2-hydroxy-5-(1,3-oxazol-2-yl)phényl]-1-pipéridin-3-ylpyrazin-2(1H)-one ;
• la 3-amino-5-(2-hydroxy-5-isoxazol-4-ylphényl)-1-pipéridin-3-ylpyrazin-2(1H)-one ;
• la 3-amino-5-[2-hydroxy-5-(1,3-oxazol-5-yl)phényl]-1-pipéridin-3-ylpyrazin-2(1H)-one ;
• la 3-amino-5-(2-hydroxy-5-isoxazol-5-ylphényl)-1-pipéridin-3-ylpyrazin-2(1H)-one ;
• la 3-amino-5-(2-hydroxy-5-pipéridin-1-ylphényl)-1-pipéridin-3-ylpyrazin-2(1H)-one ;
• la 3-amino-5-(2-hydroxy-5-pipéridin-2-ylphényl)-1-pipéridin-3-ylpyrazin-2(1H)-one ;
• la 3-amino-5-(2-hydroxy-5-pipéridin-3-ylphényl)-1-pipéridin-3-ylpyrazin-2(1H)-one ;
• la 3-amino-5-(2-hydroxy-5-pipéridin-4-ylphényl)-1-pipéridin-3-ylpyrazin-2(1H)-one ;
• la 3-amino-5-(2-hydroxy-5-pipérazin-1-ylphényl)-1-pipéridin-3-ylpyrazin-2(1H)-one ;
• la 3-amino-5-(2-hydroxy-5-pipérazin-2-ylphényl)-1-pipéridin-3-ylpyrazin-2(1H)-one;
• la 3-amino-5-(2-hydroxy-5-pyridin-2-ylphényl)-1-pipéridin-3-ylpyrazin-2(1H)-one ;
• la 3-amino-5-(2-hydroxy-5-pyridin-3-ylphényl)-1-pipéridin-3-ylpyrazin-2(1H)-one ;
• la 3-amino-5-(2-hydroxy-5-pyridin-4-ylphényl)-1-pipéridin-3-ylpyrazin-2(1H)-one ;
• la 3-amino-5-(2-hydroxy-5-pyridazin-3-ylphényl)-1-pipéridin-3-ylpyrazin-2(1H)-one ;
• la 3-amino-5-(2-hydroxy-5-pyridazin-4-ylphényl)-1-pipéridin-3-ylpyrazin-2(1H)-one ;
• la 3-amino-5-(2-hydroxy-5-pyrazin-2-ylphényl)-1-pipéridin-3-ylpyrazin-2(1H)-one ;
• la 3-amino-5-(5-benzyl-2-hydroxyphényl)-1-pipéridin-3-ylpyrazin-2(1H)-one ;
• la 3-amino-5-(2,5-dihydroxyphényl)-1-pipéridin-3-ylpyrazin-2(1H)-one ;
• la 3-amino-5-(2-hydroxy-5-méthoxyphényl)-1-pipéridin-3-ylpyrazin-2(1H)-one ;
• la 3-amino-5-(5-éthoxy-2-hydroxyphényl)-1-pipéridin-3-ylpyrazin-2(1H)-one ;
• la 3-amino-5-(2-hydroxy-5-propoxyphényl)-1-pipéridin-3-ylpyrazin-2(1H)-one ;
• la 3-amino-5-(2-hydroxy-5-isopropoxyphényl)-1-pipéridin-3-ylpyrazin-2(1H)-one ;
• la 3-amino-5-(2-hydroxy-5-t-butoxyphényl)-1-pipéridin-3-ylpyrazin-2(1H)-one ;
• la 3-amino-5-(2-hydroxy-5-phénoxyphényl)-1-pipéridin-3-ylpyrazin-2(1H)-one ;
• la 3-amino-5-[2-hydroxy-5-(1-naphtyloxy)phényl]-1-pipéridin-3-ylpyrazin-2(1H)-one ;
• la 3-amino-5-[2-hydroxy-5-(2-naphtyloxy)phényl]-1-pipéridin-3-ylpyrazin-2(1H)-one ;
• la 3-amino-5-[5-(benzyloxy)-2-hydroxyphényl]-1-pipéridin-3-ylpyrazin-2(1H)-one ;
• la 3-amino-5-[5-(cyclopropylméthoxy)-2-hydroxyphényl]-1-pipéridin-3-ylpyrazin-2(1H)-one ;
• la 3-amino-5-[5-(cyclopentylméthoxy)-2-hydroxyphényl]-1-pipéridin-3-ylpyrazin-2(1H)-one ;
• la 3-amino-5-[5-(cyclohexylméthoxy)-2-hydroxyphényl]-1-pipéridin-3-ylpyrazin-2(1H)-one ;
• la 3-amino-5-[5-(cyclohexyloxy)-2-hydroxyphényl]-1-pipéridin-3-ylpyrazin-2 (1H)-one ; et
• la 3-amino-5-[5-(cyclopentyloxy)-2-hydroxyphényl]-1-pipéridin-3-ylpyrazin-2(1H)-one.

13. Composition pharmaceutique comprenant un composé selon l'une quelconque des revendications 1-12 ou un sel pharmaceutiquement acceptable correspondant, et un support, diluant ou adjuvant pharmaceutiquement acceptable.

14. Utilisation d'un composé selon l'une quelconque des revendications 1-2 dans la préparation d'un médicament pour le traitement du cancer, de l'inflammation ou d'un trouble associé à une inflammation chez un sujet.

15. Utilisation selon la revendication 14, dans laquelle le médicament est destiné au traitement de l'arthrite, du cancer, de l'asthme, de la MPOC, de la fragilité, du diabète, de l'athérosclérose, de la douleur et/ou d'une maladie dermatologique.
